# EUROPEAN PATENT SPECIFICATION

(11) **EP 1 043 319 B1**
(45) Date of publication and mention of the grant of the patent: **16.03.2005**
(21) Application number: 98951687.7
(22) Date of filing: 04.11.1998
(51) Int. Cl.: C07D 277/68, C07D 277/70, C07D 277/82, C07D 417/06, A61K 31/425, A61K 31/445

(54) **NOVEL ALKYLAMINO DERIVATIVES**
ALKYLAMINODERIVATE
NOUVEAUX DERIVES ALKYLAMINO

(30) Priority: 05.11.1997 JP 30260797
(43) Date of publication of application: 11.10.2000
(73) Proprietor: Mitsubishi Chemical Corporation, Tokyo 108-0014 (JP)
(72) Inventor: ROCHER, Jean-Philippe, Tsukuba-shi Ibaraki 305-0821 (JP); YAMABE, Haruko Mitsubishi Chemical Coporation, Yokohama-shi Kanagawa 227-8502 (JP); CHAKI, Haruyuki Mitsubishi Chemical Coporation, Kashima-gun, Ibaraki 314-0255 (JP); SAITO, Ken-ichi Mitsubishi Chemical Coporation, Yokohama-shi Kanagawa 227-8502 (JP); ABE, Michikazu Mitsubishi Chemical Coporation, Yokohama-shi Kanagawa 227-8502 (JP); OKUYAMA, Masahiro Mitsubishi Chemical Coporation, Yokohama-shi Kanagawa 227-8502 (JP)
(74) Representative: Sternagel, Fleischer, Godemeyer & Partner
(86) International application number: PCT/JP1998/004973
(87) International publication number: WO 1999/023083

(56) References cited:
- WO-A-96/05185
- JP-A- 54 151 966
- JP-A- 55 015 455
- JP-T- 5 507 918
- UCAR H ET AL: "2(3H)-Benzoxazolone and 2(3H)-benzothiazolone derivatives: novel, potent and selective sigma1 receptor ligands" EUROPEAN JOURNAL OF PHARMACOLOGY, vol. 335, no. 2-3, 24 September 1997 (1997-09-24), pages 267-273, XP002920030
- UCAR HUSEYIN et al., "2(3H)-Benzoxazolone and 2(3H)-Benzothiazolone Derivatives: Novel, Potent and Selective sigma 1 Receptor Ligands", EUR. J. PHARMACOL., Vol. 335, No. 2/3, (1997), p. 267-273, XP002920030

## Description

### Technical Field

The present invention relates to novel compounds that act as a ligand for the sigma receptor/binding site and medicaments comprising said compounds as an active ingredient.

### Background Art

The recently identified sigma receptor/binding site of the brain is an important target for the development of antipsychotic drugs that are free from the side affects of currently available antipsychotic drugs having antagonistic activity on the dopamine D2 receptor (J.M. Walker and W.D. Bowen, F.O. Walker and R.R. Matsumoto, B. de Costa and K.C. Rice, Pharmacological Reviews, 42, pp.355-402, 1990; G. Debonnel, J. Psychiatr. Neurosci., 18, 4, pp.157-172, 1993; G. Debonnel and C. de Montigny, Life Sciences, 58, 9, pp.721-734, 1996). Further, some data have been reported that suggest the regulation of signal transmission by a sigma ligand (also referred to as "sigma receptor ligand" in the specification) and its receptor through control of a calcium level in synaptosomes (P. J. Brent, H. Saunders and P.R. Dunkley, Neurosci. Lett., 211, pp-138-142, 1996).

The term "receptor" used herein means a membrane binding type receptor and other binding sites. Existence of at least two sorts of sigma receptor subtypes, i.e., sigma 1 and sigma 2, has been revealed, and classification of sigma binding sites has been proposed (R. Quirion, W.D. Bowen, Y. Itzhak, J.L. Junien, J.M. Musacchio, R.B. Rothman, T.P. Su, W. Tam and D.P. Taylor, TiPS, 13, pp.85-86, 1992). The sigma 1 binding site is characterized to have high affinity for haloperidol, di-o-tolylguanidine (DTG) and (+)-benzomorphane such as (+)-pentazocine, whilst the sigma 2 binding site is characterized to have high affinity for haloperidol and DTG, but have low affinity for (+)-benzomorphane.

The sigma 1 ligand has an action on the gastrointestinal tract, and it seems that the sigma 1 site may mediate suppression to muscarine-like acetylcholine receptor/phosphoinositide response by the sigma ligands. The sigma 1 binding site is present not only in brains, but on spleen cells (Y. Lin, B.B. Whitlock, J.A. Pultz and S.A. Wolfe Jr, J. Neuroimmunol., 58, pp.143-154, 1995), and such sigma ligands may suppress the immune system (H.H. Garza, S. Mayo, W.D. Bowen. B.R. DeCosta and D.J.J. Carr, J. of Immunology, 151, 9, pp.4672-4680, 1993).

The sigma 2 binding site is abundant in livers (A.E. Bruce, S.B. Hellewell and W.D. Bowen, Neurosci. Abstr., 16, 370, 1990; A.S. Basile, I.A. Paul and B. DeCosta, Eur. J. Pharmacol. Mol. Pharm. Sect., 227, pp.95-98, 1992; C. Torrence-Campbell and W.D. Bowen, Eur. J. Pharmacol., 304, pp.201-210, 1996), kidneys (W.D. Bowen, G. Feinstein and J.S. Orringer, Soc. Neurosci. Abstr., 18, 456, abstract 195.8, 1992), and hearts (M. Dumont and S. Lemaire, Eur. J. Pharmacol., 209, pp.245-248, 1991).

The sigma 2 binding site in brains exists in hypothalamus, cerebellum, pons medulla and medulla oblongata. In hippocampus, frontal lobe and occipital lobe in rat brains, it exists more abundantly than the sigma 1 binding site (D. J. McCann, A.D. Weissmann and T.P. Su, Soc. Neurosci. abstr. 18, 22, abstract 16.5, 1992). In hippocampus synaptosomes of guinea pig, there are also the sigma 2 binding site that is selectively labeled with [³H] BIMU (D.W. Bonhaus, D.N. Loury, L.B. Jakeman, Z. To, A. DeSouza, R.M. Eglen and E.H.F. Wong, J. Pharmacol. Exp. Ther., 267, 2, pp.961-970, 1993). The relationship between the sigma 2 binding site and cortex as well as limbic system supports the usefulness of compounds used for treatment of mental diseases (D.C. Mash and C.P. Zabetian, Synapse, 12, pp.195-205, 1992).

It has been believed that the sigma 2 binding site is involved in motility functions, especially dystonia (R.R. Matsumoto, M.K. Hemstreet, N.L. Lai and A. Thurkauf, B.R. DeCosta, K.C. Rice, S.B. Hellewell, W.D. Bowen and J.M. Walker, Pharmacol. Biochem. Behav., 36, pp.151-155, 1990). However, no evidence demonstrating such an action has been found in primate models of functional disorders of extrapyramidal tract (L.T. Meltzer, C.L. Christoffersen, K.A. Serpa, T.A. Pugsley, A. Razmpour and T.G. Heffner, Neuropharmacology, 31, 9, pp.961-967, 1992).

Haloperidol, which is a clinically effective dopaminergic antipsychotic agent, shows high affinity for these two sigma subtypes. However, a reduced metabolite of haloperidol that acts on the central nervous system has more excellent affinity and selectivity for the sigma 2 receptor than dopamine D2 as compared to haloperidol (J.C. Jaen., B.W. Caprathe, T.A. Pugsley, L.D. Wise and H. Akunne, J. Med. Chem., 36, pp.3929-3936, 1993). Since a selective agent has not been available, pharmacological significance, distribution and functions of the sigma 2 binding site have not yet been elucidated. On the other hand, recent studies revealed that the sigma 2 site played a role for controlling functions of ileum (G.G. Kinney, E.W. Haris, R. Ray and T.J. Hudzik, Europ. J. Pharmacol., 294, pp.547-553, 1995). These data suggest that the selective sigma 2 ligand is useful for the treatment of irritable bowel syndrome.

There are lot of publications relating to heteroarylalkylamino derivatives. However, almost no data is available as to compounds having benzothiazoline as a heteroaryl moiety and similar compounds. As compounds having anti-glutamic acid effect, several classes of polyfluoroalkoxy-2-iminobenzothiazolines have been reported (P. Jimonet P., F. Audiaus, M. Barreau, F. Beaudoin, J.-C. Blanchard, G. Dutruc-Rosset, C. Gueremy, P. Laduron, J. Le Blevec, S. Mignani, C. Nemecek, J. Rataud and J.-M. Stutzmann, presented at XIIth International Symposium on Medicinal Chemistry, Basel, Switzerland, 1992; P. Jimonet P., M. Barreau, J.-C. Blanchard, A. Boireau, A. Doble, P. Laduron, J. Lavayre, C. Malgouris, O. Piot, J. Pratt, J. Rataud, M. Reibaud, S. Mignani and J.-M. Stutzmann, Bioorg. and Med. Chem., 2, 8, pp.793-798, 1994).

Benzimidazolemethylpiperidine derivatives are disclosed in Japanese Patent Application No. 58-180481, International Patent Publication WO87/02359, WO87/02035, WO87/02666 and U.S. Patent No. 4,215,119. European Patent Publication No. 546,388 discloses heteroarylmethylpiperidine derivatives of chroman, however, it does not discloses examples of any benzothiazoline derivatives. International Patent Publication WO93/22309 discloses 4-indomethyl-1-[2'-phenyl-2'-oxoethyl]piperidines as serotonine 5-HT₂ antagonists. U.S. Patent No. 5,500,433 discloses piperidinoethanones as a dopamine and serotonine antagonist. However, it has not been known that the aforementioned compounds have affinity for the sigma binding site.

Moreover, from WO 96/05185 alkylamino derivatives as σ₂ selective ligands are known.

Ucar et al. refers in European Journal of Pharmacology, vol. 335, no. 2-3, 24 september 1997, pages 267-273 to 2(3H)-benzoxazolone and 2(3H)-benzothiazolone derivatives as being novel, potent and selective σ₁ receptor ligands.

### Disclosure of the Invention

An object of the present invention is to provide a novel compound that has excellent affinity for the sigma binding site. Another object of present invention is to provide a medicament comprising a compound having the aforementioned characteristics as an active ingredient which is useful for therapeutic and/or preventive treatment of diseases caused by nerve control effect of the sigma ligand.

The inventors of the present invention earnestly studied to achieve the aforementioned objects. As a result, they found that compounds represented by the following formula had high affinity for the sigma ligand binding site and low inhibition constant Ki for sigma 1 and/or sigma 2. They also found that these compound showed a selective binding profile completely different from those of conventional known compounds, and were useful for therapeutic and/or preventive treatment of diseases caused or promoted by the nerve control function of the sigma ligands. The present invention was achieved on the basis of these findings.

The present invention thus provides compounds represented by the following formula (I): X-Q-C(R¹)(R²)-Z, salts thereof, and hydrates thereof and solvates thereof, wherein:
R¹ and R² each independently represent hydrogen atom, an alkyl group, a cycloalkyl group, a hydroxyalkyl group, or an alkenyl group;
Z represents 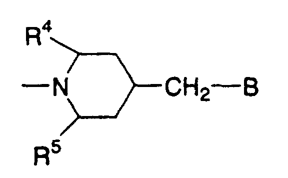 wherein R⁴ and R⁵ each independently represent hydrogen atom or an alkyl group, or R⁴ and R⁵ represent a 5- to 7-membered heterocyclic group together with other intervening atoms; and B represents a group represented by the following formula: 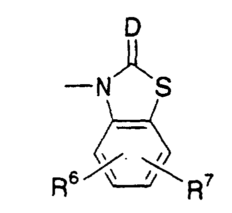 wherein R⁶ and R⁷ each independently represent a substituent selected from the group consisting of hydrogen atom, a halogen atom, nitro group, an alkyl group, a cycloalkyl group, an alkenyl group, a halogenated alkyl group, hydroxyl group, an alkoxyl group, a halogenated alkoxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group, and an alkynyl group; and D represents sulfur atom, oxygen atom, or a group represented by NR⁸ wherein R⁸ represents a substituent selected from the group consisting of hydrogen atom, an alkyl group, a cycloalkyl group, a cycloalkyl-substituted alkyl group, an alkenyl group, a halogenated alkyl group, a hydroxyalkyl group, an alkoxyalkyl group, an alkoxycarbonyl group, phenyl group, an alkylsulfinyl group, an alkylsulfonyl group, a substituted or unsubstituted sulfamoyl group, an alkylsulfonylamino group, an alkylcarbonylamino group, a substituted or unsubstituted carbamoyl group, an acyl group, cyano group and an alkynyl group; and
when X represents an aryl group which may have one or more substituents on the ring selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group, or a heteroaryl group which may have one or more substituents on the ring selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group, Q represents a group represented by -CO-, -C(=NOH)- or -C(Y)(A)- wherein Y represents hydrogen atom, an alkyl group, an alkenyl group, an alkynyl group, a monocyclic or polycyclic cycloalkyl group, a cycloalkyl-substituted alkyl group, an aryl group, an aryl-substituted alkyl group, a heteroaryl group, or a heteroaryl-substituted alkyl group, and A represents a group represented by -OR⁹ wherein R⁹ represents hydrogen atom, an alkyl group, a cycloalkyl group, a cycloalkyl-substituted alkyl group, an alkenyl group, an aryl group, an aryl-substituted alkyl group, a hydroxyalkyl group, or an acyl group, and when X represents a 8- to 10-membered bicyclic heteroaryl group containing one or two hetero atoms, Q represents a single bond.

According to another aspect of the present invention, there are provided medicaments which comprises a substance selected from the group consisting of the aforementioned compounds and salts thereof, and hydrates thereof and solvates thereof as an active ingredient. As a preferred embodiment thereof, there are provided the aforementioned medicaments which are used for therapeutic and/or preventive treatment of diseases caused or promoted by the nerve controlling function of sigma ligands (for example, central nervous system diseases, gastroenteric diseases or heart defective diseases and the like). The present invention also provides sigma ligands which comprise a substance selected from the group consisting of the aforementioned compounds and salts thereof, and hydrates thereof and solvates thereof.

According to further aspects of the present invention, there are provided a use of a substance selected from the group consisting of the aforementioned compounds and salts thereof, and hydrates thereof and solvates thereof for the manufacture of the aforementioned medicaments, preferably in the form of a pharmaceutical composition, and methods for therapeutic and/or preventive treatment of diseases caused or promoted by the nerve controlling function of sigma ligands, which comprise the step of administering to a mammal including a human an effective amount of a substance selected from the group consisting of the aforementioned compounds and salts thereof, and hydrates thereof and solvates thereof.

### Best Mode for Carrying out the Invention

In the aforementioned formula (I), R¹ and R² each independently represent hydrogen atom, an alkyl group, a cycloalkyl group, a hydroxyalkyl group, or an alkenyl group. Among them, both of R¹ and R² may preferably be hydrogen atoms.

In the specification, the term "alkyl group" and the term "alkyl" for a group containing an alkyl group as a moiety encompass, for example, linear or branched alkyl groups having 1 to 6 carbon atoms, preferably 1 to 4 carbon atoms. More specifically, as the alkyl group, methyl group, ethyl group, n-propyl group, isopropyl group, n-butyl group, isobutyl group, sec-butyl group, tert-butyl group, n-pentyl group, isopentyl group, neopentyl group, tert-pentyl group, n-hexyl group and the like may be used.

In the specification, the term "cycloalkyl group" and the term "cycloalkyl" for a group containing a cycloalkyl group as a moiety encompass, for example, 3- to 8-membered cycloalkyl groups, preferably approximately 3- to 6-membered cycloalkyl groups (monocyclic cycloalkyl groups unless otherwise specifically mentioned). These cycloalkyl groups may have one or more alkyl groups on their rings. More specifically, for example, cyclopropyl group, methylcyclopropyl group, cyclobutyl group, cyclopentyl group, cyclohexyl group, methylcyclohexyl group, dimethylcyclohexyl group and the like may be used as the cycloalkyl group.

The term "hydroxyalkyl group" used herein encompasses alkyl groups having one or more hydroxyl groups, preferably one hydroxyl group. As the hydroxyalkyl group, a hydroxyalkyl group having 2-6 carbon atoms, more specifically, 2-hydroxyethyl group, 3-hydroxypropyl group, 4-hydroxybutyl group, 5-hydroxypentyl group, 6-hydroxyhexyl group and the like may preferably be used.

The term "alkenyl group" used herein encompass linear or branched alkenyl groups having 2 to 6 carbon atoms, preferably 3 to 6 carbon atoms, and containing one or more double bonds, preferably one double bond. For example, 1-propenyl group, allyl group, isopropenyl group, 1-butenyl group, 3-butenyl group, 1-pentenyl group, 2-pentenyl group, 3-pentenyl group, 4-pentenyl group, 1-hexenyl group, 2-hexenyl group and the like may be used as the alkenyl group. The double bond contained in the alkenyl group may be either in cis- or trans-configuration.

In the aforementioned group represented by Z, R⁴ and R⁵ each independently represent hydrogen atom or an alkyl group, or R⁴ and R⁵ represent a 5- to 7-membered heterocyclic ring together with other intervening atoms. Among them, preferred compounds include those wherein R⁴ and R⁵ each independently represent hydrogen atom or an alkyl group.

In the aforementioned group represented by B, R⁶ and R⁷ each independently represents a substituent selected from the group consisting of hydrogen atom, a halogen atom, nitro group, an alkyl group, a cycloalkyl group, an alkenyl group, a halogenated alkyl group, hydroxyl group, an alkoxy group, a halogenated alkoxy group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group.

When the terms "halogen atom" or "halogenated" is used in this specification, any of fluorine atom, chlorine atom, bromine atom and iodine atom may be used as the halogen atom. The term "halogenated alkyl group" used in this specification encompasses, for example, monofluoromethyl group, difluoromethyl group, trifluoromethyl group, trifluoroethyl group, pentafluoroethyl group, monochloromethyl group, dichloromethyl group, trichloromethyl group and the like, and preferred examples thereof include trifluoromethyl group.

In the specification, the term "alkoxyl group" and the term "alkoxy" for a group containing an alkoxyl group as a moiety encompass, for example, linear or branched alkoxyl groups having 1 to 6 carbon atoms, preferably 1 to 4 carbon atoms. More specifically, methoxy group, ethoxy group, n-propoxy group, isopropoxy group, n-butoxy group, isobutoxy group, tert-butoxy group, n-pentyloxy group, n-hexyloxy group and the like may be used as the alkoxy group. For example, trifluoromethoxy group, trifluoroethoxy group, monochloromethoxy group, trichloromethoxy group and the like may be used as the halogenated alkoxy group.

As the alkoxycarbonyl group, for example, alkoxycarbonyl groups formed by the aforementioned alkoxyl groups may be used, and more specifically, methoxycarbonyl group, ethoxycarbonyl group, n-propoxycarbonyl group, isopropoxycarbonyl group and the like may be used. Examples of the alkylthio group include, for example, methylthio group, ethylthio group, n-propylthio group, isopropylthio group, n-butylthio group, isobutylthio group, n-pentylthio group, n-hexylthio group and the like.

Examples of the substituted amino group include, for example, an alkyl amino group such as a monoalkylamino group and a dialkylamino group, an acylamino group such as an alkanoylamino group and an arylcarbonylamino group, an aralkylamino group, an alkylsulfonylamino group and the like. More specifically, examples include an alkylamino group such as monomethylamino group, dimethylamino group, ethylamino group, diethylamino group, methylethylamino group, propylamino group, dipropylamino group, butylamino group, pentylamino group and hexyl amino group; an acylamino group such as acetylamino groups, trifluoroacetylamino group, propionylamino group, benzoylamino group and p-methoxybenzoylamino group; an aralkylamino group such as benzylamino group and p-methoxybenzylamino group; and an alkylsulfonylamino group such as methylsulfonylamino group, ethylsulfonylamino group, n-propylsulfonylamino group and n-hexylsulfonylamino group.

Examples of the alkylsulfinyl group include, for example, methylsulfinyl group, ethylsulfinyl group, n-propylsulfinyl group, n-butylsulfinyl group, n-pentylsulfinyl group, n-hexylsulfinyl group and the like. Examples of the alkylsulfonyl group include, for example, methylsulfonyl group, ethylsulfonyl group, n-propylsulfonyl group, n-butylsulfonyl group, n-pentylsulfonyl group, n-hexylsulfonyl and the like. Examples of the substituted sulfamoyl group include, for example, a sulfamoyl group which is substituted with one or two alkyl groups, and more specifically, methylaminosulfonyl group, dimethylaminosulfonyl group, ethylaminosulfonyl group, diethylaminosulfonyl group and the like may be used.

Examples of the substituted carbamoyl group include, for example, a carbamoyl group which is substituted with one or two alkyl groups, and more specifically, examples include methylcarbamoyl group, dimethylcarbamoyl group, ethylcarbamoyl group and the like. Examples of the acyl group include an alkanoyl group (e.g., an alkylcarbonyl group, a halogenated alkylcarbonyl group and the like), an arylcarbonyl groups, a heteroarylcarbonyl group and the like. More specifically, formyl group, acetyl group, propionyl group, trifluoromethylcarbonyl group, pentafluoroethylcarbonyl group, benzoyl group, p-methoxybenzoyl group, 3-pyridylcarbonyl group and the like may be used. Examples of the alkynyl group include, for example, a linear or branched alkynyl group having 2 to 6 carbon atoms, preferably 3 to 6 carbon atoms, for example, 2-propynyl group, 3-butynyl group, 4-pentynyl group, 5-hexynyl groups and the like.

Among them, R⁶ and R⁷ may preferably be independently a substituent selected from the group consisting of hydrogen atom, a halogen atom, an alkyl group, a halogenated alkyl group, and an alkoxyl group, and more preferably, R⁶ may be hydrogen atom. Compounds wherein R⁶ is hydrogen atom and R⁷ is hydrogen atom, fluorine atom, chlorine atom, bromine atom, methyl group, trifluoromethyl group, or methoxy group are more preferred, and particularly preferred are those wherein R⁶ is hydrogen atom and R⁷ is chlorine atom or bromine atom. Substituting positions of R⁶ and R⁷ are not particularly limited. R⁷ may preferably substitute in the 6-position of the benzothiazolin-3-yl group represented by B when R⁶ is hydrogen atom and R⁷ is a group other than hydrogen atom.

In the group represented by B according to the aforementioned formula, D represents sulfur atom, oxygen atom or a group represented as NR⁸. R⁸ represents a substituent selected from the group consisting of hydrogen atom, an alkyl group, a cycloalkyl group, a cycloalkyl-substituted alkyl group, an alkenyl group, a halogenated alkyl group, a hydroxyalkyl group, an alkoxyalkyl group, an alkoxycarbonyl group, phenyl group, an alkylsulfinyl group, an alkylsulfonyl group, a substituted or unsubstituted sulfamoyl group, an alkylsulfonylamino group, an alkylcarbonylamino group, a substituted or unsubstituted carbamoyl group, an acyl group, cyano group, and an alkynyl group.

Examples of the cycloalkyl-substituted alkyl group include, for example, cyclopropylmethyl group, methylcyclopropylmethyl group, cyclopropylethyl group, cyclopropylpropyl group, cyclobutylmethyl group, cyclobutylethyl group, cyclopentylmethyl group, cyclohexylmethyl group, dimethylcyclohexylmethyl group and the like. Examples of the alkoxyalkyl group include, for example, 2-methoxyethyl group, 3-methoxypropyl group, 2-ethoxyethyl group, 3-ethoxypropyl group, or 3-propoxypropyl group and the like.

As the alkoxycarbonyl group, alkylsulfinyl group, alkylsulfonyl group, substituted sulfamoyl group, alkylsulfonylamino group, alkylcarbonylamino group, substituted carbamoyl group, acyl group and alkynyl group, those exemplified above can be used. Among them, preferred examples include compounds wherein R⁸ is selected from the group consisting of hydrogen atom, an alkyl group, a halogenated alkyl group, and an acyl group, and more preferred examples include those wherein R⁸ is selected from the group consisting of hydrogen atom, methyl group, ethyl group, 2,2,2-trifluoroethyl group, and acetyl group.

In the aforementioned formula (I), X represents an aryl group which may have one or more substituents on the ring selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group, or a heteroaryl group which may have one or more substituents on the ring selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group, Q represents a group represented by -CO-, -C(=NOH)- or -C(Y)(A)- wherein Y represents hydrogen atom, an alkyl group, an alkenyl group, an alkynyl group, a monocyclic or polycyclic cycloalkyl group, a cycloalkyl-substituted alkyl group, an aryl group, an aryl-substituted alkyl group, a heteroaryl group, or a heteroaryl-substituted alkyl group, and A represents a group represented by -OR⁹ wherein R⁹ represent hydrogen atom, an alkyl group, a cycloalkyl group, a cycloalkyl-substituted alkyl group, an alkenyl group, an aryl group, an aryl-substituted alkyl group, a hydroxyalkyl group, or an acyl group. Alternatively, X represents a 8- to 10-membered bicyclic heteroaryl group containing one or two hetero atoms, and Q represents a single bond. When Q is a group other than a single bond, Q may preferably be -CH(OH)- or -CO-.

As the polycyclic cycloalkyl group, a 5- to 12-membered polycycloalkyl group, for example, norbornyl group, adamantyl group and the like may be used, and adamantyl group may preferably be used. As the cycloalkyl-substituted alkyl group, those exemplified above may be used. As the aryl group, an monocyclic to tricyclic aryl group, for example, besides phenyl group, 1-naphthyl group and 2-naphthyl group, phenanthryl group, anthracenyl group and the like may be used.

As the heteroaryl group, a 5- to 10-membered monocyclic to tricyclic heterocyclic group containing 1 to 4 hetero atoms which are selected from oxygen atom, sulfur atom and nitrogen atom as ring-constituting atoms. For example, there can be used furyl group (furan ring), benzofuranyl group (benzofuran ring), isobenzofuranyl group (isobenzofuran ring), thienyl group (thiophene ring), benzothiophenyl group (benzothiophene ring), pyrrolyl group (pyrrole ring), imidazolyl group (imidazole ring), pyrazolyl group (pyrazole ring), thiazolyl group (thiazole ring), benzothiazolyl group (benzothiazole ring), isothiazolyl group (isothiazole ring), benzisothiazolyl group (benzisothiazole ring), triazolyl group (triazole ring), tetrazolyl group (tetrazole ring), pyridyl group (pyridine ring), pyrazinyl group (pyrazine ring), pyrimidinyl group (pyrimidine ring), pyridazinyl group (pyridazine ring), indolyl group (indole ring), isoindolyl group (isoindole ring), benzimidazolyl group (benzimidazole ring), purinyl group (purine ring), quinolyl group (quinoline ring), isoquinolyl group (isoquinoline ring), dihydroisoquinolyl group (dihydroquinoline ring), phthalazinyl group (phthalazine ring), naphthylidinyl group (naphthylidine ring), quinoxalinyl group (quinoxaline ring), cinnolinyl group (cinnoline ring), pteridinyl group (pteridine ring), oxazolyl group (oxazole ring), isoxazolyl group (isoxazole ring), benzoxazolyl group (benzoxazole ring), benzisoxazolyl group (benzisoxazole ring), furazanyl group (furazan ring) and the like may be used.

In the specification, when "aryl group" or "heteroaryl group" is referred to, the term encompasses an aryl group or a heteroaryl group having one or more substituents on the ring, unless otherwise indicated. Examples of the substituent include a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group, an alkynyl group and so forh. As these substituents, those explained above may preferably be used.

In the group of -C(Y)(A) represented by Q, Y represents hydrogen atom, an alkyl group, an alkenyl group, an alkynyl group, a monocyclic or polycyclic cycloalkyl group, a cycloalkyl-substituted alkyl group, an aryl group, an aryl-substituted alkyl group, a heteroaryl group or a heteroaryl-substituted alkyl group. Symbol "A" represents a group represented by -OR⁹ wherein R⁹ represent hydrogen atom, an alkyl group, a cycloalkyl group, a cycloalkyl-substituted alkyl group, an alkenyl group, an aryl group, an aryl-substituted alkyl group, a hydroxyalkyl group, or an acyl group.

As the alkynyl group, polycyclic cycloalkyl group, cycloalkyl-substituted alkyl group, aryl group, heteroaryl group, and acyl group in the aforementioned symbols "Y" and "A", those exemplified above can be used. Examples of the aryl-substituted alkyl group include, for example, benzyl group, 1-phenylethyl group, 2-phenylethyl group, 1-phenylpropyl group, 2-phenylpropyl group, 3-phenylpropyl group, 1-naphthylmethyl group, 2-naphthylmethyl group and the like. As the heteroaryl-substituted alkyl group, an alkyl group which is substituted with any of the aforementioned heteroaryl groups may be used. More specifically, 2-furylmethyl group, 3-furylmethyl group, 2-thienylmethyl group, 3-thienylmethyl group, 1-imidazolylmethyl group, 2-imidazolylmethyl group, 2-thiazolylmethyl group. 1-pyridylmethyl group, 2-pyridylmethyl group, 3-pyridylmethyl group, 4-pyridylmethyl group, 1-quinolylmethyl group, 2-quinolylmethyl group and the like may be used.

When X represents a 8- to 10-membered bicyclic heteroaryl group containing one or two hetero atoms, Q represents a single bond. As the 8- to 10-membered bicyclic heteroaryl group, condensed bicyclic heteroaromatic groups as well as bicyclic heteroaryl groups one of whose ring is saturated or partially reduced may be used. For example, benzofuranyl group, indolyl group, benzisothiazolyl group, benzothiazolyl group, benzisoxazolyl group, benzoxazolyl group, benzimidazolyl group, isoquinolinyl group, quinolinyl group, 1,2,3,4-tetrahydroisoquinolinyl group, 1,2,3,4-tetrahydroquinolyl group and the like may be used. Examples of groups preferred as X are shown below.

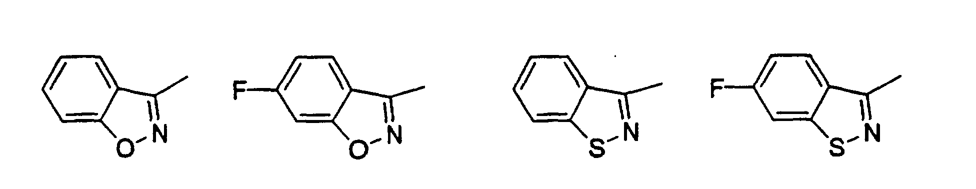

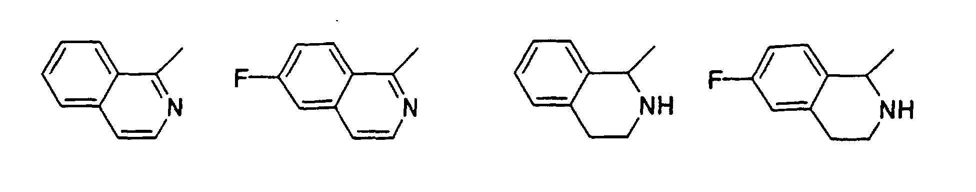

An examples of preferred classes of compounds falling within the aforementioned formula (I) includes:
(1) those wherein R¹ and R² each independently represent hydrogen atom or an alkyl group, both of R⁴ and R⁵ are hydrogen atoms, R⁶ and R⁷ each independently represent a substituent selected from the group consisting of hydrogen atom, a halogen atom, an alkyl group, a halogenated alkyl group, and an alkoxyl group, R⁸ represents a substituent selected from the group consisting of hydrogen atom, an alkyl group, a halogenated alkyl group and an acyl group; when X represents a monocyclic or polycyclic cycloalkyl group which may be substituted with an alkyl group or a substituted or unsubstituted phenyl group (the substituent is at least one substituent selected from the group consisting of a halogen atom, an alkyl group, a halogenated alkyl group, an alkoxyl group, a halogenated alkoxyl group, cyano group, and a substituted or unsubstituted aminosulfonyloxy group), Q is a group represented by -CO-, -C(=NOH)- or -C(Y)(A)- wherein Y is hydrogen atom, and A is a group represented by -OR⁹ wherein R⁹ is hydrogen atom, an alkyl group, or an acyl group, and when X is a 8- to 10-membered bicyclic heteroaryl group containing one or two hetero atoms, Q is a single bond.
   An example of more preferred classes of compounds includes:
(2) those wherein both of R⁴ and R⁵ are hydrogen atoms, R⁶ is hydrogen atom, R⁷ is a substituent selected from the group consisting of hydrogen atom, a halogen atom, an alkyl group, a halogenated alkyl group, and an alkoxyl group, X represents a substituted or unsubstituted phenyl group (the substituent is at least one substituent selected from the group consisting of a halogen atom, an alkyl group, a halogenated alkyl group, an alkoxyl group, a halogenated alkoxyl group, cyano group, and a substituted or unsubstituted aminosulfonyloxy group), and Q is a group represented by -CO-, -C(=NOH)- or -C(Y)(A)- wherein Y is hydrogen atom, and A is a group represented by -OR⁹ wherein R⁹ is hydrogen atom, an alkyl group, or an acyl group.
   Examples of particularly preferred classes of compounds include:
(3) compounds of the above (2) wherein X is a substituted or unsubstituted phenyl group; (4) compounds of the above (3) wherein X is p-fluorophenyl group; (5) compounds of the above (3) or (4) wherein R⁶ is hydrogen atom, R⁷ is a halogen atom (preferably a halogen atom substituting in the 6-position of benzothiazolin-3-yl group), and D is oxygen atom or a group represented by NR⁸ wherein R⁸ is hydrogen atom or an alkyl group; and (6) compounds of the above (3) to (5) wherein Q is -CO- or -CH(OH)-.

More specifically, examples include the aforementioned compounds wherein X is p-fluorophenyl group, R⁷ is chlorine atom, D is NH, and Q is -CO- or -CH(OH)-; the aforementioned compounds wherein X is p-fluorophenyl group, R⁷ is chlorine atom, D is NCH₃, and Q is -CO- or -CH(OH)-; the aforementioned compounds wherein X is p-fluorophenyl group, R⁷ is fluorine atom, D is NH and Q is -CO- or -CH(OH)-; the aforementioned compounds wherein X is p-fluorophenyl group, R⁷ is trifluoromethyl group, D is NH, and Q is -CO- or -CH(OH)-; the aforementioned compounds wherein X is p-fluorophenyl group, R⁷ is bromine atom, D is oxygen atom, and Q is -CO- or -CH(OH)-; the aforementioned compounds wherein X is p-fluorophenyl group, R⁷ is chlorine atom, D is oxygen atom, and Q is -CO- or -CH(OH)-; the aforementioned compounds wherein X is p-fluorophenyl group, R⁷ is fluorine atom, D is oxygen atom, and Q is -CO- or -CH(OH)-; and the aforementioned compounds wherein X is unsubstituted phenyl group, R⁷ is bromine atom, D is oxygen atom, and Q is -CO- or -CH(OH)-. However, the compounds of present invention are not limited to these preferred compounds.

Specific examples of particularly preferred compounds of the present invention are listed in the following tables. However, the compounds of present invention are not limited to these examples. In the compounds mentioned below, both of R¹ and R² are hydrogen atoms. In the tables, for example, the description of "p-F-C₆H₄-" for X means that X is p-fluorophenyl group, and the description of "6-Cl" as for R⁷ means that chlorine atom substitutes in the 6-position of the benzothiazolin-3-yl group. Other descriptions are indicated in similar manners. Table 1 shows piperidine-type compounds. The compounds specifically disclosed in the examples of the present specification are also preferred according to the present invention, as well as those described below.

**Table 1-1**

| Compound No. | X | Q | R⁴ | R⁵ | D | R⁶ | R⁷ |
|---|---|---|---|---|---|---|---|
| 103 | p-F-C₆H₄- | -CO- | H | H | =NH | H | 6-Cl |
| 104 | p-F-C₆H₄- | -CH(OH)- | H | H | =NH | H | 6-Cl |
| 105 | p-Cl-C₆H₄- | -CO- | H | H | =NH | H | 6-Cl |
| 106 | p-Cl-C₆H₄- | -CH(OH)- | H | H | =NH | H | 6-Cl |
| 107 | p-CH₃-C₆H₄- | -CO- | H | H | =NH | H | 6-Cl |
| 108 | p-CH₃-C₆H₄- | -CH(OH)- | H | H | =NH | H | 6-Cl |
| 109 | p-CF₃-C₆H₄- | -CO- | H | H | =NH | H | 6-Cl |
| 110 | p-CF₃-C₆H₄- | -CH(OH)- | H | H | =NH | H | 6-Cl |
| 111 | C₆H₅- | -CO- | H | H | =NH | H | 6-Cl |
| 112 | C₆H₅- | -CH(OH)- | H | H | =NH | H | 6-Cl |
| 115 | p-F-C₆H₄- | -CO- | H | H | =NCH₃ | H | 6-Cl |
| 116 | p-F-C₆H₄- | -CH(OH)- | H | H | =NCH₃ | H | 6-Cl |
| 117 | p-Cl-C₆H₄- | -CO- | H | H | =NCH₃ | H | 6-Cl |
| 118 | p-Cl-C₆H₄- | -CH(OH)- | H | H | =NCH₃ | H | 6-Cl |
| 119 | p-CH₃-C₆H₄- | -CO- | H | H | =NCH₃ | H | 6-Cl |
| 120 | p-CH₃-C₆H₄- | -CH(OH)- | H | H | =NCH₃ | H | 6-Cl |
| 121 | p-CF₃-C₆H₄- | -CO- | H | H | =NCH₃ | H | 6-Cl |
| 122 | p-CF₃-C₆H₄- | -CH(OH)- | H | H | =NCH₃ | H | 6-Cl |
| 123 | C₆H₅- | -CO- | H | H | =NCH₃ | H | 6-Cl |
| 124 | C₆H₅- | -CH(OH)- | H | H | =NCH₃ | H | 6-Cl |
| 127 | p-F-C₆H₄- | -CO- | H | H | =NH | H | 6-F |
| 128 | p-F-C₆H₄- | -CH(OH)- | H | H | =NH | H | 6-F |
| 129 | p-Cl-C₆H₄- | -CO- | H | H | =NH | H | 6-F |
| 130 | p-Cl-C₆H₄- | -CH(OH)- | H | H | =NH | H | 6-F |
| 131 | p-CH₃-C₆H₄- | -CO- | H | H | =NH | H | 6-F |
| 132 | p-CH₃-C₆H₄- | -CH(OH)- | H | H | =NH | H | 6-F |
| 133 | p-CF₃-C₆H₄- | -CO- | H | H | =NH | H | 6-F |
| 134 | p-CF₃-C₆H₄- | -CH(OH)- | H | H | =NH | H | 6-F |
| 135 | C₆H₅- | -CO- | H | H | =NH | H | 6-F |
| 136 | C₆H₅- | -CH(OH)- | H | H | =NH | H | 6-F |

**Table 1-2**

| | | | | | | | |
|---|---|---|---|---|---|---|---|
| 139 | p-F-C₆H₄- | -CO- | H | H | =NCH₃ | H | 6-F |
| 140 | p-F-C₆H₄- | -CH(OH)- | H | H | =NCH₃ | H | 6-F |
| 141 | p-Cl-C₆H₄- | -CO- | H | H | =NCH₃ | H | 6-F |
| 142 | p-Cl-C₆H₄- | -CH(OH)- | H | H | =NCH₃ | H | 6-F |
| 143 | p-CH₃-C₆H₄- | -CO- | H | H | =NCH₃ | H | 6-F |
| 144 | p-CH₃-C₆H₄- | -CH(OH)- | H | H | =NCH₃ | H | 6-F |
| 145 | p-CF₃-C₆H₄- | -CO- | H | H | =NCH₃ | H | 6-F |
| 146 | p-CF₃-C₆H₄- | -CH(OH)- | H | H | =NCH₃ | H | 6-F |
| 147 | C₆H₅- | -CO- | H | H | =NCH₃ | H | 6-F |
| 148 | C₆H₅- | -CH(OH)- | H | H | =NCH₃ | H | 6-F |
| 151 | p-F-C₆H₄- | -CO- | H | H | =NH | H | 6-CF₃ |
| 152 | p-F-C₆H₄- | -CH(OH)- | H | H | =NH | H | 6-CF₃ |
| 153 | p-Cl-C₆H₄- | -CO- | H | H | =NH | H | 6-CF₃ |
| 154 | p-Cl-C₆H₄- | -CH(OH)- | H | H | =NH | H | 6-CF₃ |
| 155 | p-CH₃-C₆H₄- | -CO- | H | H | =NH | H | 6-CF₃ |
| 156 | p-CH₃-C₆H₄- | -CH(OH)- | H | H | =NH | H | 6-CF₃ |
| 157 | p-CF₃-C₆H₄- | -CO- | H | H | =NH | H | 6-CF₃ |
| 158 | p-CF₃-C₆H₄- | -CH(OH)- | H | H | =NH | H | 6-CF₃ |
| 159 | C₆H₅- | -CO- | H | H | =NH | H | 6-CF₃ |
| 160 | C₆H₅- | -CH(OH)- | H | H | =NH | H | 6-CF₃ |
| 163 | p-F-C₆H₄- | -CO- | H | H | =NCH₃ | H | 6-CF₃ |
| 164 | p-F-C₆H₄- | -CH(OH)- | H | H | =NCH₃ | H | 6-CF₃ |
| 165 | p-Cl-C₆H₄- | -CO- | H | H | =NCH₃ | H | 6-CF₃ |
| 166 | p-Cl-C₆H₄- | -CH(OH)- | H | H | =NCH₃ | H | 6-CF₃ |
| 167 | p-CH₃-C₆H₄- | -CO- | H | H | =NCH₃ | H | 6-CF₃ |
| 168 | p-CH₃-C₆H₄- | -CH(OH)- | H | H | =NCH₃ | H | 6-CF₃ |
| 169 | p-CF₃-C₆H₄- | -CO- | H | H | =NCH₃ | H | 6-CF₃ |
| 170 | p-CF₃-C₆H₄- | -CH(OH)- | H | H | =NCH₃ | H | 6-CF₃ |
| 171 | C₆H₅- | -CO- | H | H | =NCH₃ | H | 6-CF₃ |
| 172 | C₆H₅- | -CH(OH)- | H | H | =NCH₃ | H | 6-CF₃ |

**Table 1-3**

| | | | | | | | |
|---|---|---|---|---|---|---|---|
| 175 | p-F-C₆H₄- | -CO- | H | H | =O | H | 6-Br |
| 176 | p-F-C₆H₄- | -CH(OH)- | H | H | =O | H | 6-Br |
| 177 | p-Cl-C₆H₄- | -CO- | H | H | =O | H | 6-Br |
| 178 | p-Cl-C₆H₄- | -CH(OH)- | H | H | =O | H | 6-Br |
| 179 | p-CH₃O-C₆H₄- | -CO- | H | H | =O | H | 6-Br |
| 180 | p-CH₃O-C₆H₄- | -CH(OH)- | H | H | =O | H | 6-Br |
| 181 | C₆H₅- | -CO- | H | H | =O | H | 6-Br |
| 182 | C₆H₅- | -CH(OH)- | H | H | =O | H | 6-Br |
| 185 | p-F-C₆H₄- | -CO- | H | H | =O | H | 6-Cl |
| 186 | p-F-C₆H₄- | -CH(OH)- | H | H | =O | H | 6-Cl |
| 187 | p-Cl-C₆H₄- | -CO- | H | H | =O | H | 6-Cl |
| 188 | p-Cl-C₆H₄- | -CH(OH)- | H | H | =O | H | 6-Cl |
| 189 | p-CH₃O-C₆H₄- | -CO- | H | H | =O | H | 6-Cl |
| 190 | p-CH₃O-C₆H₄- | -CH(OH)- | H | H | =O | H | 6-Cl |
| 191 | C₆H₅- | -CO- | H | H | =O | H | 6-Cl |
| 192 | C₆H₅- | -CH(OH)- | H | H | =O | H | 6-Cl |
| 195 | p-F-C₆H₄- | -CO- | H | H | =O | H | 6-F |
| 196 | p-F-C₆H₄- | -CH(OH)- | H | H | =O | H | 6-F |
| 197 | p-Cl-C₆H₄- | -CO- | H | H | =O | H | 6-F |
| 198 | p-Cl-C₆H₄- | -CH(OH)- | H | H | =O | H | 6-F |
| 199 | p-CH₃O-C₆H₄- | -CO- | H | H | =O | H | 6-F |
| 200 | p-CH₃O-C₆H₄- | -CH(OH)- | H | H | =O | H | 6-F |
| 201 | C₆H₅- | -CO- | H | H | =O | H | 6-F |
| 202 | C₆H₅- | -CH(OH)- | H | H | =O | H | 6-F |
| 205 | p-F-C₆H₄- | -CO- | H | H | =O | H | 5-Br |
| 206 | p-F-C₆H₄- | -CH(OH)- | H | H | =O | H | 5-Br |
| 207 | p-Cl-C₆H₄- | -CO- | H | H | =O | H | 5-Br |
| 208 | p-Cl-C₆H₄- | -CH(OH)- | H | H | =O | H | 5-Br |
| 209 | p-CH₃O-C₆H₄- | -CO- | H | H | =O | H | 5-Br |
| 210 | p-CH₃O-C₆H₄- | -CH(OH)- | H | H | =O | H | 5-Br |

**Table 1-4**

| | | | | | | | |
|---|---|---|---|---|---|---|---|
| 211 | C₆H₅- | -CO- | H | H | =O | H | 5-Br |
| 212 | C₆H₅- | -CH(OH)- | H | H | =O | H | 5-Br |
| 213 | p-F-C₆H₄- | -CO- | H | H | =O | H | 5-Cl |
| 214 | p-F-C₆H₄- | -CH(OH)- | H | H | =O | H | 5-Cl |
| 215 | p-Cl-C₆H₄- | -CO- | H | H | =O | H | 5-Cl |
| 216 | p-Cl-C₆H₄- | -CH(OH)- | H | H | =O | H | 5-Cl |
| 217 | p-CH₃O-C₆H₄- | -CO- | H | H | =O | H | 5-Cl |
| 218 | p-CH₃O-C₆H₄- | -CH(OH)- | H | H | =O | H | 5-Cl |
| 219 | C₆H₅- | -CO- | H | H | =O | H | 5-Cl |
| 220 | C₆H₅- | -CH(OH)- | H | H | =O | H | 5-Cl |
| 221 | p-F-C₆H₄- | -CO- | H | H | =O | H | 5-F |
| 222 | p-F-C₆H₄- | -CH(OH)- | H | H | =O | H | 5-F |
| 223 | p-Cl-C₆H₄- | -CO- | H | H | =O | H | 5-F |
| 224 | p-Cl-C₆H₄- | -CH(OH)- | H | H | =O | H | 5-F |
| 225 | p-CH₃O-C₆H₄- | -CO- | H | H | =O | H | 5-F |
| 226 | p-CH₃O-C₆H₄- | -CH(OH)- | H | H | =O | H | 5-F |
| 227 | C₆H₅- | -CO- | H | H | =O | H | 5-F |
| 228 | C₆H₅- | -CH(OH)- | H | H | =O | H | 5-F |

The compounds of the present invention represented by the aforementioned general formula (I) may have one or more asymmetric carbons, and therefore the compounds may exist as optical isomers. Any of racemates, optical isomers in an optically pure form, and any mixtures of optical isomers fall within the scope of the present invention. Racemates can be resolved into optically pure enantiomers by a method well known to those skilled in the art. Furthermore, diastereomers based on two or more asymmetric carbons, and any mixtures thereof also fall within the scope of the present invention. Acid addition salts, preferably pharmaceutically acceptable acid addition salts, hydrates and any solvates of the compounds of the present invention represented by the aforementioned general formula (I) also fall within the scope of the present invention.

Examples of salts that can be formed with the compounds of the present invention include, for example, inorganic acid salts such as hydrochlorides, hydrobromides, hydroiodides, sulfates, nitrates and phosphates, organic acid salts such as succinates, acetates, glycolates, methanesulfonates and toluenesulfonates, alkali metal salts such as sodium salts and potassium salts, alkaline earth metal salts such as magnesium salts and calcium salts, ammonium salts such as ammonium salts and alkylammonium salts and the like. Examples of solvents possessed by the solvates that can be formed with the compounds of present invention include methanol, ethanol, isopropanol, acetone, ethyl acetate and the like. However, the salts and solvates are not limited to those exemplified above.

The compounds of the present invention have high affinity for the sigma binding site (when a certain compound is referred to as a "sigma ligand" in the specification, it means that the compound has the aforementioned feature). Therefore, the compounds of the present invention are useful as medicaments for therapeutic and/or preventive treatment of various diseases caused or promoted by the nerve controlling function of the sigma ligands in mammals including human, preferably in human. Such diseases include, for example, diseases of central nervous system, gastrointestinal tract and cardiovascular system.

The diseases of central nervous system include, for example, anxiety, depression or emotional abnormality, schizophrenia, narcotic intoxication or narcotic addiction, sharp pain, dyskinesia, cerebrovascular disease, epilepsy, dementia including Alzheimer's disease, Parkinson's syndrome, brain tumor, attention deficit disorder and the like. The gastrointestinal diseases include, for example, irritable bowel syndrome, irritable colon, spastic colon, colitis mucosus, enterocolitis, diverticulitis, dysentery. The diseases of cardiovascular system include, for example, hypertension, arrhythmia, angina pectoris. However, diseases treated by the medicaments of the present invention are not limited to these specific diseases and/or symptoms, and the medicaments can be used for therapeutic and/or preventive treatment of variety of diseases and/or symptoms in which sigma ligands in vivo are involved.

As the active ingredient of the medicaments of the present invention, one or more substances selected from the group consisting of the aforementioned compounds and salts thereof, and hydrates thereof and solvates thereof can be used. The administration route of the medicaments of the present invention is not particularly limited, and they can be administered orally or parenterally. As the medicament of the present invention, the aforementioned substances, per se, may be administered to patients. Preferably, the medicaments may be administered as preparations in the form of pharmaceutical compositions containing the active ingredient and one ore more pharmacologically and pharmaceutically acceptable additives. Examples of the pharmacologically and pharmaceutically acceptable additives include, for example, excipients, disintegrators or disintegrating aids, binders, lubricants, coating agents, colorants, diluents, bases, dissolving agents or dissolving aids, isotonic agents, pH modifiers, stabilizers, propellants, adhesives and the like. Examples of pharmaceutical preparations suitable for oral administration include, for example, tablets, capsules, powders, subtilized granules, granules, liquids, syrups and the like. Examples of pharmaceutical preparations suitable for parenteral administration include, for example, injections, drip infusions, ointments, creams, transmucosal preparations, eye drops, ear drops, inhalants, suppositories and the like. However, the forms of the preparations are not limited to these examples.

For the preparations suitable for oral administration, for example, excipients such as glucose, lactose, D-mannitol, starch and crystalline cellulose; disintegrating agents or disintegrating aids such as carboxymethylcellulose, starch and calcium carboxymethylcellulose; binders such as hydroxypropylcellulose, hydroxypropylmethylcellulose, polyvinylpyrrolidone and gelatin; lubricants such as magnesium stearate and talc; coating agents such as hydroxypropylmethylcellulose, sucrose, polyethylene glycol and titanium oxide; and bases such as Vaseline, liquid paraffin, polyethylene glycol, gelatin, kaolin, glycerin, purified water and hard fat may be used as additives. For the preparations suitable for injection or drip infusion, for example, dissolving agents or dissolving aids that can constitute aqueous injections or injections that are dissolved upon use such as distilled water for injections, physiological saline and propylene glycol; isotonic agents such as glucose, sodium chloride, D-mannitol and glycerin; pH modifiers such as inorganic acids, organic acids, inorganic bases and organic bases and so forth may be used as additives for pharmaceutical preparations.

The doses of the medicament of the present invention should be suitably determined depending on a type of a disease to be treated, purpose of preventive or therapeutic treatment, the age, body weight and conditions of a patient and the like. Oral doses for adults are generally within the range of about from 0.05 to 500 mg per day. In general, the medicament in the daily dose may be administered as two or more divided portions in a day, or may be administered every few days.

Methods for preparing the compounds of present invention represented by formula (I) are not particularly limited. For example, the compounds can be prepared by any one of the methods described below. The preparations of preferred compounds falling within the compounds of present invention will be more specifically explained in the examples of the specification. Accordingly, those skilled in the art can prepare any of the compounds of present invention falling within the formula (I) by referring to the general explanations which follows and specific explanations of the examples, and suitably modifying or altering starting materials, reaction conditions, reagents and the like, if necessary.

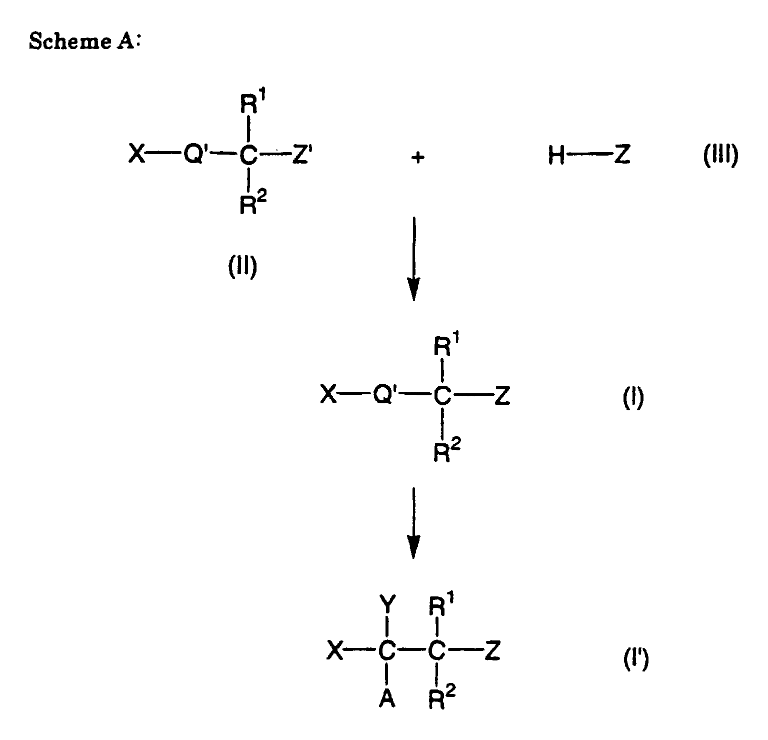

A compound of the formula (II) (in the formula, Z' represents a halogen atom such as chlorine atom, bromine atom or iodine atom, or tosylate or mesylate or the like, and Q' represents a single bond or -CO-) can be reacted with a nucleophilic amino derivative represented by the formula H-Z (III) (Z has the same meaning as that defined above) to obtain a corresponding compound of the formula (I). This reaction may usually be performed in a polar solvent such as dimethylformamide, ethanol or acetonitrile in the presence of a base such as triethylamine, sodium hydrogencarbonate or potassium carbonate.

When Q' is -CO-, the amino-keto derivative of the formula (I) can be reduced to obtain a hydroxy derivative of the formula (I') wherein A is hydroxyl group and Y is hydrogen atom. In general, the reduction can be performed at room temperature in an organic solvent such as ethanol, methanol or tetrahydrofuran using sodium borohydride. The amino-keto derivative of the formula (I) can also be reacted with an organic metal reagent such as Y-MgBr to obtain a compound of the formula (I') wherein A is hydroxyl group.

The acyloxy and alkoxyl compounds can be prepared by usual methods starting from a free hydroxy derivative. An O-alkyl derivative can be prepared by solvolysis of a sulfonyl ester intermediate [Advanced Organic Chemistry, J. March., John Wiley & Sons, New York, pp.264-317, 1985]. A chiral ether can also be obtained by solvolysis of a chiral sulfonyl ester derivative such as camphorsulfonate. An oxime of the keto derivative of the formula (I) wherein Q' is -CO- can be prepared by an oxime preparation such as those described in Organic Functional Group Preparation Vol. III, S.R. Sandler and W. Karo, Academic Press, London, pp.430-481.

As the compound of formula (II), commercially available compounds may be used, or the compounds can be obtained by halogenation using a methyl group (when Q is a single bond) or a methyl ketone group (when Q is -CO-) as a starting material or a similar method. As the syntheses of benzoxazolidine derivative and benzisothioxazoline derivative, examples includes the methods described in known references [H. Uno, M. Kurokawa, K. Natsuka, Y. Yamato and H. Nishimura, Chem. Pharm. Bull., 24 (4), pp.632-643, 1976; H. Uno and M. Kurokawa, Chem. Pharm. Bull., 26 (12), pp.3888-3891, 1978].

A compound of the formula (III) wherein D is NR⁸, which is used as a starting material for the aforementioned Scheme A, can be prepared by the method shown in the following scheme. In the scheme, the compound having a piperidine ring is shown as the target derivative. However, non-cyclic compounds can also be prepared in a similar manner. The substituent P of the compounds shown in the scheme represents a protective group for amino group such as those described in Protective Groups in Organic Synthesis [T.W. Greene and P.G.M. Wuts, John Wiley & Sons, New York, 1991], and the protective group is readily eliminated to give a piperidine compound of the formula (III).

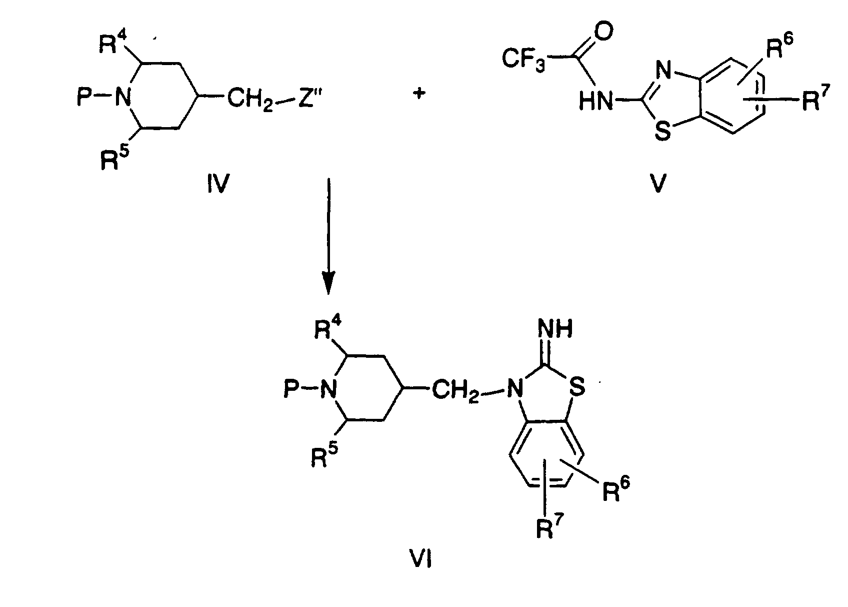

N-alkylation of a trifluoroacetamide precursor of the formula (V) can be performed by using an activated derivative of the formula (IV) (in the formula (IV), Z'' represents a halogen atom such as chlorine atom, bromine atom and iodine atom, or tosylate or mesylate or the like). The N-alkylation can preferably be performed at a temperature of 50°C or higher in dimethylformamide in the presence of sodium hydride as a base. If desired, an iodide salt (e.g., NaI, KI) of one molar equivalence or less may be added to the reaction as a catalyst. The reaction may also be performed by the phase-transfer method in the presence of a catalytic amount of tetrabutylammonium hydrogensulfate or other suitable salts by using sodium hydroxide or potassium hydroxide as a base and toluene as a solvent. The N-alkylation of a derivative of the formula (V) can be directly performed by using a hydroxy derivative of the formula (IV) (in the formula (IV), Z'' = OH) in accordance with the Mitsunobu reaction (O. Mitsunobu, Synthesis, pp. 1-28, 1981).

The trifluoroacetamide of the formula (V) can be prepared by allowing trifluoroacetic anhydride to react with a 2-aminobenzothiazole derivative in the presence of one equivalence of a base. As the 2-aminobenzothiazole, commercially available compounds may be used, or the compounds may be synthesized by the method of R. Schubart et al. [R. Schubart, H. Kropf and F. Wohrle in Methoden der Organischen Chemie, Houben-Weil, Stuttgart-New-York: Thieme, Band E-8b, pp.865-1062, 1994]. The imino group of the derivative of the formula (VI) can be converted into a carboxamide derivative, urea derivative or carbamoyl derivative by known methods. A 2-alkyliminobenzothiazoline derivative can be obtained by reduction of a carboxamide derivative of the formula (VI). Examples of preferred reducing agents include borohydride salts, borane reagents. The sulfonamide derivative, sulfonylurea derivative and sulfamoyl derivative can be similarly prepared from a compound of the formula (VI).

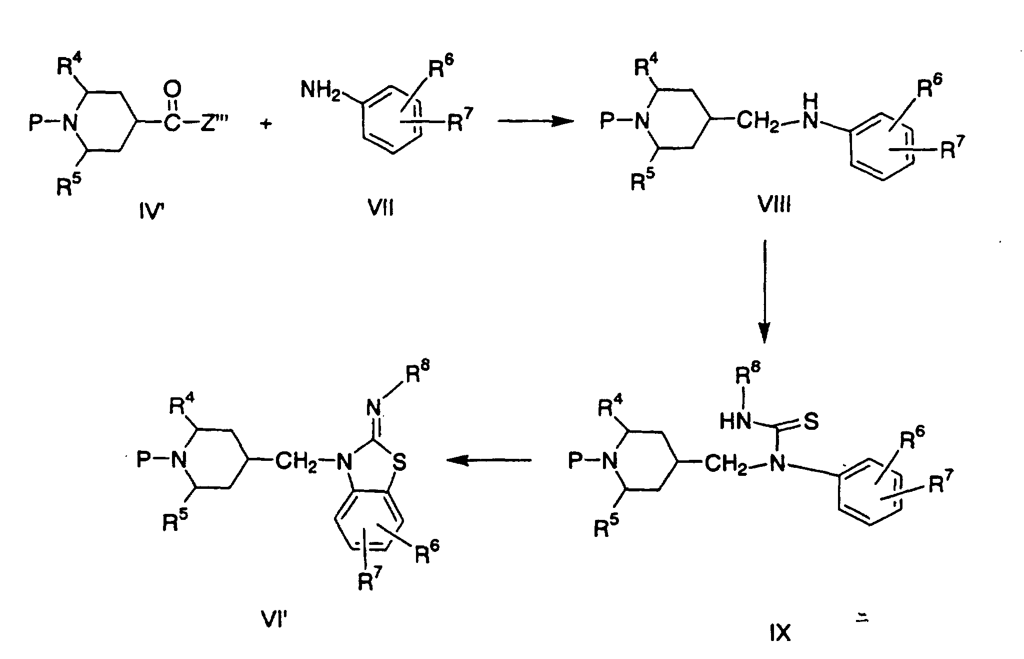

Another method for preparing a benzothiazoline derivative represented by the general formula (VI') is shown in the above scheme. Acylation of the aniline derivative of the formula (VII) can be performed according to a known method by using a compound of the formula (IV') (in the formula (IV'), Z''' represents hydroxyl group, a halogen atom such as chlorine atom, mesylate or the like). Reduction of the resulting amide compound can be performed to obtain an amino derivative represented by the formula (VIII) in an organic solvent such as tetrahydrofuran by using a reducing agent such as lithium aluminium hydride. The thiourea derivative represented by the formula (IX) can be synthesized by the reaction of an amino derivative of the formula (VIII) and an isothiocyanic acid ester.

The synthesis of the benzothiazoline derivative of the formula (VI') by oxidative cyclization of the thiourea derivative of the formula (IX) can be performed by methods described in the literature [R. Schubart, H. Kropf and F. Wohrle in Methoden der Organischen Chemie, Houben-Weil, Stuttgart-New-York : Thieme, Band E-8b, pp.865-1062, 1994]. When R⁸ is hydrogen atom, a benzothiazoline derivative of the formula (VI') (in the formula, R⁸ = H) can be obtained by treating an intermediate of the formula (VIII) with a thiocyanate salt such as sodium thiocyanate, potassium thiocyanate or ammonium thiocyanate and bromine in a solvent such as methanol and acetic acid.

When D is oxygen atom in the compound of the formula (III) as the starting material in the aforementioned Scheme A, the compound can be synthesized by the method shown as shown in the scheme below. In the scheme, the compound having a piperidine ring is shown as the target derivative. However, non-cyclic compounds can also be produced in a similar manner. The substituent P of the compounds shown in the scheme represents a protective group for amino group such as those described in Protective Groups in Organic Synthesis [T.W. Greene and P.G.M. Wuts, John Wiley & Sons, New York, 1991], and the protective group is readily eliminated to give a piperidine compound of the formula (III).

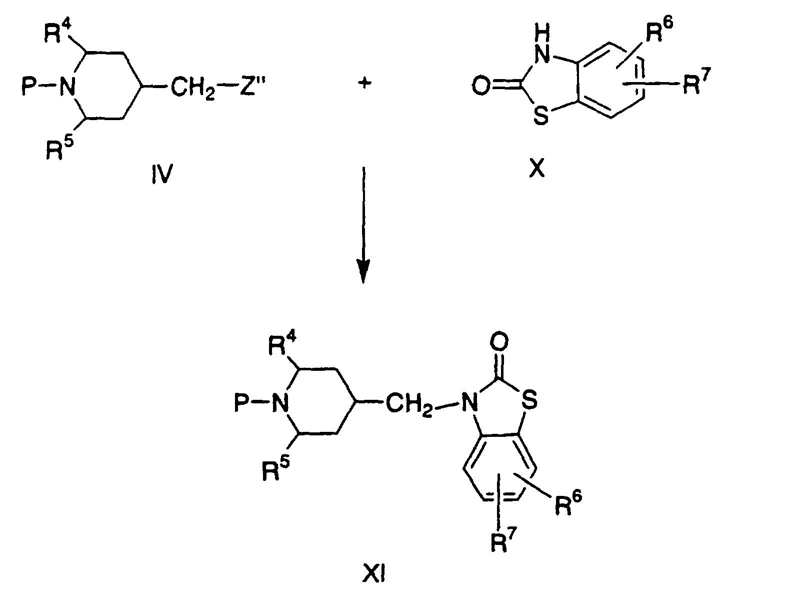

N-alkylation of the 2-oxobenzothiazoline derivative of the formula (X) can be performed by using an activated derivative of the formula (IV) (in the formula (IV), Z'' represents a halogen atom such as chlorine atom, bromine atom and iodine atom, or tosylate or mesylate or the like). The N-alkylation reaction can preferably be performed at room temperature or a higher temperature in dimethylimidazolidinone in the presence of sodium hydride as a base. If desired, an iodide salt (e.g., NaI, KI) of one molar equivalence or less may be added to the reaction as a catalyst. This reaction can also be performed at a temperature of 50°C or higher by using dimethylformamide as a solvent and potassium carbonate as a base. The N-alkylation of a derivative of the formula (V) can be directly performed by using a hydroxy derivative of the formula (IV) (in the formula (IV), Z'' = OH) in accordance with the Mitsunobu reaction (O. Mitsunobu, Synthesis, pp. 1-28, 1981).

As the 2-oxobenzothiazoline derivative of the formula (X), commercially available compounds may be used, or the compounds can be synthesized by converting a 2-aminobenzothiazole as a starting material into a 2-aminothiophenol derivative, and then reacting with 1,1'-carbonyl-bis-1H-imidazole preferably in tetrahydrofuran according to the method of R.R. Gupta et al. (Synthetic Communications, 17(2). 229-240 (1987)).

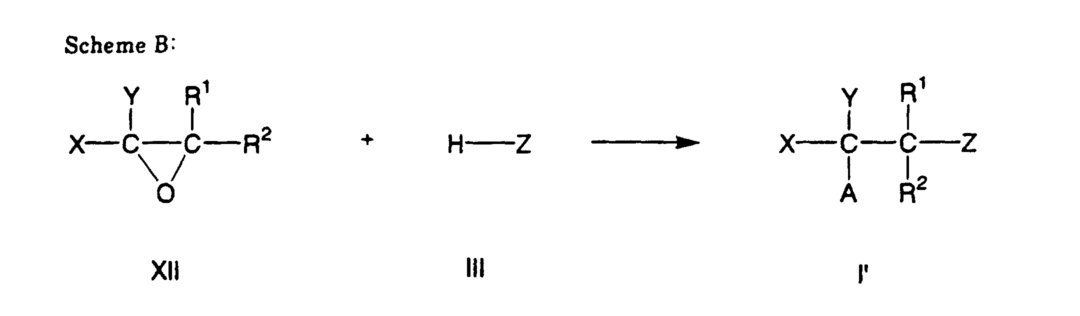

Another synthetic route of the compound of the formula (I') wherein A is -OH is shown in Scheme B. Aminolysis of epoxy derivatives is usually performed by heating reagents in a solvent such as ethanol [N.S. Isaacs and R.E. Parker, J., pp.3497-3504, 1960]. A metal salt may be used as a catalyst [M. Chini, P. Crotti and F. Macchia, Tetrahedron Letters, 31 (32), pp.4661-4664, 1990]. The epoxy derivative of the formula (XII) can be prepared by oxidization of alkenyl derivative or dehydrohalogenation of halohydrin compound, or by allowing a carboxyaldehyde to react with dimethylsulfonium methylide [T. Kutsuma, I. Nagayama, T. Ozaki., T. Sakamoto, S. Akaboshi, Heterocycles, 8, pp.397-401, 1977]. Where an optically active epoxy derivative of the formula (XII) is used as the starting material, an optically active aminoalcohol compound of the formula (I') can be obtained.

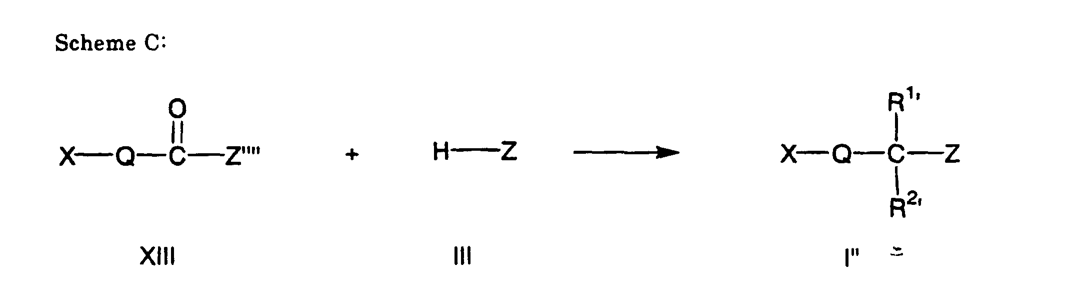

A compound represented by the formula (I'') wherein R^{1'} = R^{2'} = H can also be obtained by condensation of a compound of the formula (XIII) (in the formula (XIII), Z'''' represents hydroxyl group, a halogen atom such as chlorine atom, or mesylate or the like) and an amino derivative of the formula (III), followed by reduction of the resulting amide intermediate. The condensation is performed in the presence (when Z is hydroxyl group) or absence of a condensing agent such as dicyclohexylcarbodiimide depending on the type of Z''''. The reduction can be performed by using a reducing agent such as borane dimethyl sulfide and aluminium hydride.

Compounds used as starting materials in the aforementioned methods may be a racemate or a desired optically active substance, and a corresponding racemate or an optical active compound can be produced. It is also possible to produce a desired optically active substance by optical resolution according to known conventional methods. Basic compounds of the formula (I) can form acid addition salts, preferably pharmacologically acceptable salts, with various inorganic and organic acids. These salts can be easily prepared by treating a basic compound of the formula (I) with a mineral acid or an organic acid in a suitable organic solvent such as methanol, ethanol, isopropanol, or ethyl acetate.

### Examples

The present invention will be explained more specifically with reference to the following examples. However, the scope of the present invention is not limited by these examples.

### Comparative Example 1:

### (R,S)-1-(1-Adamantyl)-2-[4-(6-chloro-2-iminobenzothiazolin-3-ylmethyl)piperidin-1-yl] ethanol dihydrochloride

### a) Piperidine-1,4-dicarboxylic acid 1-tert-butyl ester

To a solution of isonipecotic acid (60 g, 464 mmol) and sodium hydroxide (37.6 g, 940 mmol) in water (86 ml) and tert-butyl alcohol (176 ml), di-tert-butyl dicarbonate (101.1 g, 464 mmol) was added with stirring. After the addition was completed, the mixture was added with tert-butyl alcohol (100 ml), and stirred at room temperature for 3 hours, and the resulting solution was diluted with water (200 ml) and extracted twice with pentane (150 ml). The aqueous layer was acidified with potassium hydrogensulfate (70 g) with cooling, and extracted with ethyl acetate. After an ordinary work-up, the title compound was obtained as white powder (102.3 g, yield: 96%). Melting point: 144-146°C

### b) tert-Butyl 4-(hydroxymethyl)piperidine-1-carboxylate

To the compound obtained in Example 1 (a) (13.74 g, 60 mmol) dissolved in 1,2-dimethoxyethane (60 ml), N-methylmorpholine (6.66 ml, 60 mmol) and isobutyl chloroformate (8.16 ml, 60 mmol) were successively added with cooling (-15°C), and the precipitates were removed by filtration after 10 minutes, and washed with 60 ml of 1,2-dimethoxyethane. The filtrate was carefully added dropwise with 30 ml of aqueous solution of sodium borohydride (3.42 g, 90 mmol) with cooling on an ice-salt bath. After the addition was completed, the mixture was stirred for 45 minutes, and added with water (800 ml). The target alcoholic compound was extracted with ethyl acetate (400 ml), and the organic layer was washed successively with 0.05 N hydrochloric acid, water and saturated aqueous sodium hydrogencarbonate. After the layer was dried, the solvent was evaporated under reduced pressured to obtain the title compound as colorless oil (9.8 g, yield: 75%). When the product was left stand at room temperature, the oil gave white solid. Melting point: 74-76°C.

### c) tert-Butyl 4-(methanesulfonyloxymethyl)piperidine-1-carboxylate

To a solution of the compound obtained in Example 1 (b) (26.8 g, 125 mmol) dissolved in dry tetrahydrofuran (350 ml) and triethylamine (19.1 ml, 137.5 mmol), methanesulfonyl chloride (11.3 ml, 137.5 mmol) was added dropwise with ice cooling. After the reaction mixture was stirred for 20 minutes, the mixture was added with water and extracted with ethyl acetate. The organic layer was washed with saturated aqueous sodium hydrogencarbonate. After an ordinary work-up, the title compound was obtained as white solid (yield: quantitative). Melting point: 72-74°C.

### d) 6-Chloro-2-trifluoroacetylaminobenzothiazole

2-Amino-6-chlorobenzothiazole (51.3 g, 278 mmol) and triethylamine (44 ml, 306 mmol) were dissolved in a mixed solvent of ethyl acetate (450 ml) and tetrahydrofuran (350 ml), and added dropwise with trifluoroacetic anhydride (43.2 ml, 306 mmol) with ice cooling. After the addition was completed, the mixture was warmed up to room temperature and stirred for 1 hour. Then, the mixture was added with water (500 ml), extracted with ethyl acetate, and the organic layer was washed successively with water and saturated aqueous sodium hydrogencarbonate, and dried over magnesium sulfate. The solvent was evaporated under reduced pressured, and the resulting solid was washed with a mixed solvent of ethyl acetate and hexane (3:7) to obtain the title compound as white solid (69.6 g, yield: 89%).
Melting point: 232-234°C.

### e) tert-Butyl 4-(6-chloro-2-trifluoroacetyliminobenzothiazolin-3-ylmethyl)piperidine-1-carboxylate

The compound obtained in Example 1 (d) (28.05 g, 100 mmol) was dissolved in dimethylformamide (300 ml) and added to sodium hydride (4 g, 100 mmol, 60% content in oil) suspended in dimethylformamide (80 ml). The reaction mixture was stirred for 20 minutes at room temperature under nitrogen atmosphere, and added with a solution of the methanesulfonate obtained in Example 1 (c) (29.3 g, 100 mmol) dissolved in dimethylformamide (200 ml) and sodium iodide (5 g, 33 mmol). The reaction mixture was stirred at 85°C for 28 hours. After the reaction mixture was cooled, the mixture was poured into water, extracted with ethyl acetate, and subjected to an ordinary work-up to obtain brown solid. The resulting solid was washed with a mixed solvent of ethyl acetate and hexane (3:7) to give the title compound as white solid at a yield of 63%. Melting point: 197-199°C. Rf = 0.45 (ethyl acetate/hexane = 2:8).

### f) tert-Butyl 4-(6-chloro-2-iminobenzothiazolin-3-ylmethyl)piperidine-1-carboxylate

The compound obtained in Example 1 (e) (27.4 g, 58 mmol) was dissolved in methanol (400 ml), and the solution was added with 10% solution of potassium carbonate in a mixture of methanol and water (7:3, 200 ml). The reaction mixture was stirred at room temperature for 1 hour, and then the solvent was evaporated under reduced pressure. The resulting residue was added with water, and extracted with ethyl acetate. The organic layer was dried over sodium sulfate, and the solvent was evaporated to obtain the title compound as white solid (yield: quantitative).
Melting point: 142-144°C. Rf = 0.3 (ethyl acetate/hexane = 3:7).
¹H-NMR (CDCl₃): 7.23 (d, 1H), 7.16 (dd, 1H), 6.74 (d, 1H), 4.1 (m, 2H), 3.8 (d, 2H), 2.65 (m, 2H), 2.1 (m, 1 H), 1.64 (m, 2H), 1.45(s, 9H), 1.25 (m, 2H)

### g) 4-(6-Chloro-2-iminobenzothiazolin-3-ylmethyl)piperidine

The compound obtained in Example 1 (f) (7.7 g, 19 mmol) was dissolved in ethyl acetate (80 ml), and added with 4 N hydrochloric acid solution in ethyl acetate (15 ml, 60 mmol). The reaction mixture was stirred for 4 hours under reflux. After the reaction mixture was cooled, the resulting solid was filtered and washed with ethyl acetate to obtain hydrochloride of the title compound as white solid. Melting point: 296-298°C. The resulting hydrochloride was treated with 0.5 N aqueous sodium hydroxide, and extracted with methylene chloride. Then, the organic layer was dried over sodium sulfate, and the solvent was evaporated to obtain the title compound as white solid (4.4 g, yield: 89%). Melting point: 115-117°C.
¹H-NMR (CDCl₃): 7.27 (d, 1H), 7.17 (dd, 1H), 6.74 (d, 1H), 3.78 (d, 2H), 3.06 (m, 2H), 2.58 (dt, 2H), 2.05 (m, 1H), 1.64 (m, 3H), 1.26 (dq, 2H)

### h) 3-[[1-[2-(1-Adamantyl)-2-oxoethyl]-4-piperidinyl]methyl]-6-chloro-2-iminobenzothiazoline

A solution of the compound obtained in Example 1 (g) (1.6 g, 5.7 mmol), 1-(bromoacetyl)adamantane (1.54 g, 6 mmol) and potassium carbonate (866 mg, 6.3 mmol) in acetonitrile (80 ml) was stirred under reflux for 2 hours. After the mixture was cooled, the mixture was added with water and extracted with methylene chloride. The organic layer was dried over sodium sulfate, and the solvent was evaporated to obtain the title compound as foamy powder (yield: quantitative). Rf = 0.55 (methylene chloride/methanol = 9:1).

### i) (R,S)-1-(1-Adamantyl)-2-[4-(6-chloro-2-iminobenzothiazolin-3-ylmethyl)piperidin-1-yl]ethanol dihydrochloride

The compound obtained in Example 1 (h) (2.9 g, 5.7 mmol) was dissolved in a mixed solvent of ethanol (50 ml) and tetrahydrofuran (30 ml), and added with sodium borohydride (1.2 g, 31.5 mmol). After the reaction mixture was stirred at room temperature for 4 hours, the solvent was evaporated under reduced pressure, and the residue was added with water and extracted with ethyl acetate. The organic layer was washed twice with saturated aqueous sodium hydrogencarbonate and dried over sodium sulfate. The solvent was evaporated under reduced pressure, and the residue was purified by silica gel column chromatography (eluent: methylene chloride/methanol = 9:1, Rf = 0.4) to obtain the free form of the title compound as white solid (2.1 g, yield: 80%). Melting point: 120-123°C.
¹H-NMR (CDCl₃): 7.22 (d, 1 H), 7.17 (dd, 1H), 6.74 (d, 1H), 3.78 (d, 2H), 3.47 (s, 2H), 3.17 (dd, 1H), 3.0 (d, 1H), 2.80 (d, 1H), 2.24 (m, 3H), 1.95-1.38 (m, 20H)

The aforementioned free compound was dissolved in ethanol, and added with 4 N hydrochloric acid solution in ethyl acetate to obtain the title compound as white solid (yield: 69%). Melting point: > 250°C.
¹H-NMR (DMSO-d₆): 11.04 (m, 2H), 9.61 (m, 1H), 8.13 (d, 1H), 7.88-7.77 (dd, 1H), 7.57 (dd, 1H), 5.33 (m, 1H), 4.55-4.36 (dd, 2H), 3.60-2.90 (m, 8H), 2.25-1.48 (m, 20H)

| Elemental analysis (C₂₅H₃₆Cl₃N₃OS + H₂O) | | | | | |
|---|---|---|---|---|---|
| Calcd. | C, 54.49%; | H, 6.95%; | N, 7.63%; | Cl, 19.30%; | S, 5.82% |
| Found | C, 54.64%; | H, 6.95%; | N, 7.58%; | Cl, 19.45%; | S, 5.63% |

### Example 2: 3-[[1-[2-(4-Fluorophenyl)-2-oxoethyl]-4-piperidinyl]methyl]-6-chloro-2-iminobenzothiazoline dihydrochloride (Compound 103 in Table 1)

In the presence of sodium iodide (200 mg, 1.3 mmol), the compound obtained in Example 1 (g) (1.6 g, 5.7 mmol) and 2-chloro-4'-fluoroacetophenone (984 mg, 5.7 mmol) were reacted in the same manner as mentioned in Example 1 (h), and the product was purified by silica gel column chromatography (eluent: methylene chloride/methanol = 9:1, Rf = 0.40) to obtain the free form of the title compound as foamy powder (2.1 g, yield: 80%).
¹H-NMR (CDCl₃): 8.09-8.04 (m, 2H), 7.21-7.06 (m, 4H), 6.75 (d, 1 H), 3.80 (d, 2H), 3.70 (s, 2H), 2.98-2.93 (m, 2H), 2.15-2.06 (m, 2H), 2.06-1.96 (m, 1H), 1.70-1.65 (m, 2H), 1.56-1.45 (m, 2H)

The above free compound was dissolved in ethanol, and the solution was added with 4 N hydrochloric acid solution in ethyl acetate to obtain the title compound as white solid. Melting point: 240-245°C.
¹H-NMR (DMSO-d₆): 11.02 (m, 2H), 10.06 (m, 1H), 8.25-7.76 (m, 4H), 7.60 (m, 1H), 7.49-7.43 (m, 2H), 5.30-4.90 (m, 2H), 4.42 (m, 2H), 3.35-3.01 (m, 4H), 2.19 (m, 2H), 1.80-1.65 (m, 3H)

### Example 3: (R,S)-1-(4-Fluorophenyl)-2-[4-(6-chloro-2-iminobenzothiazolin-3-ylmethyl)-piperidin-1-yl]ethanol dihydrochloride (Compound 104 in Table 1)

The free form of the compound obtained in Example 2 was used to perform a reaction in the same manner as described in Example 1 (i), and the product was purified by silica gel column chromatography (eluent: methylene chloride/methanol = 9:1, Rf = 0.45) to obtain the free form of the title compound as white solid (yield: 75%).
Melting point: 186-188°C.
¹H-NMR (CDCl₃): 7.34-7.16 (m, 4H), 7.04-6.98 (m, 2H), 6.76 (d, 1H), 4.70 (dd, 1H), 3.82 (d, 2H), 3.14 (d, 1H), 2.80 (d, 1H), 2.49-2.34 (m, 3H), 1.99-1.96 (dt, 2H), 1.70 (m, 2H), 1.50 (m, 2H)

The above free compound was dissolved in ethanol, and added with 4 N hydrochloric acid solution in ethyl acetate to obtain the title compound as white solid (yield: 83%). Melting point: 225°C (decomposition).
¹H-NMR (DMSO-d₆): 10.95 (m, 2H), 10.05 (m, 1H), 8.13 (d, 1 H), 7.76 (m, 1H), 7.59-7.45 (m, 3H), 7.17 (m, 2H), 6.25 (m, 1H), 5.18 (m, 1H), 4.40 (m, 2H), 3.60-2.90 (m, 7H), 2.25-1.85(m, 4H)

| Elemental analysis (C₂₁H₂₅Cl₃FN₃OS + H₂O) | | | | | | |
|---|---|---|---|---|---|---|
| Calcd. | C, 49.37%; | H, 5.3%; | N, 8.22%; | Cl, 20.8%; | F, 3.72%; | S, 6.27% |
| Found | C, 49.1%; | H, 4.9%; | N, 8.1%; | Cl, 21.2%; | F, 3.6%; | S, 6.2% |

### Example 4: 3-[[1-[2-(4-Fluorophenyl)-2-oxoethyl]-4-piperidinyl]methyl]-6-chloro-2-methyliminobenzothiazoline dihydrochloride (Compound 115 in Table 1)

### a) tert-Butyl 4-(6-chloro-2-formyliminobenzothiazolin-3-ylmethyl)piperidine-1-carboxylate

Acetic formic anhydride was prepared according to the method of S. Krishnamurthy et al. (Tetrahedron Lett., 23 (33), pp.3315-3318, 1982). Acetic anhydride (13.5 g, 130 mmol) was added dropwise with 98% formate (7.5 g, 160 mmol) at 0°C, and then the mixture was heated at 50-60°C for 2 hours with stirring. The mixture was cooled to room temperature and added with tetrahydrofuran (60 ml). The mixture was then cooled to -30°C, and added dropwise with a solution of the compound obtained in Example 1 (f) (16.7 g, 44 mmol) in tetrahydrofuran (80 ml). After the reaction mixture was stirred for 30 minutes at -30°C, the mixture was added with ethyl acetate and the warmed up to room temperature. Then, the reaction mixture was added with water to separate an organic layer. The resulting organic layer was washed with water and saturated aqueous sodium hydrogencarbonate. The organic layer was dried over sodium sulfate, and the solvent was evaporated to obtain the title compound as white solid (16.2 g, 90%). Melting point: 168-170°C. Rf = 0.4 (ethyl acetate/hexane 4:6).

### b) tert-Butyl 4-(6-chloro-2-methyliminobenzothiazolin-3-ylmethyl)piperidine-1-carboxylate

The compound obtained in Example 4 (a) (12.5 g, 30.5 mmol) was suspend in tetrahydrofuran (45 ml), and the suspension was slowly added with a solution of borane in dimethyl sulfide (7.5 ml, 75 mmol) at 0°C under nitrogen atmosphere. After the addition was completed, the reaction mixture was warmed up to room temperature and stirred for 1.5 hours. The mixture was then slowly added with a mixed solvent of tetrahydrofuran (120 ml) and methanol (12 ml) with ice cooling. Then, the reaction mixture was stirred at 40°C for 30 minutes, and added with 4 N hydrochloric acid solution in ethyl acetate (9 ml) with ice cooling. The resulting solid was filtered, and washed with tetrahydrofuran to obtain hydrochloride of the title compound (8.7 g, yield: 66%). Melting point: 180-182°C.

Preparation of the free form of the above compound was performed in the same manner as described in Example 1 (g). The title compound was obtained as white solid (yield: quantitative). Melting point: 106-108°C. Rf = 0.3 (ethyl acetate/methylene chloride = 2:8).
¹H-NMR (CDCl₃): 7.31 (d, 1H), 7.16 (dd, 1H), 6.74 (d, 1H), 4.1 (m, 2H), 3.76 (d, 2H), 3.07 (s, 3H), 2.64 (m, 2H), 2.07 (m, 1H), 1.64 (m, 2H), 1.45 (s, 9H), 1.22 (dq, 2H)

### c) 4-(6-Chloro-2-methyliminobenzothiazolin-3-ylmethyl)piperidine

Using the compound obtained in Example 4 (b), a reaction was performed in the same manner as described in Example 1 (g) to obtain the title compound as white solid (yield: 92%). Melting point: 106-108°C
¹H-NMR (CDCl₃): 7.30 (d, 1H), 7.17 (dd, 1H), 6.75 (d, 1H), 3.75 (d, 2H), 3.06 (m, 5H), 2.54 (dt, 2H), 2.04 (m, 1H), 1.64 (m, 3H), 1.23 (dq, 2H)

### d) 3-[[1-[2-(4-Fluorophenyl)-2-oxoethyl]-4-piperidinyl]methyl]-6-chloro-2-methyl-iminobenzothiazoline dihydrochloride

In the presence of sodium iodide (675 mg, 4.5 mmol), the compound obtained in Example 4 (c) (5.5 g, 18.6 mmol) and 2-chloro-4'-fluoroacetophenone (3.45 g, 20 mmol) were used to perform a reaction in the same manner as described in Example 1 (h), and the product was purified by silica gel column chromatography (eluent: methylene chloride/methanol = 19:1, Rf = 0.30) to obtain the free form of the title compound as foamy powder (4.0 g, yield: 50%).
¹H-NMR (CDCl₃): 8.07 (m, 2H), 7.30 (d, 1H), 7.17-6.71 (m, 3H), 6.75 (d, 1H), 3.77 (d, 2H), 3.69 (s, 2H), 3.06 (s, 3H), 2.94 (m, 2H), 2.09 (dt, 2H), 1.95 (m, 1H), 1.61 (m, 2H), 1.47 (dq, 2H)

The above free compound was dissolved in ethanol, and the solution was added with 4 N hydrochloric acid solution in ethyl acetate to obtain the title compound as white solid (yield: 66%). Melting point: 228-234°C.
¹H-NMR (DMSO-d₆): 11.87 (m, 1H), 10.16 (m, 1H), 8.25 (m, 2H), 8.06-7.82 (m, 2H), 7.64 (m, 1H), 7.42 (m, 2H), 5.33 (s, 1H), 5.02 (s, 1H), 4.56 (m, 2H), 3.60-3.05 (m, 10H), 2.43-1.71(m, 4H)

| Elemental analysis (C₂₂H₂₅Cl₃FN₃OS + 1.3H₂O) | | | | | | |
|---|---|---|---|---|---|---|
| Calcd. | C, 50.02%; | H, 5.26%; | N, 7.95%; | Cl, 20.13%; | F, 3.59%; | S, 6.07% |
| Found | C, 49.73%; | H, 4.85%; | N, 7.80%; | Cl, 20.43%; | F, 3.38%; | S, 5.79% |

### Example 5: (R,S)-1-(4-Fluorophenyl)-2-[4-(6-chloro-2-methyliminobenzothiazolin-3-yl-methyl)piperidin-1-yl)ethanol dihydrochloride (Compound 116 in Table 1)

The free compound of the compound obtained in Example 4 was used to perform a reaction in the same manner as described in Example 1 (i), and the resulting solid was washed with ethanol to obtain the free form of the title compound as white solid (yield 79%). Melting point: 183-187°C. Rf = 0.55 (methylene chloride/methanol = 9:1).
¹H-NMR (CDCl₃): 7.31 (m, 3H), 7.18 (dd, 1H), 7.04-6.98 (m, 2H), 6.76 (d, 1H), 4.66 (dd, 1H), 3.80 (d, 2H), 3.07 (d+s, 4H), 2.80 (d, 1H), 2.49-2.26 (m, 3H), 2.01-1.94 (m, 2H), 1.66 (m, 2H), 1.43(m, 2H)

The above free compound was dissolved in ethanol/ethyl acetate, and the solution was added with 4 N hydrochloric acid solution in ethyl acetate to obtain the title compound as white solid (yield: 78%). Melting point: 218-232°C.
¹H-NMR (DMSO-d₆): 11.7 (m, 1H), 10.1 (m, 1H), 8.25 (s, 1H), 7.90-7.43 (m, 4H), 7.17 (m, 2H), 6.25 (m, 1H), 5.15 (m, 1H), 4.54 (m, 2H), 3.62-2.90 (m, 10H), 2.18-1.78(m, 4H)

| Elemental analysis (C₂₂H₂₇Cl₃FN₃OS + H₂O) | | | | | | |
|---|---|---|---|---|---|---|
| Calcd. | C, 50.34%; | H, 5.57%; | N, 8.00%; | Cl, 20.26%; | F, 3.62%; | S, 6.10% |
| Found | C, 49.94%; | H, 5.22%; | N, 7.82%; | Cl, 20.23%; | F, 3.46%; | S, 6.03% |

### Example 6: (R,S)-1-(3,4-Difluorophenyl)-2-[4-(6-chloro-2-iminobenzothiazolin-3-yl-methyl)piperidin-1-yl]ethanol dihydrochloride

### a) 3-[[1-[2-(3,4-Difluorophenyl)-2-oxoethyl]-4-piperidinyl]methyl]-6-chloro-2-imino-benzothiazoline

The compound obtained in Example 1 (g) (1.69 g, 6.0 mmol) and 2-bromo-3',4'-difluoroacetophenone (1.37 g, 6.0 mmol) were used to perform a reaction in the same manner as described in Example 1 (h), and the product was purified by silica gel column chromatography (eluent: methylene chloride/methanol = 19:1, Rf = 0.40) to obtain the title compound as an oil (yield: 69%).

### b) (R,S)-1-(3,4-Difluorophenyl)-2-[4-(6-chloro-2-iminobenzothiazolin-3-ylmethyl)-piperidin-1-yl]ethanol dihydrochloride

The compound obtained in Example 6 (a) was used to perform a reaction in the same manner as described in Example 1 (i), and the resulting solid was washed with ethanol to obtain the free form of the title compound as white solid yield: 78%). Melting point: 146-148°C. Rf = 0.45 (methylene chloride/methanol = 9:1).
¹H-NMR (CDCl₃): 7.23-7.02 (m, 5H), 6.76 (d, 1H), 4.64 (dd, 1H), 3.82 (d, 2H), 3.10 (d, 1H), 2.80 (d, 1H), 2.50-2.28 (m, 3H), 2.0 (m, 2H), 1.71 (m, 2H), 1.46 (m, 2H)

The above free compound was dissolved in ethyl acetate, and added with 4 N hydrochloric acid solution in ethyl acetate to obtain the title compound as light yellow solid (yield: 83%). Melting point: 195-197°C.
¹H-NMR (DMSO-d₆): 11.05 (m, 2H), 10.1 (m, 1H), 8.14 (d, 1H), 7.89-7.28 (m, 6H), 6.38 (m, 1H), 5.17 (m, 1H), 4.50 (m, 2H), 3.66-2.96 (m, 7H), 2.16-1.78 (m, 4H)

### Example 7: (R,S)-1-(3-Methoxyphenyl)-2-[4-(6-chloro-2-iminobenzothiazolin-3-yl-methyl)piperidin-1-yl]ethanol dihydrochloride

### a) 3-[[1-[2-(3-Methoxyphenyl)-2-oxoethyl]-4-piperidinyl]methyl]-6-chloro-2-imino-benzothiazoline

The compound obtained in Example 1 (g) (1.69 g, 6.0 mmol) and 2-bromo-3'-methoxyacetophenone (1.37 g, 6.0 mmol) were used to perform a reaction in the same manner as described in Example 1 (h), and the product was purified by silica gel column chromatography (eluent: methylene chloride/methanol = 19:1, Rf = 0.40) to obtain the title compound as an oil (yield: 78%).

### b) (R,S)-1-(3-Methoxyphenyl)-2-[4-(6-chloro-2-iminobenzothiazolin-3-ylmethyl)-piperidin-1-yl]ethanol dihydrochloride

The compound obtained in Example 7 (a) was used to perform a reaction in the same manner as described in Example 1 (i), and the resulting solid was washed with ethanol to obtain the free form of the title compound as white solid (yield: 80%). Melting point: 156-158°C. Rf = 0.4 (methylene chloride/methanol = 9:1).
¹H-NMR (CDCl₃): 7.26-7.16 (m, 3H), 6.94 (m, 2H), 6.75 (m, 2H), 4.67 (dd, 1H), 3.80 (m, 5H), 3.14 (d, 1H), 2.80 (d, 1H), 2.48-2.26 (m, 3H), 1.97 (m, 2H), 1.69 (m, 2H), 1.45 (m, 2H)

The above free compound was dissolved in ethyl acetate, and the solution was added with 4 N hydrochloric acid solution in ethyl acetate to obtain the title compound as light orange solid (yield: 83%). Melting point: 186-188°C
¹H-NMR (DMSO-d₆): 11.0 (m, 2H), 10.1 (m, 1H), 8.15 (d, 1H), 7.88-7.76 (m, 2H), 7.30-6.84 (m, 4H), 6.20 (m, 1H), 5.10 (m, 1H), 4.40 (m, 2H), 3.80-2.95 (m, 10H), 2.16-1.76 (m, 4H)

### Example 8: (R,S)-1-(4-Trifluoromethylphenyl)-2-[4-(6-chloro-2-iminobenzothiazolin-3-yl-methyl)piperidin-1-yl]ethanol dihydrochloride (Compound 110 in Table 2)

### a) 3-[[1-[2-(4-Trifluoromethylphenyl)-2-oxoethyl]-4-piperidinyl]methyl]-6-chloro-2-iminobenzothiazoline

The compound obtained in Example 1 (g) (1.69 g, 6.0 mmol) and 2-bromo-4'-trifluoromethylacetophenone (1.6 g, 6.0 mmol) were used to perform a reaction in the same manner as described in Example 1 (h), and the product was purified by silica gel column chromatography (eluent: methylene chloride/methanol = 19:1, Rf = 0.45) to obtain the title compound as an oil (yield: 56%).

### b) (R,S)-1-(4-Trifluoromethylphenyl)-2-[4-(6-chloro-2-iminobenzothiazolin-3-yl-methyl)piperidin-1-yl]ethanol dihydrochloride

The compound obtained in Example 8 (a) was used to perform a reaction in the same manner as described in Example 1 (i), and the resulting solid was washed with a mixed solvent of ethanol and hexane (1:1) to obtain the free form of the title compound as a light yellow solid (yield: 78%). Melting point: 168-170°C. Rf = 0.50 (methylene chloride/methanol = 9:1).
¹H-NMR (CDCl₃): 7.58 (d, 2H), 7.48 (d, 2H), 7.23 (d, 1H), 7.17 (dd, 1H), 6.76 (d, 1H), 4.74 (dd, 1H), 3.83 (d, 2H), 3.14 (d, 1H), 2.80 (d, 1H), 2.50 (dd, 1H), 2.41-2.29 (m, 2H), 2.03 (m, 2H), 1.70 (m, 2H), 1.47 (m, 2H)

The above free compound was dissolved in ethyl acetate, and added with 4 N hydrochloric acid solution in ethyl acetate to obtain the title compound as white solid (yield: 86%). Melting point: 202-204°C.
¹H-NMR (DMSO-d₆): 11.0 (m, 2H), 10.15 (m, 1H), 8.14 (d, 1H), 7.95-7.57 (m, 7H), 6.42 (m, 1H), 5.32 (m, 1H), 4.40 (m, 2H), 3.70-2.99 (m, 7H), 2.20-1.86 (m, 4H)

| Elemental analysis (C₂₂H₂₅Cl₃F₃N₃OS + 2H₂O) | | | | | | |
|---|---|---|---|---|---|---|
| Calcd. | C, 46.90%; | H, 4.47%; | N, 7.46%; | Cl, 18.89%; | F, 10.12%; | S, 5.69% |
| Found | C, 47.19%; | H, 4.56%; | N, 7.38%; | Cl, 19.51%; | F, 9.87%; | S, 5.53% |

### Example 9: 3-[[1-[2-(4-Fluorophenyl)-2-oxoethyl]-4-piperidinyl]methyl]-6-chloro-2-acetyliminobenzothiazoline hydrochloride

### a) tert-Butyl 4-(6-chloro-2-acetyliminobenzothiazolin-3-ylmethyl)piperidine-1-carboxylate

The imino compound obtained in Example 1 (f) (6.10 g, 16 mmol) and triethylamine (2.36 ml, 16.5 mmol) were dissolved in a mixed solvent of ethyl acetate (80 ml) and tetrahydrofuran (20 ml), and the solution was added dropwise with acetic anhydride (1.56 ml, 16.5 mmol) at 0°C. After the addition was completed, the mixture was stirred for 2 hours at room temperature, added with water and extracted with ethyl acetate. The organic layer was dried over sodium sulfate, and the solvent was evaporated. The resulting solid was collected by filtration and washed with a mixed solvent of hexane and ethyl acetate (7:3) to obtain the title compounds as white powder (6.0 g, yield: 88.5%). Rf = 0.7 (methylene chloride/ethyl acetate = 8:2).

### b) 4-(6-Chloro-2-acetyliminobenzothiazolin-3-ylmethyl)piperidine

Using the compound obtained in Example 9 (a), a reaction was performed in the same manner as described in Example 1 (g) to obtain the title compound as white solid (yield: 80%). Melting point: 183-185°C.
¹H-NMR (CDCl₃): 7.61 (d, 1H), 7.39 (dd, 1H), 7.20 (d, 1H), 4.23 (d, 2H), 3.06 (m, 2H), 2.54 (dt, 2H), 2.33 (s, 3H), 2.1 (m, 1H), 1.59 (m, 3H), 1.37(dq, 2H)

### c) 3-[[1-[2-(4-Fluorophenyl)-2-oxoethyl]-4-piperidinyl]methyl]-6-chloro-2-acetyl-iminobenzothiazoline hydrochloride

The compound obtained in Example 9 (b) (1.24 g, 3.8 mmol) and 2-bromo-4'-fluoroacetophenone (825 mg, 3.8 mmol) were used to perform a reaction in the same manner as described in Example 1 (h), and the product was purified by silica gel column chromatography (eluent: methylene chloride/ethyl acetate = 1:1, Rf = 0.40) to obtain the free form of the title compound as white solid (yield: 75%). Melting point: 168-170°C.
¹H-NMR (CDCl₃): 8.05 (m, 2H), 7.61 (d, 1H), 7.39 (dd, 1H), 7.19-7.09 (m, 3H), 4.25 (d. 2H), 3.74 (s, 2H), 2.96 (m, 2H), 2.33 (s, 2H), 2.09 (m, 3H), 1.61 (m, 4H)

The above free compound was dissolved in a mixed solvent of methylene chloride and methanol (9:1), and the solution was added with 4 N hydrochloric acid solution in ethyl acetate to obtain the title compound as white solid (yield: 80%).
Melting point: 234-242°C.
¹H-NMR (DMSO-d₆): 10.10 (m, 1H), 8.28-8.00 (m 3H), 7.90-7.75 (m, 1H), 7.56-7.38 (m, 3H), 5.03 (m, 1H), 5.02 (s, 2H), 4.50 (m, 2H), 3.60-3.05 (m, 4H), 2.29 (s, 3H), 2.10-1.80 (m, 3H)

### Example 10: (R,S)-1-Phenyl-2-[4-(6-chloro-2-iminobenzothiazolin-3-ylmethyl)-piperidin-1-yl]ethanol dihydrochloride

### a) 3-[[1-(2-Phenyl-2-oxoethyl)-4-piperidinyl]methyl]-6-chloro-2-iminobenzothiazoline (Compound 112 in Table 1)

The compound obtained in Example 1 (g) (1.69 g, 6.00 mmol) and 2-bromoacetophenone (1.20 g, 6.03 mmol) were used to perform reaction in the same manner as described in Example 1 (h), and the product was purified by silica gel column chromatography (eluent: methylene chloride/methanol = 19:1) to obtain the title compound as white crystals (2.32 g, yield: 96.7%).
¹H-NMR (CDCl₃): 8.00 (m, 2H), 7.57 (m, 1H), 7.45 (m, 2H), 7.23-7.15 (m, 2H), 6.76 (d, 1H), 3.81 (d, 2H), 3.78 (s, 2H), 3.00 (m, 2H), 2.17-1.96 (m, 3H), 1.71-1.51 (m, 4H)

### b) (R,S)-1-Phenyl-2-[4-(6-chloro-2-iminobenzothiazolin-3-ylmethyl)piperidin-1-yl]-ethanol dihydrochloride

The compound obtained in Example 10 (a) (2.32 g) was used to perform a reduction reaction in the same manner as described in Example 1 (i), and the product was purified by silica gel column chromatography (eluent: methylene chloride/methanol = 92:8) to obtain the free form of the title compound as viscous oil (1.08 g, 46.3%).
¹H-NMR (CDCl₃): 7.38-7.16 (m, 7H). 6.78 (d, 1H), 4.72 (dd, 1H), 3.83 (d, 2H), 3.19 (d, 1H), 2.85 (d, 1H), 2.53-2.24 (m, 3H), 2.03 (m, 2H), 1.70 (m, 2H), 1.50 (m, 2H)

The above free compound was dissolved in ethanol, and the solution was added with 4 N hydrochloric acid solution in ethyl acetate to obtain the title hydrochloride as white crystals at a yield of 95.5%. Melting point: 188-198°C.
¹H-NMR (DMSO-d₆): 11.00 (m, 2H), 9.99 (m, 1 H), 8.14 (s, 1H), 7.85-7.31 (m, 7H), 6.24 (m, 1H), 5.13 (m, 1H), 4.36 (m, 2H), 3.60-2.77 (m, 7H), 2.20-1.71 (m, 4H)

### Example 11: (R,S)-1-(3-Fluorophenyl)-2-[4-(6-chloro-2-imino benzothiazolin-3-yl-methyl)piperidin-1-yl]ethanol dihydrochloride

### a) 2-Bromo-3'-fluoroacetophenone

Bromine (1.65 ml, 32.1 mmol) was added dropwise to a solution of 3-fluoroacetophenone (4.15 g, 30.0 mmol) in acetic acid (23 ml) at room temperature, and then the mixture was allowed to react at room temperature for 1.5 hours. The reaction mixture was added with water, and extracted with ethyl acetate. The organic layer was washed successively with water, aqueous sodium hydrogencarbonate and then with saturated brine, and dried over anhydrous magnesium sulfate. After the solvent was evaporated, the residue was purified by silica gel column chromatography (eluent: ethyl acetate/hexane = 1:40) to obtain the title compound as white crystals (4.64 g, yield: 71.3%).
¹H-NMR (CDCl₃): 7.79 (dd, 1H), 7.70 (m 1H), 7.48 (m, 1H), 7.30 (m, 1H), 4.43 (s, 2H)

### b) 3-[[1-[2-(3-Fluorophenyl)-2-oxoethyl)-4-piperidinyl]methyl]-6-chloro-2-iminobenzo-thiazoline

The compound obtained in Example 1 (g) (1.69 g, 6.00 mmol) and 2-bromo-3'-fluoroacetophenone (1.31 g, 6.03 mmol) obtained in Example 11 (a) were used to perform a reaction in the same manner as described in Example 1 (h), and the product was purified by silica gel column chromatography (eluent: methylene chloride/methanol = 19:1) to obtain the title compound as white crystals (2.25 g, yield: 89.7%).
¹H-NMR (CDCl₃): 7.80 (m, 1H), 7.70 (m, 1H), 7.42 (m, 1H), 7.30-7.15 (m, 3H), 6.76 (d, 1H), 3.81 (d, 2H), 3.73 (s, 2H), 2.97 (m, 2H), 2.17-1.96 (m, 3H), 1.71-1.50 (m, 4H)

### c) (R,S)-1-(3-Fluorophenyl)-2-[4-(6-chloro-2-iminobenzothiazolin-3-ylmethyl)piperidin-1-yl]ethanol dihydrochloride

Using the ketone compound obtained in Example 11 (b) (2.25 g), a reduction reaction was performed in the same manner as described in Example 1 (i) to obtain the free form of the title compound as white crystals (1.95 g, yield: 86.3%).
¹H-NMR (CDCl₃): 7.30-7.00 (m, 5H), 6.95 (m, 1H), 6.78 (d, 1H), 4.79 (dd, 1H), 3.83 (d, 2H), 3.21 (d, 1H), 2.92 (d, 1H), 2.58-2.34 (m, 3H), 2.08 (m, 2H), 1.74 (m, 2H), 1.50 (m, 2H)

The above free compound was dissolved in ethanol, and the solution was added with 4 N hydrochloric acid solution in ethyl acetate to obtain the title hydrochloride as white crystals at a yield of 94.9%. Melting point: 183 to 191°C.
¹H-NMR (DMSO-d₆): 11.00 (m, 2H), 10.00 (m, 1H), 8.15 (s, 1H), 7.87-7.12 (m, 6H), 6.39 (m, 1H), 5.20 (m, 1H), 4.36 (m, 2H), 3.60-2.77 (m, 7H), 2.20-1.71 (m, 4H)

### Example 12: (R,S)-1-(4-Trifluoromethoxyphenyl)-2-[4-(6-chloro-2-iminobenzothiazolin-3-ylmethyl)piperidin-1-yl]ethanol dihydrochloride

### a) 2-Bromo-4'-trifluoromethoxyacetophenone

By using 4-trifluoromethoxyacetophenone (8.03 g, 39.3 mmol), the title compound was obtained in the same manner as described in Example 11 (a) as white crystals (8.13 g, yield: 73.1%).

### b) 3-[[1-[2-(4-Trifluoromethoxyphenyl)-2-oxoethyl]-4-piperidinyl]methyl]-6-chloro-2-iminobenzothiazoline

The compound obtained in Example 1 (g) (1.41 g, 5.00 mmol) and 2-bromo-4'-trifluoromethoxyacetophenone obtained in Example 12 (a) (1.42 g, 5.02 mmol) were used to perform a reaction in the same manner as described in Example 1 (h), and the product was purified by silica gel column chromatography (eluent: methylene chloride/methanol = 97:3) to obtain the title compound as white crystals (2.05 g, yield: 84.7%).
¹H-NMR (CDCl₃): 8.09 (d, 2H), 7.29-7.13 (m, 4H), 6.76 (d, 1H), 3.81 (d, 2H), 3.72 (s, 2H), 2.96 (m, 2H), 2.15-1.95 (m, 3H), 1.71-1.41 (m, 4H)

### c) (R,S)-1-(4-Trifluoromethoxyphenyl)-2-[4-(6-chloro-2-iminobenzothiazolin-3-yl-methyl)piperidin-1-yl]ethanol dihydrochloride

Using the ketone compound obtained in Example 12 (b) (2.05 g), a reduction reaction was performed in the same manner as described in Example 1 (i) to obtain the free form of the title compound as white crystals (1.81 g, yield: 87.8%).
¹H-NMR (CDCl₃): 7.38 (d, 2H), 7.27-7.15 (m, 4H), 6.77 (d, 1H), 4.70 (dd, 1H), 3.83 (d, 2H), 3.16 (d, 1H), 2.83 (d, 1H), 2.53-2.24 (m, 3H), 2.02 (m, 2H), 1.74 (m, 2H), 1.59-1.23 (m, 2H)

The above free compound was dissolved in ethanol, and the solution was added with 4 N hydrochloric acid solution in ethyl acetate to obtain the title hydrochloride as white crystals at a yield of 96.7%. Melting point: 183-193°C.
¹H-NMR (DMSO-d₆): 11.00 (m, 2H), 10.00 (m, 1H), 8.14 (s, 1H), 7.87-7.30 (m, 6H), 6.36 (m, 1H), 5.20 (m, 1H), 4.36 (m, 2H), 3.60-2.80 (m, 7H), 2.25-1.70 (m, 4H)

### Example 13: (R,S)-1-(4-Methoxyphenyl)-2-[4-(6-chloro-2-iminobenzothiazolin-3-yl-methyl)piperidin-1-yl]ethanol dihydrochloride

### a) 3-[[1-[2-(4-Methoxyphenyl)-2-oxoethyl]-4-piperidinyl]methyl]-6-chloro-2-imino-benzothiazoline

The compound obtained in Example 1 (g) (1.41 g, 5.00 mmol) and 2-bromo-4'-methoxyacetophenone (1.15 g, 5.02 mmol) were used to perform a reaction in the same manner as described in Example 1 (h), and the product was purified by silica gel column chromatography (eluent: methylene chloride/methanol = 97:3) to obtain the title compound as white crystals (1.96 g, 91.2%).
¹H-NMR (CDCl₃): 8.01 (d, 2H), 7.18 (m, 2H), 6.91 (d, 2H), 6.76 (d, 1H), 3.87 (s, 3H), 3.81 (d, 2H), 3.71 (s, 2H), 2.98 (m, 2H), 2.15-1.95 (m, 3H), 1.71-1.50 (m, 4H)

### b) (R,S)-1-(4-Methoxyphenyl)-2-[4-(6-chloro-2-iminobenzothiazolin-3-ylmethyl)-piperidin-1-yl]ethanol dihydrochloride

Using the ketone compound obtained in Example 13 (a) (1.96 g), a reduction reaction was performed in the same manner as described in Example 1 (i) to obtain the free form of the title compound as white crystals (0.51 g, 2.5.9%).
¹H-NMR (CDCI₃): 7.30-7.15 (m, 4H), 6.85 (d, 2H), 6.77 (d, 1 H), 4.66 (dd, 1H), 3.83 (d, 2H), 3.79 (s, 3H), 3.16 (d, 1H), 2.85 (d, 1H), 2.43 (m, 2H), 2.27 (m, 1H), 2.00 (m, 2H), 1.70 (m, 2H), 1.50 (m, 2H)

The above free compound was dissolved in ethanol, and added with 4 N hydrochloric acid solution in ethyl acetate to obtain the title hydrochloride as white crystals at a yield of 95.6%. Melting point: 180-190°C.
¹H-NMR (DMSO-d₆): 11.00 (m, 2H), 10.00 (m, 1H), 8.16 (s, 1H), 7.87-7.30 (m, 4H), 7.31 (d, 2H), 6.93 (d, 2H), 5.09 (m, 1H), 4.36 (m, 2H), 3.70-2.80 (m, 7H), 2.25-1.70 (m, 4H)

### Example 14: (R,S)-1-(4-Methylphenyl)-2-[4-(6-chloro-2-iminobenzothiazolin-3-yl-methyl)piperidin-1-yl]ethanol dihydrochloride (Compound 108 in Table 1)

### a) 3-[[1-[2-(4-Methylphenyl)-2-oxoethyl]-4-piperidinyl]methyl]-6-chloro-2-imino-benzothiazoline

The compound obtained in Example 1 (g) (1.69 g, 6.00 mmol) and 2-bromo-4'-methylacetophenone (1.28 g, 6.01 mmol) were used to perform a reaction in the same manner as described in Example 1 (h), and the product was purified by silica gel column chromatography (eluent: methylene chloride/methanol = 97:3) to obtain the title compound as white crystals (2.20 g, yield: 88.6%).
¹H-NMR (CDCl₃): 7.90 (d, 2H), 7.26-7.13 (m, 4H), 6.76 (d, 1H), 3.80 (d, 2H), 3.75 (s, 3H), 2.98 (m, 2H), 2.41 (s, 3H), 2.15-1.95 (m, 3H), 1.71-1.50 (m, 4H)

### b) (R,S)-1-(4-Methylphenyl)-2-[4-(6-chloro-2-iminobenzothiazolin-3-ylmethyl)-piperidin-1-yl]ethanol dihydrochloride

Using ketone compound obtained in Example 14 (a) (1.04 g), a reduction reaction was performed in the same manner as described in Example 1 (i) to obtain the free form of the title compound as white crystals (0.84 g, yield: 80.5%).
¹H-NMR (CDCl₃): 7.27-7.12 (m, 6H), 6.77 (d, 1H), 4.67 (dd, 1H), 3.83 (d, 2H), 3.17 (d, 1H), 2.80 (d, 1H), 2.46-2.20 (m, 3H), 2.33 (s, 3H), 2.00 (m, 2H), 1.70 (m, 2H), 1.50 (m, 2H)

The above free compound was dissolved in ethanol, and the solution was added with 4 N hydrochloric acid solution in ethyl acetate to obtain the title hydrochloride as white crystals at a yield of 94.2%. Melting point: 187-197°C.
¹H-NMR (DMSO-d₆): 11.00 (m, 2H), 10.00 (m, 1H), 8.16 (s, 1H), 7.87-7.50 (m, 2H), 7.40-7.15 (m, 4H), 5.09 (m, 1H), 4.35 (m, 2H), 3.70-2.80 (m, 7H), 2.29 (s, 3H), 2.25-1.70 (m, 4H)

### Example 15: 3-[[1-[2-(4-Fluorophenyl)-2-oxoethyl]-4-piperidinyl]methyl]-2-imino-benzothiazoline dihydrochloride

### a) 2-Trifluoroacetylaminobenzothiazole

Starting from 2-aminobenzothiazole (7.51 g, 50.0 mmol), the title compound was obtained as white crystals (11.5 g, yield: 93.4%) in the same manner as described in Example 1 (d).
¹H-NMR (CDCl₃ + DMSO-d₆): 7.75 (d, 1H), 7.61 (d, 1H), 7.47 (m, 1H), 7.36 (m, 1H)

### b) tert-Butyl 4-(2-trifluoroacetyliminobenzothiazolin-3-ylmethyl)piperidine-1-carboxylate

Using the amide compound obtained in Example 15 (a) (493 mg), a reaction was performed in the same manner as described in Example 1 (e) to obtain the title compound as white crystals (410 mg, yield: 46.2%).
¹H-NMR (CDCl₃): 7.78 (d, 1H), 7.57 (m, 1H), 7.44 (m, 2H), 4.39 (m, 2H), 4.12 (m, 2H), 2.67 (t, 2H), 2.18 (m, 1H), 1.60-1.26 (m, 4H), 1.46 (s, 9H)

### c) tert-Butyl 4-(2-iminobenzothiazolin-3-ylmethyl)piperidine-1-carboxylate

Using the crystals obtained in Example 15 (b) (410 mg), a reaction was performed in the same manner as described in Example 1 (f) to obtain the title compound as foamy powder (330 mg, yield: quantitative). Rf = 0.1 (ethyl acetate/hexane = 1:2).

### d) 4-(2-Iminobenzothiazolin-3-ylmethyl)piperidine

Using the powder obtained in Example 15 (c) (330 mg), a reaction was performed in the same manner as described in Example 1 (g) to obtain the title compound as foamy powder (210 mg, yield: 91.8%).
¹H-NMR (CDCl₃): 7.23 (m, 2H), 6.99 (dt, 1H), 6.86 (d, 1H), 3.82 (d, 2H), 3.10 (m, 2H), 2.58 (m, 2H), 2.12 (m, 1H), 1.75-1.23 (m, 5H)

### e) 3-[[1-[2-(4-Fluorophenyl)-2-oxoethyl]-4-piperidinyl]methyl]-2-iminobenzothiazoline dihydrochloride

The compound obtained in Example 15 (d) (210 mg) and 2-chloro-4'-fluoroacetophenone (150 mg) were used to perform a reaction in the same manner as described in Example 1 (h), and the product was purified by silica gel column chromatography (eluent: methylene chloride/methanol = 97:3) to obtain the free form of the title compound (230 mg, yield: 70.6%).
¹H-NMR (CDCl₃): 8.06 (m, 2H), 7.26-7.08 (m, 4H), 6.99 (dt, 1H), 6.86 (d, 1H), 3.84 (d, 2H), 3.71 (s, 2H), 2.96 (m, 2H), 2.16-2.00 (m, 3H), 1.70 (m, 2H), 1.50 (m, 2H)

The above free compound was dissolved in ethanol, and added with 4 N hydrochloric acid solution in ethyl acetate to obtain the title hydrochloride as white crystals at a yield of 91.3%. Melting point: 185-195°C.
¹H-NMR (DMSO-d₆): 11.00 (m, 2H), 10.13 (m, 1H), 8.28 (s, 1H), 8.07-7.40 (m, 7H), 5.32-5.02 (m, 2H), 4.53-4.42 (m, 2H), 3.70-3.00 (m, 4H), 2.50-1.60 (m, 5H)

### Example 16: 3-[[1-[2-(4-Fluorophenyl)-2-oxoethyl]-4-piperidinyl]methyl]-6-methoxy-2-iminobenzothiazoline dihydrochloride

### a) 6-Methoxy-2-trifluoroacetylaminobenzothiazole

Starting from 6-methoxy-2-aminobenzothiazole (7.21 g, 40.0 mmol), the title compound was obtained as white crystals (8.75 g, yield: 79%) in the same manner as described in Example 1 (d).

### b) tert-Butyl 4-(6-methoxy-2-trifluoroacetyliminobenzothiazolin-3-ylmethyl)-piperidine-1-carboxylate

Using the amide compound obtained in Example 16 (a) (8.75 g), a reaction was performed in the same manner as described in Example 1 (e) to obtain the title compound as white crystals (5.70 g, yield: 38%).
¹H-NMR (CDCl₃): 7.35 (d, 1H), 7.26 (d, 1H), 7.12 (dd, 1H), 4.40-4.30 (m, 2H), 4.20-4.05 (m, 2H), 3.89 (s, 3H), 2.66 (t, 2H), 2.22-2.10 (m, 1H), 1.70-1.32 (m, 4H), 1.46 (s, 9H)

### c) tert-Butyl 4-(6-methoxy-2-iminobenzothiazolin-3-ylmethyl)piperidine-1-carboxylate

Using the crystal obtained in Example 16 (b) (5.70 g), a reaction was performed in the same manner as described in Example 1 (f) to obtain the title compound as foamy powder (4.52 g, yield: quantitative).
¹H-NMR (CDCl₃): 6.85 (d, 1H), 6.76-6.71 (m, 2H), 4.20-4.05 (m, 2H), 3.85-3.68 (m, 2H), 3.78 (s, 3H), 2.65 (t, 2H), 2.20-2.08 (m, 1H), 1.72-1.60 (m, 2H), 1.45 (s, 9H), 1.40-1.21 (m, 2H)

### d) 4-(6-Methoxy-2-iminobenzothiazolin-3-ylmethyl)piperidine

Using the powder obtained in Example 16 (c) (4.52 g), a reaction was performed in the same manner as described in Example 1 (g) to obtain the title compound as foamy powder (3.10 g, yield: 94%).

### e) 3-[[1-[2-(4-Fluorophenyl)-2-oxoethyl]-4-piperidinyl]methyl]-6-methoxy-2-imino-benzothiazoline dihydrochloride

The compound obtained in Example 16 (d) (555 mg) and 2-chloro-4'-fluoroacetophenone (362 mg) were used to perform a reaction in the same manner as described in Example 1 (h), and the product was purified by silica gel column chromatography (eluent: methylene chloride/methanol = 97:3) to obtain the free form of the title compound (778 mg, yield: 94%).
¹H-NMR (CDCl₃): 8.15-8.03 (m, 2H), 7.10 (t, 2H), 6.84 (s, 1H), 6.76-6.70 (m, 2H), 3.86-3.74 (m, 2H), 3.78 (s, 3H), 3.69 (s, 2H), 2.95 (m, 2H), 2.16-1.98 (m, 3H), 1.70 (m, 2H), 1.58-1.45 (m, 2H)

The above free compound (327 mg) was dissolved in ethanol, and added with 4 N hydrochloric acid solution in ethyl acetate to obtain the title hydrochloride (278 mg, yield: 72%). Melting point: 207-212°C.
¹H-NMR (DMSO-d₆): 10.67 (m, 2H), 10.07 (m, 1H), 8.27-8.04 (m, 2H), 7.78-7.68 (m, 2H), 7.47 (t, 2H), 7.14 (m, 1H), 5.29-4.80 (m, 2H), 4.46-4.35 (m, 2H), 3.81 (s, 3H), 3,51-3.34 (m, 2H), 3.34-2.85 (m, 2H), 2.49-1.57 (m, 5H)

### Example 17: (R,S)-1-(4-Fluorophenyl)-2-[4-(6-methoxy-2-iminobenzothiazolin-3-yl-methyl)piperidin-1-yl]ethanol dihydrochloride

Using the free compound of the ketone obtained in Example 16 (e) (465 mg), a reduction reaction was performed in the same manner as described in Example 1 (i) to obtain the free form of the title compound (417 mg, yield: 90%).
¹H-NMR (CDCl₃): 7.35-7.28 (m, 2H), 7.00 (t, 2H), 6.85 (s, 1H), 6.78-6.75 (m, 2H), 4.67 (dd, 1H), 3.83-3.74 (m, 2H), 3.78 (s, 3H), 3.13 (m, 1H) 2.80 (m, 1H), 2.45-2.20 (m, 3H), 2.04-1.95 (m, 2H), 1.80-1.65 (m, 2H), 1.55-1.41 (m, 2H)

The above free compound was dissolved in ethanol, and added with 4 N hydrochloric acid solution in ethyl acetate to obtain the title hydrochloride (400 mg, yield: 82%). Melting point: 268-272°C.
¹H-NMR (DMSO-d₆): 10.54 (m, 2H), 10.02 (m, 1H), 7.74-7.25 (m, 4H), 7.25-7.10 (m, 3H), 6.28 (m, 1H), 5.18 (m, 1H), 4.34 (m, 2H), 3.81 (s, 3H), 3.62-2.49 (m, 6H), 2.48-1.70 (m, 5H)

### Example 18: 3-[[1-[2-(4-Fluorophenyl)-2-oxoethyl]-4-piperidinyl]methyl]-6-methyl-2-iminobenzothiazoline dihydrochloride

### a) 6-Methyl-2-trifluoroacetylaminobenzothiazole

Starting from 6-methyl-2-aminobenzothiazole (4.93 g), the title compound was obtained as white crystals (2.16 g, yield: 28%) in the same manner as described in Example 1 (d).

### b) tert-Butyl 4-(6-methyl-2-trifluoroacetyliminobenzothiazolin-3-ylmethyl)piperidine-1-carboxylate

Using the amide compound obtained in Example 18 (a) (1.98 g), a reaction was performed in the same manner as described in Example 1 (e) to obtain the title compound as white crystals (2.32 g, yield: 68%).
¹H-NMR (CDCl₃): 7.57 (s, 1H), 7.38-7.30 (m, 2H), 4.40-4.32 (m, 2H), 4.14-4.06 (m, 2H), 2.66 (t, 2H), 2.49 (s, 3H), 2.17 (m, 1H), 1.61-1.33 (m, 4H), 1.46 (s, 9H)

### c) tert-Butyl 4-(6-methyl-2-iminobenzothiazolin-3-ylmethyl)piperidine-1-carboxylate

Using the crystals obtained in Example 18 (b) (2.32 g) to perform a reaction in the same manner as described in Example 1 (f) to obtain the title compound as foamy powder (1.83 g, yield: quantitative).

### d) 4-(6-Methyl-2-iminobenzothiazolin-3-ylmethyl)piperidine

Using the powder obtained in Example 18 (c) (1.83 g), a reaction was performed in the same manner as described in Example 1 (g) to obtain the title compound as foamy powder (1.69 g, yield: quantitative).
¹H-NMR (CDCl₃): 7.06-6.95 (m, 2H), 6.74 (d, 1H), 3.78 (m, 2H), 2.55 (t, 2H), 2.32 (s, 3H), 2.09 (m, 1H), 1.34 (m, 2H), 1.26 (m, 2H)

### e) 3-[[1-[2-(4-Fluorophenyl)-2-oxoethyl]-4-piperidinyl]methyl]-6-methyl-2-imino-benzothiazoline dihydrochloride

The compound obtained in Example 18 (d) (1.69 g) and 2-chloro-4'-fluoroacetophenone (897 mg) were used to perform a reaction in the same manner as described in Example 1 (h), and the product was purified by silica gel column chromatography (eluent: methylene chloride/methanol = 97:3) to obtain the free form of the title compound (1.92 g, yield: 95%).
¹H-NMR (CDCl₃): 8.10-8.05 (m, 2H), 7.13-6.96 (m, 3H), 6.73 (d, 2H), 3.80 (d, 2H), 3.68 (s, 2H), 2.94 (m, 2H), 2.30 (s, 3H), 2.18-2.00 (m, 3H), 1.74-1.50 (m, 4H)

The above free compound (993 mg) was dissolved in ethanol, and added with 4 N hydrochloric acid solution in ethyl acetate to obtain the title hydrochloride (654 mg, yield: 56%). Melting point: 215-222°C.
¹H-NMR (DMSO-d₆): 10.80 (m, 2H), 10.09 (m, 1H), 8.28-8.03 (m, 2H), 7.80-7.64 (m, 2H), 7.47 (t, 2H), 7.37 (m, 1H), 5.29-4.97 (m, 2H), 4.58-4.40 (m, 2H), 3.56-3.28 (m, 2H), 3.28-2.96 (m, 2H), 2.39 (s, 3H), 2.37-1.60 (m, 5H)

### Example 19:3-{[1-[2-(4-Fluorophenyl)-2-oxoethyl]-4-pipertdinyl]methyl]-6-fluoro-2-iminobenzothiazoline dihydrochloride (Compound 127 in Table 1)

### a) 6-Fluoro-2-trifluoroacetylaminobenzothiazole

Starting from 6-fluoro-2-aminobenzothiazole (5.05 g), the title compound was obtained as white crystals (3.65 g, yield: 46%) in the same manner as described in Example 1 (d).

### b) tert-Butyl 4-(6-fluoro-2-trifluoroacetyliminobenzothiazolin-3-ylmethyl)piperidine-1-carboxylate

Using the amide compound obtained in Example 19 (a) (980 mg), a reaction was performed in the same manner as described in Example 1 (e) to obtain the title compound as white crystals (1.06 g, yield: 62%).
¹H-NMR (CDCl₃): 7.50 (m, 1H), 7.40 (m, 1H), 7.29 (m, 1H), 4.39-4.36 (m, 2H), 4.15-4.06 (m, 2H), 2.67 (t, 2H), 2.16 (m, 1H), 1.70-1.52 (m, 2H), 1.42-1.30 (m, 2H), 1.46 (s, 9H)

### c) tert-Butyl 4-(6-fluoro-2-iminobenzothiazolin-3-ylmethyl)piperidine-1-carboxylate

Using the crystals obtained in Example 19 (b) (1.06 g), a reaction was performed in the same manner as described in Example 1 (f) to obtain the title compound as foamy powder (841 mg, yield: quantitative).

### d) 4-(6-Fluoro-2-iminobenzothiazolin-3-ylmethyl)piperidine

Using the powder obtained in Example 19 (c) (841 mg), a reaction was performed in the same manner as described in Example 1 (g) to obtain the title compound as foamy powder (570 mg, yield: 94%).

### e) 3-[[1-[2-(4-Fluorophenyl)-2-oxoethyl]-4-piperidinyl]methyl]-6-fluoro-2-imino-benzothiazoline dihydrochloride

The compound obtained in Example 19 (d) (570 mg) and 2-chloro-4'-fluoroacetophenone (371 mg) were used to perform a reaction in the same manner as described in Example 1 (h), and the product was purified by silica gel column chromatography (eluent: methylene chloride/methanol = 97:3) to obtain the free form of the title compound (803 mg, yield: 93%).
¹H-NMR (CDCl₃): 8.12-8.04 (m, 2H), 7.12-7.00 (m, 2H), 7.00-6.70 (m, 2H), 6.75 (m, 1H), 3.85-3.76 (m, 2H), 3.69 (s, 2H), 2.95 (m, 2H), 2.10 (t, 2H), 2.00 (m, 1H), 1.71-1.45 (m, 4H)

The above free compound (400 mg) was dissolved in ethanol, and added with 4 N hydrochloric acid solution in ethyl acetate to obtain the title hydrochloride (116 mg, yield: 25%). Melting point: 217-221°C.
¹H-NMR (DMSO-d₆): 10.86 (m, 2H), 10.05 (m, 1H), 8.25 (m, 2H), 7.96-7.78 (m, 2H), 7.47 (m, 3H), 5.27-4.96 (m, 2H), 4.48-4.36 (m, 2H), 3.51 (m, 2H), 3.31-2.95 (m, 2H), 2.29-2.17 (m, 2H), 2.09-1.65 (m, 3H)

### Example 20: 3-[[1-[2-(4-Fluorophenyl)-2-oxoethyl]-4-piperidinyl]methyl]-6-trifluoromethoxy-2-iminobenzothiazoline dihydrochloride

### a) 6-Trifluoromethoxy-2-trifluoroacetylaminobenzothiazole

Starting from 6-trifluoromethoxy-2-aminobenzothiazole (4.89 g), the title compound was obtained as white crystals (3.57 g, yield: 52%) in the same manner as described in Example 1 (d).

### b) tert-Butyl 4-(6-trifluoromethoxy-2-trifluoroacetyliminobenzothiazolin-3-ylmethyl)-piperidine-1-carboxylate

Using the amide compound obtained in Example 20 (a) (2.0 g), a reaction was performed in the same manner as described in Example 1 (e) to obtain the title compound as white crystals (1.64 g, yield: 51%).
¹H-NMR (CDCl₃): 7.67 (m, 1H), 7.48-7.40 (m, 2H), 4.39 (m, 2H), 4.16-4.05 (m, 2H), 2.67 (t, 2H), 2.17 (m, 1H), 1.61 (m, 2H), 1.45 (s, 9H), 1.45-1.23 (s, 9H)

### c) tert-Butyl 4-(6-trifluoromethoxy-2-iminobenzothiazolin-3-ylmethyl)piperidine-1-carboxylate

Using the crystals obtained in Example 20 (b) (1.64 g), a reaction was performed in the same manner as described in Example 1 (f) to obtain the title compound as foamy powder (1.34 g, yield: quantitative).

### d) 4-(6-Trifluoromethoxy-2-iminobenzothiazolin-3-ylmethyl)piperidine

Using the powder obtained in Example 20 (c) (1.34 g), a reaction was performed in the same manner as described in Example 1 (g) to obtain the title compound as foamy powder (1.03 g, yield: quantitative).

### e) 3-[[1-[2-(4-Fluorophenyl)-2-oxoethyl]-4-piperidinyl]methyl]-6-trifluoromethoxy-2-iminobenzothiazoline dihydrochloride

The compound obtained in Example 20 (d) (1.03 g) and 2-chloro-4'-fluoro-acetophenone (537 mg) were used to perform a reaction in the same manner as described in Example 1 (h), and the product was purified by silica gel column chromatography (eluent: methylene chloride/methanol = 97:3) to obtain the free form of the title compound (965 mg, yield: 66%).
¹H-NMR (CDCl₃): 8.09-8.03 (m, 2H), 7.14-7.05 (m, 4H), 6.80 (d, 1H), 3.83 (d, 2H), 3.72 (s, 2H), 3.00-2.95 (m, 2H), 2.13 (t, 2H), 1.99 (m, 1H), 1.73-1.65 (m, 2H), 1.60-1.47 (m, 2H)

The above free compound (247 mg) was dissolved in ethanol, and added with 4 N hydrochloric acid solution in ethyl acetate to obtain the title hydrochloride (180 mg, yield: 63%). Melting point: 202-206°C.
¹H-NMR (DMSO-d₆): 11.07 (m, 2H), 10.04 (m, 1H), 8.26-7.85 (m, 4H), 7.60 (m, 1H), 7.47 (t, 2H), 5.29-4.96 (m, 2H), 4.51-4.35 (m, 2H), 3.55-2.95 (m, 4H), 2.30-2.15 (m, 2H), 1.98-1.63 (m, 3H)

### Example 21: 3-[[1-[2-(4-Aminosulfonylphenyl)-2-oxoethyl]-4-piperidinyl]methyl]-6-chloro-2-methyliminobenzothiazoline dihydrochloride

### a) 2-Bromo-4'-aminosulfonylacetophenone

4-Aminosulfonylacetophenone (4.97 g, 25 mmol) was used to obtain the title compound as light yellow solid (3.9 g, yield: 57%) in the same manner as described in Example 11 (a). Melting point: 146-149°C.

### b) 3-[[1-[2-(4-Aminosulfonylphenyl)-2-oxoethyl]-4-piperidinyl]methyl]-6-chloro-2-methyliminobenzothiazoline dihydrochloride

The compound obtained in Example 4 (c) (1.69 g, 5.7 mmol) and 2-bromo-4'-aminosulfonylacetophenone (1.6 g, 7.0 mmol) obtained in Example 21 (a) were used to perform a reaction in the same manner as described in Example 1 (h) to obtain the free form of the title compound as pink solid (2.2 g, yield: 77%). Melting point: 178-180°C. Rf = 0.25 (methylene chloride/ethyl acetate/methanol = 10:9:1).
¹H-NMR (CDCl₃ + DMSO-d₆): 8.1 (d, 2H), 8.01 (d, 2H), 7.32 (d, 1H), 7.17 (dd, 1H), 6.87 (m, 2H), 6.80 (d, 1H), 3.78 (d, 2H), 3.73 (s, 2H), 3.05 (s, 3H), 2.92 (d, 2H), 2.1 (dt, 2H), 2.0 (m, 1H), 1.62 (m, 2H), 1.42 (m, 2H)

The above free compound was dissolved in ethanol/diethyl ether, and the solution was added with 4 N hydrochloric acid solution in ethyl acetate to obtain the title hydrochloride as white solid (yield: 40%). Melting point: 224-227°C.
¹H-NMR (DMSO-d₆): 11.8 (m, 1H), 10.15 (m, 1H), 8.36-7.62 (m, 9H), 5.37 (m, 1H), 5.07 (m, 1H), 4.54 (m, 2H), 3.59-3.06 (m, 8H), 2.50-1.85 (m, 4H)

### Example 22: (R,S)-1-(2,4-Difluorophenyl)-2-[4-(6-chloro-2-methyliminobenzothiazolin-3-ylmethyl)piperidin-1-yl]ethanol dihydrochloride

### a) 2,4-Difluorophenyloxirane

2-Chloro-2',4'-difluoroacetophenone (2.26 g, 11.9 mmol) was dissolved in isopropyl alcohol (10 ml), and added with sodium borohydride (0.3 g, 8 mmol). The mixture was stirred at room temperature for 2 hours, added with 1 N aqueous sodium hydroxide (10 ml), and further stirred at 60°C for 2 hours. After the reaction mixture was cooled, the mixture was added with water and extracted with diethyl ether. The organic layer was dried over sodium sulfate, and the solvent was evaporated. The resulting residue was purified by silica gel column chromatography (eluent: hexane) to obtain the title compound as an oil (890 mg, yield: 48%). Rf = 0.85 (hexane/ethyl acetate = 9:1).

### b) (R,S)-1-(2,4-Difluorophenyl)-2-[4-(6-chloro-2-methyliminobenzothiazolin-3-yl-methyl)piperidin-1-yl]ethanol dihydrochloride

A solution of the compound obtained in Example 4 (c) (1.48 g, 5.0 mmol) and 4-difluorophenyloxirane obtained in Example 22 (a) (790 mg, 5.0 mmol) in ethanol (30 ml) was refluxed for 8 hours by heating. After the reaction mixture was cooled, the solvent was evaporated, and the resulting solid was washed with ethanol to obtain the free form of the title compound as white solid (1.57 g, yield: 69%). Melting point: 161-163°C. Rf = 0.3 (methylene chloride/methanol = 19:1).
¹H-NMR (CDCl₃): 7.53 (q, 1H), 7.32 (d, 1H), 7.16 (dd, 1H), 6.87 (dt, 1H), 6.75 (m, 2H), 4.95 (dd, 1H), 3.79 (d, 2H), 3.12 (d, 1H), 3.08 (s, 3H), 2.79 (d, 1H), 2.58 (dd, 1H), 2.30 (m, 2H), 2.0 (m, 2H), 1.67 (m, 2H), 1.43 (dq, 2H)

The above free compound was dissolved in ethanol/diethyl ether, and the solution was added with 4 N hydrochloric acid solution in ethyl acetate to obtain the title hydrochloride as white solid. Melting point: 233-244°C.
¹H-NMR (DMSO-d₆): 11.7 (m, 1H), 10.1 (m, 1H), 8.20-6.80 (m, 6H), 6.30 (m, 1H), 5.35 (m, 1H), 4.47 (m, 2H), 3.64-2.7 (m, 8H), 2.30-1.60 (m, 6H)

### Example 23: 3-[[1-[2-(4-Fluorophenyl)-2-oxoethyl]-4-piperidinyl]methyl]-2-imino-6-trifluoromethylbenzothiazoline dihydrochloride (Compound 151 in Table 1)

### a) 6-Trifluoromethyl-2-trifluoroacetylaminobenzothiazole

Starting from 2-amino-6-trifluoromethylbenzothiazole (6.54 g, 30 mmol), the title compound was obtained as white crystals (7.4 g, yield: 78%) in the same manner as described in Example 1 (d). Melting point: 200-202°C.

### b) tert-Butyl 4-(6-trifluoromethyl-2-trifluoroacetyliminobenzothiazolin-3-ylmethyl)-piperidine-1-carboxylate

The amide compound obtained in Example 23 (a) (6.28 g, 20 mmol) was used to perform a reaction in the same manner as described in Example 1 (e), and the product was purified by silica gel column chromatography (eluent: methylene chloride/hexane/ethyl acetate = 5:4:1) to obtain the title compound as white crystals (yield: 42%). Melting point: 136-137°C.

### c) tert-Butyl 4-(6-trifluoromethyl-2-iminobenzothiazolin-3-ylmethyl)piperidine-1-carboxylate

Using the compound obtained in Example 23 (b) (4.2 g, 8 mmol), a reaction was performed in the same manner as described in Example 1 (f) to obtain the title compound as foamy powder. Rf = 0.2 (hexane/ethyl acetate = 7:3).

### d) 4-(6-Trifluoromethyl-2-iminobenzothiazolin-3-ylmethyl)piperidine

Using the powder obtained in Example 23 (c), a reaction was performed in the same manner as described in Example 1 (g) to obtain the title compound as white solid (yield: 87%). Melting point: 110-112°C.
¹H-NMR (CDCl₃): 7.47 (m, 2H), 7.0 (m, 1H), 6.90 (d, 1H), 3.84 (d, 2H), 3.1 (d, 2H), 2.59 (dt, 2H), 2.08 (m, 1H), 1.66 (m, 3H), 1.25 (dq, 2H)

### e) 3-[[1-[2-(4-Fluorophenyl)-2-oxoethyl]-4-piperidinyl]methyl]-2-imino-6-trifluoromethylbenzothiazoline dihydrochloride

The compound obtained in Example 23 (d) (2.1 g, 6.7 mmol) and 2-bromo-4'-fluoroacetophenone (1.45 g, 6.7 mmol) were used to perform a reaction in the same manner as described in Example 1 (h), and the product was purified by silica gel column chromatography (eluent: methylene chloride/ethyl acetate/methanol = 5:4.7:0.3) to obtain the free form of the title compound as light yellow foamy powder (2.2 g, yield: 72%).
¹H-NMR (CDCl₃): 8.08 (m, 2H), 7.48 (s+d, 2H), 7.12 (m, 2H), 6.90 (d, 1H), 3.86 (d, 2H), 3.72 (s, 2H), 2.98 (d, 2H), 2.1 (dt, 2H), 2.0 (m, 1H), 1.70 (m, 2H), 1.53 (dq, 2H)

The above free compound was dissolved in ethanol/diethyl ether, and added with 4 N hydrochloric acid solution in ethyl acetate to obtain the title hydrochloride as white solid (yield: 77%). Melting point: 213-221°C.
¹H-NMR (DMSO-d₆): 11.4 (m, 2H), 10.1 (m, 1H), 8.49 (s, 1H), 8.30-7.87 (m, 4H), 7.42 (m, 2H), 5.33 (m, 1H), 5.03 (m, 1H), 4.54 (m, 2H), 3.59-3.05 (m, 5H), 2.48-1.74(m, 4H)

### Example 24: 3-[[1-[2-(4-Fluorophenyl)-2-oxoethyl]-4-piperidinyl]methyl]-6-chloro-2-(2,2,2-trifluoroethyl)iminobenzothiazoline dihydrochloride

### a) 6-Chloro-2-(2,2,2-trifluoroethyl)aminobenzothiazole

To a suspension of lithium aluminium hydride (2.65 g, 60 mmol) in tetrahydrofuran (150 ml), a solution of 6-chloro-2-trifluoroacetylaminobenzothiazole obtained in Example 1 (d) (14.02 g, 50 mmol) in tetrahydrofuran (150 ml) was added dropwise at room temperature, and then the mixture was stirred for 12 hours. After the reaction mixture was cooled with ice, the mixture was successively added with water (2.7 ml), 1 N aqueous sodium hydroxide (2.7 ml) and water (7 ml), and then the organic layer was separated and dried over sodium sulfate. The solvent was evaporated under reduced pressure, and the residue was purified by silica gel column chromatography (eluent: hexane/ethyl acetate = 9:1) to obtain the title compound as white solid (5.1 g, yield: 38%). Melting point: 147-148°C.

### b) tert-Butyl 4-[6-chloro-2-(2,2,2-trifluoroethyl)iminobenzothiazolin-3-ylmethyl]-piperidine-1-carboxylate

The compound obtained in Example 24 (a) was used to perform a reaction in the same manner as described in Example 1 (e), and the product was purified by silica gel column chromatography (eluent: hexane/ethyl acetate/methylene chloride = 5:1:4) to obtain the title compound as an orange solid (yield: 21%). Melting point: 104°C.

### c) 4-[6-Chloro-2-(2,2,2-trifluoroethyl)iminobenzothiazolin-3-ylmethyl]piperidine

Using the compound obtained in Example 24 (b), a reaction was performed in the same manner as described in Example 1 (g) to obtain the title compound as light orange powder (yield: 78%). Melting point: 80-82°C
¹H-NMR (CDCl₃): 7.34 (d, 1H), 7.20 (dd, 1H), 6.83 (d, 1H), 3.85 (d, 2H), 3.68 (q, 2H), 3.06 (d, 2H), 2.57 (dt, 2H), 2.01 (m, 2H), 1.62 (m, 2H), 1.28 (dq, 2H)

### d) 3-[[1-[2-(4-Fluorophenyl)-2-oxoethyl]-4-piperidinyl]methyl]-6-chloro-2-(2,2,2-trifluoroethyl)iminobenzothiazoline dihydrochloride

The compound obtained in Example 24 (c) (370 mg, 1.7 mmol) and 2-bromo-4'-fluoroacetophenone (350 mg, 1.6 mmol) were used to perform a reaction in the same manner as described in Example 1 (h), and the product was purified by silica gel column chromatography (eluent: methylene chloride/ethyl acetate = 8:2) to obtain the free form of the title compound as an oil (300 mg, 37%).
¹H-NMR (CDCl₃): 8.07 (m, 2H), 7.34 (d, 1H), 7.22 (dd, 1H), 7.11 (m, 2H), 6.83 (d, 1H), 3.87 (d, 2H), 3.72 (s, 2H), 3.64 (q, 2H), 2.95 (d, 2H), 2.13 (dt, 2H), 1.92 (m, 1H), 1.66 (m, 2H), 1.53 (dq, 2H)

The above free compound was dissolved in ethyl acetate, and added with 4 N hydrochloric acid solution in ethyl acetate to obtain the title hydrochloride as orange solid (yield: 87%). Melting point: 125-142°C.
¹H-NMR (DMSO-d₆): 10.1 (m, 1H), 9.0-8.6 (m, 1H), 8.30-7.82 (m, 3H), 7.48-7.34 (m, 4H), 5.18-5.02 (m, 2H), 4.19-3.02 (m, 7H), 2.15-1.76 (m, 6H)

### Example 25: 3-[[1-[2-(2,4-Difluorophenyl)-2-hydroxyiminoethyl]-4-piperidinyl]-methyl]-6-chloro-2-methyliminobenzothiazoline dihydrochloride

### a) 2-Chloro-2',4'-difluoroacetophenone oxime

2-Chloro-2',4'-difluoroacetophenone (3.51 g, 18.4 mmol), hydroxylamine hydrochloride (1.40 g, 20.1 mmol) and sodium acetate (1.70 g, 20.7 mmol) were dissolved in ethanol (30 ml), and the mixture was stirred with ice cooling for 1 hour and then at room temperature for 24 hours. The solvent was evaporated under reduced pressure, and the resulting residue was added with aqueous sodium hydrogencarbonate and extracted with ethyl acetate. The organic layer was washed with water and dried over sodium sulfate, and the solvent was evaporated to obtain the title compound (3.76 g, 99%).
¹H-NMR (CDCl₃): 8.73 (s, 1H), 7.55-7.47 (m, 1H), 6.97-6.85 (m, 1H), 4.62 (s, 2H)

### b) 3-[[1-[2-(2,4-Difluorophenyl)-2-hydroxyiminoethyl]-4-piperidinyl]methyl]-6-chloro-2-methyliminobenzothiazoline dihydrochloride

In the presence of sodium iodide, the compound obtained in Example 4 (c) (1.61 g, 5.44 mmol) and 2-chloro-2',4'-difluoroacetophenone oxime obtained in Example 25 (a) (1.50 g, 7.30 mmol) were used to perform a reaction in the same manner as described in Example 1 (h), and the product was purified by silica gel column chromatography (eluent: methylene chloride/methanol = 50:1) to obtain the free form of the title compound as an oil (2.05 g, yield: 81%).
¹H-NMR (CDCl₃): 9.23 (s, 1H), 7.33-7.25 (m, 2H), 7.14 (dd, 1H), 6.94-6.74 (m, 3H), 3.73 (d, 2H), 3.32 (s, 2H), 3.04 (s, 3H), 2.86 (d, 2H), 2.00-1.89 (m, 3H), 1.62-1.56 (m, 2H), 1.35-1.22 (m, 2H)

The above free compound was dissolved in ethyl acetate, and the solution was added with 4 N hydrochloric acid solution in ethyl acetate to obtain the title hydrochloride as white solid (yield: 88.5%). Melting point: 230°C (decomposition). ¹H-NMR (DMSO-d₆): 12.1 (1H), 11.8 (1H), 10.3 (1H), 8.25 (s, 1H), 7.76-7.18 (m, 5H), 4.49 (m, 4H), 3.55-3.45 (m, 2H), 3.10 (s, 3H), 2.93-2.89 (m, 2H), 2.12-1.68 (m, 5H)

### Example 26: 3-[[1-[(6-Fluoro-1,2-benzisoxazol-3-yl)methyl]-4-piperidinyl]methyl]-6-chloro-2-methyliminobenzothiazoline dihydrochloride

To a solution of free compound of the compound obtained in Example 25 (b) (1.12 g, 2.41 mmol) in methanol (60 ml), a solution of sodium methoxide (0.54 ml, 2.66 mmol) in methanol was added, and the mixture was stirred at 60°C for 22 hours. The solvent was evaporated under reduced pressure, and the resulting residue was added with water, and extracted with ethyl acetate. The organic layer was washed with water and dried over sodium sulfate, and the solvent was evaporated to obtain the free form of the title compound as white solid (845 mg, yield: 79.0%).
¹H-NMR (CDCl₃): 7.92-7.87 (m, 1H), 7.32-7.03 (m, 4H), 6.75 (d, 1H), 3.86 (s, 2H), 3.78 (d, 2H), 3.06 (s, 3H), 2.89 (d, 2H), 2.11-2.02 (m, 2H), 1.94-1.88 (m, 1H), 1.66-1.61 (m, 2H), 1.48-1.38 (m, 2H)

The above free compound was dissolved in ethyl acetate, and added with 4 N hydrochloric acid solution in ethyl acetate to obtain the title hydrochloride as white solid (92.0%). Melting point: 178-183°C (decomposition).
¹H-NMR (DMSO-d₆): 11.7-11.4 (m, 2H), 8.32-8.25 (m, 2H), 7.86-7.63 (m, 3H), 7.44-7.38 (m, 1H), 3.58 (d, 2H), 3.10 (s, 3H), 2.13-1.77 (m, 5H)

### Example 27: (R)-1-(4-Fluorophenyl)-2-[4-(6-chloro-2-methyliminobenzothiazolin-3-yl-methyl)piperidin-1-yl]ethanol dihydrochloride

### a) 2-Chloro-1-(4-fluorophenyl)ethanol

2-Chloro-4'-fluoroacetophenone (25 g, 145 mmol) was dissolved in methanol (250 ml), and the solution was added with sodium borohydride (3.78 g, 100 mmol). After the reaction mixture was stirred at room temperature for 5 hours, the solvent was evaporated under reduced pressure. The residue was added with water, and extracted with methylene chloride. The organic layer was washed with water and dried over sodium sulfate, and the solvent was evaporated under reduced pressure. The residue was purified by silica gel column chromatography (eluent: methylene chloride) to obtain the title compound as colorless oil (18.5 g, yield: 73%). ¹H-NMR (CDCl₃): 7.35 (m, 2H), 7.06 (m, 2H), 4.88 (m, 2H), 3.64 (m, 2H), 2.70 (s, 1H)

### b) (R)-(-)-2-Chloro-(4-fluorophenyl)ethanol and (S)-(+)-2-chloro-(4-fluorophenyl)-ethanol acetate

The title compounds were synthesized according to the method described in Tetrahedron Asymmetry, 2, pp.113 (1991). The compound obtained in Example 27 (a) (1.0 g, 5.73 mmol) was dissolved in diethyl ether (15 ml), and the solution was added with isopropenyl acetate (2.2 ml, 20 mmol) and lipase PS (Amano, immobilized Lipase PS on diatomite, 2.0 g), and then the mixture was stirred at room temperature for 24 hours. The reaction mixture was filtered through Cerite, and the filtrate was concentrated. The residue was purified by silica gel column chromatography (hexane/ethyl acetate = 10:1) to obtain (R)-(-)-2-chloro-(4-fluorophenyl)ethanol (393 mg, yield: 39%) and (S)-(+)-2-chloro-(4-fluorophenyl)ethanol acetate (511 mg, yield: 41%) both as colorless oil.

### (R)-(-)-2-Chloro-(4-fluorophenyl)ethanol

¹H-NMR (CDCl₃): 7.35 (m, 2H), 7.06 (m, 2H), 4.88 (m, 2H), 3.64 (m, 2H), 2.70 (s, 1H) >99% e.e. (HPLC: Daicel CHIRALCEL OJ, UV 254 nm, hexane/ethanol = 95/5)

### (S)-(+)-2-Chloro-(4-fluorophenyl)ethanol acetate

¹H-NMR (CDCl₃): 7.35 (m, 2H), 7.06 (m, 2H), 5.93 (m, 1H), 3.75 (m, 2H), 2.13 (s, 3H) 97% e.e. (HPLC: Daicel CHIRALCEL OJ, UV254 nm, hexane/ethanol = 95/5)

### c) (R)-1-(4-Fluorophenyl)-2-[4-(6-chloro-2-methyliminobenzothiazolin-3-ylmethyl)-piperidin-1-yl]ethanol dihydrochloride

The (R)-(-)-2-chloro-(4-fluorophenyl)ethanol obtained in Example 27 (b) (340 mg, 1.95 mmol) was dissolved in ethanol (10 ml) and the solution was added with the 4-(6-chloro-2-methyl iminobenzothiazolin-3-ylmethyl)piperidine obtained in Example 4 (c) (577 mg, 1.95 mmol) and potassium carbonate (270 mg, 1.95 mmol), and then the mixture was stirred at 60°C for 4 hours. The deposited solid was collected by filtration, and washed with ethanol. The resulting solid was washed by suspending the solid in ethanol and water (1:1, 20 ml) at 60°C for 1 hour, then the solid was collected by filtration and washed with ethanol to obtain the free form of the title compound as white solid (534 mg, yield: 63%). Melting point: 211-212°C
¹H-NMR (CDCl₃): 7.31 (m, 3H), 7.18 (dd, 1H), 7.01 (m, 2H), 6.77 (d, 1H), 4.67 (dd, 1H), 3.80 (d, 2H), 3.12 (d, 1H), 3.08 (s, 3H), 2.81 (d, 1H), 2.44 (m, 2H), 2.27 (m, 1H), 1.98 (m, 2H), 1.67 (m, 2H), 1.43 (m, 2H)

The above free compound was dissolved in ethyl acetate, and the solution was added with 4 N hydrochloric acid solution in ethyl acetate to obtain the title compound as white solid (508 mg, yield: 96%). Melting point: 220-223°C
¹H-NMR (DMSO-d₆): 11.7 (m, 1H), 10.1 (m, 1H), 8.25 (s, 1H), 7.90-7.43 (m, 4H), 7.17 (m, 2H), 6.25 (m, 1H), 5.15 (m, 1H), 4.54 (m, 2H), 3.62-2.90 (m, 10H), 2.18-1.78 (m, 4H)
99% e.e. (HPLC: Shinwa Kako ULTRON ES-PhCD, UV 254 nm, 20 mM KH₂PO₄ (pH 3.0)/CH₃CN = 60/40

### Example 28: (S)-1-(4-Fluorophenyl)-2-[4-(6-chloro-2-methyliminobenzothiazolin-3-yl-methyl)piperidine-1-yl]ethanol dihydrochloride

### (a) (S)-4-Fluorostyrene oxide

The (S)-(+)-2-chloro-(4-fluorophenyl)ethanol acetate obtained in Example 27 (b) (565 mg, 2.61 mmol) was dissolved in methanol (10 ml) and water (5 ml), and the solution was added with sodium hydrogencarbonate (500 mg). The reaction mixture was stirred at 60°C for 1 hour, and then the solvent was evaporated under reduced pressure. The residue was added with water and extracted with methylene chloride. The organic layer was washed with water and dried over sodium sulfate, and the solvent was evaporated under reduced pressure to obtain the title compound as colorless oil (300 mg, yield: 83%).
¹H-NMR (CDCl₃): 7.25 (m, 2H), 7.04 (m, 2H), 3.85 (t, 1H), 3.14 (m, 1H), 2.70 (s, 1H)

### (b) (S)-1-(4-Fluorophenyl)-2-[4-(6-chloro-2-methyliminobenzothiazolin-3-ylmethyl)-piperidin-1-yl]ethanol dihydrochloride

The compound obtained in Example 28 (a) (300 mg, 2.17 mmol) was dissolved in ethanol (10 ml), and the solution was added with 4-(6-chloro-2-methyliminobenzothiazolin-3-ylmethyl)piperidine (642 mg, 2.17 mmol) obtained in Example 4 (c), and then the mixture was stirred at 60°C for 4 hours. The deposited solid was collected by filtration and washed with ethanol. The resulting solid was washed by suspending the solid in ethanol and water (1:1, 10 ml) at 60°C for 1 hour, and the solid was collected by filtration and washed with ethanol to obtain the free form of the title compound as white solid (438 mg, yield: 47%). Melting point: 211-212°C
¹H-NMR (CDCl₃): 7.31 (m, 3H), 7.18 (dd, 1H), 7.01 (m, 2H), 6.77 (d, 1H), 4.67 (dd, 1H), 3.80 (d, 2H), 3.12 (d, 1H), 3.08 (s, 3H), 2.81 (d, 1H), 2.44 (m, 2H), 2.27 (m, 1H), 1.98 (m, 2H), 1.67 (m, 2H), 1.43 (m, 2H)

The above free compound was dissolved in ethyl acetate, and the solution was added with 4 N hydrochloric acid solution in ethyl acetate to obtain the title compound as white solid (423 mg, yield: 95%). Melting point: 227-230°C
¹H-NMR (DMSO-d₆): 11.7 (m, 1H), 10.1 (m, 1H), 8.25 (s, 1H), 7.90-7.43 (m, 4H), 7.17 (m, 2H), 6.25 (m, 1H), 5.15 (m, 1H), 4.54 (m, 2H), 3.62-2.90 (m, 10H), 2.18-1.78(m, 4H) 99% e.e. (HPLC: Sinwa Kako ULTRON ES-PhCD, UV 254 nm, 20 mM KH₂PO₄ (pH 3.0)/CH₃CN = 60/40

### Example 31: 3-[[1-[2-(4-Fluorophenyl)-2-oxoethyl]-4-piperidinyl]methyl]-6-bromo-2-oxobenzothiazoline hydrochloride (Compound 175 in Table 1)

### a) tert-Butyl 4-(6-bromo-2-oxobenzothiazolin-3-ylmethyl)piperidine-1-carboxylate

6-Bromo-2-oxobenzothiazoline (1.50 g, 6.52 mmol) was dissolved in dimethylimidazolidinone (16 mL) and the solution was added dropwise to NaH (60% content in oil, 287 mg, 7.17 mmol) suspended in dimethylimidazolidinone (3 mL). The reaction mixture was stirred at room temperature for 1 hour under nitrogen atmosphere, and then added with the methanesulfonate obtained in Example 1 (c) (1.91 g, 6.51 mmol) and sodium iodide (977 mg. 6.52 mmol). The reaction mixture was stirred at 80°C with heating for 2.5 hours. After the reaction mixture was cooled to room temperature, the mixture was poured into water, and the deposited solid was filtered and washed with water to obtain the title compound (2,4 g, yield: 86%).
¹H-NMR (CDCl₃):7.57 (d, 1H), 7.43 (dd, 1H), 6.90 (d, 1H), 4.11 (m, 2H), 3.80 (m, 2H), 2.64 (t, 2H), 2.01 (m, 1H), 1.63 (d, 2H), 1.45 (s, 9H), 1.28 (dq, 2H)

### b) 4-(6-Bromo-2-oxobenzothiazolin-3-ylmethyl)piperidine

The compound obtained in Example 31 (a) (2.75 g, 6.43 mmol) was dissolved in dichloromethane (26 mL), and the solution was added with 4 N hydrochloric acid solution in ethyl acetate (8.0 mL, 32.2 mmol), and the mixture was stirred at room temperature for 2.5 hours. The deposited solid was filtered, and washed with ethyl acetate to obtain hydrochloride of the title compound. The resulting hydrochloride was treated with 0.5 N aqueous sodium hydroxide, and extracted with dichloromethane. The organic layer was dried over sodium sulfate, and then the solvent was evaporated to obtain the title compound (1.89 g, yield: 90%).
¹H-NMR (CDCl₃): 7.55 (d, 1H), 7.42 (dd, 1H), 6.91 (d, 1H), 3.80 (d, 2H), 3.08 (dt, 2H), 2.54 (dt, 2H), 1.96 (m, 1H), 1.64 (d, 2H), 1.27 (dq, 2H)

### c) 3-[[1-[2-(4-Fluorophenyl)-2-oxoethyl]-4-piperidinyl]methyl]-6-bromo-2-oxobenzothiazoline

A solution of the compound obtained in Example 31 (b) (14.19 g, 43.4 mmol), 2-chloro-4'-fluoroacetophenone (7.49 g, 43.4 mmol) and triethylamine (5.27 g, 52.1 mmol) in dimethylformamide (100 mL) was stirred at room temperature for 3 hours. After the reaction mixture was diluted with ethyl acetate, the mixture was washed 4 times with water and once with saturated aqueous sodium chloride solution, and then dried over sodium sulfate. The solvent was evaporated to obtain the title compound (19.23 g, yield: 96%).
¹H-NMR (CDCl₃): 8.06 (m, 2H), 7.55 (d, 1H), 7.42 (dd, 1H), 7.11 (m, 2H), 6.90 (d, 1H), 3.81 (d, 2H), 3.71 (s, 2H), 2.95 (d, 2H), 2.09 (dt, 2H), 1.88 (m, 1H), 1.65 (d, 2H), 1.50 (dq, 2H)

### d) 3-[[1-[2-(4-Fluorophenyl)-2-oxoethyl]-4-piperidinyl]methyl]-6-bromo-2-oxobenzothiazoline hydrochloride

The compound obtained in Example 31 (e) was dissolved in ethanol, and added with 4 N hydrochloric acid solution in ethyl acetate to obtain the title compound as white solid (yield: 75%). Melting point: 233-240°C.
¹H-NMR (DMSO-d₆): 9.92 (m, 1H), 8.14-7.99 (m, 3H), 7.59-7.33 (m, 4H), 5.10-4.99 (m, 2H), 3.96-3.80 (m, 2H), 3.54-2.98 (m, 4H), 2.08 (m, 1H), 1.87-1.68 (m, 4H)

### Example 32: (R,S)-1-(4-Fluorophenyl)-2-[4-(6-bromo-2-oxobenzothiazolin-3-ylmethyl)-piperidin-1-yl]ethanol hydrochloride (Compound 176 in Table 1)

### a) (R,S)-1-(4-Fluorophenyl)-2-[4-(6-bromo-2-oxobenzothiazolin-3-ylmethyl)piperidin-1-yl]ethanol

The compound obtained in Example 31 (c) (1.00 g, 2.16 mmol) was dissolved in a mixed solvent of ethanol (14 mL) and dichloromethane (3 mL), and the solution was added with sodium borohydride (82 mg, 2.16 mmol) and stirred at room temperature for 2 hours. The reaction mixture was diluted with dichloromethane, washed with water and saturated sodium chloride solution, and dried over sodium sulfate, and then the solvent was evaporated to obtain the title compound (0.97 g, 97%).
¹H-NMR (CDCl₃): 7.57 (d, 1H), 7.43 (dd, 1H), 7.32 (m, 2H), 7.02 (m, 2H), 6.92 (d, 1H), 4.67 (dd, 1H), 3.84 (d, 2H), 3.15 (d, 1H), 2.82 (d, 1H), 2.48-2.34 (m, 2H), 2.27 (dt, 1H), 1.99 (dt, 1H), 1.87 (m, 1H), 1.68 (m, 2H), 1.48 (dq, 2H)

### b) (R,S)-1-(4-Fluorophenyl)-2-[4-(6-bromo-2-oxobenzothiazolin-3-ylmethyl)piperidin-1-yl]ethanol hydrochloride

The compound obtained in Example 32 (a) was dissolved in ethanol, and the solution was added with 4 N hydrochloric acid solution in ethyl acetate to obtain the title compound as white solid (yield: 80%). Melting point: 135-138°C.
¹H-NMR (DMSO-d₆): 9.85 (m, 1H), 7.99 (d, 3H), 7.59-7.40 (m, 4H), 7.22 (t, 2H), 6.28 (d, 1H), 5.14 (m, 1H), 3.40-3.88 (m, 2H), 3.67-2.91 (m, 6H), 2.04 (m, 1H), 1.88-1.62 (m, 4H)

### Example 33: 3-[[1-[2-(4-Fluorophenyl)-2-oxoethyl]-4-piperidinyl]methyl]-6-chloro-2-oxobenzothiazoline hydrochloride (Compound 185 in Table 1)

### a) 2-Amino-5-chlorothiophenol

2-Amino-6-chlorobenzothiazole (3.00g, 16.25 mmol) was dissolved in 50% NaOH aqueous solution (30 mL), and the reaction mixture was stirred at 100°C for 6 hours with heating under a nitrogen flow. The reaction mixture was cooled to room temperature, and then poured into acetic acid (50 ml) containing ice, and the deposited solid was filtered and washed with water to obtain the title compound (2.43 g, yield: 94%).
¹H-NMR (CDCl₃): 7.15-7.11 (m, 2H), 6.67-6.63 (m, 1H), 4.32 (br s, 2H)

### b) 6-Chloro-2-oxobenzothiazoline

A solution of the compound obtained in Example 33 (a) (2.43 mg, 15.0 mmol) in tetrahydrofuran (60 mL) was added with 1,1'-carbonyl-bis-1H-imidazole, and the mixture was stirred at room temperature for 3.5 hours. The deposited solid was separated by filtration, and the resulting mother liquor was concentrated and subjected to silica gel column chromatography. A mixture was obtained from an ethyl acetate/hexane (1/4) fraction, and the deposited solid was collected by filtration to obtain the title compound (475 mg, yield: 28%).
¹H-NMR (DMSO-d₆): 12.04 (br s, 1H), 7.76 (d, 1H), 7.33 (dd, 1H), 7.12 (d, 1H)

### c) tert-Butyl 4-(6-chloro-2-oxobenzothiazolin-3-ylmethyl)piperidine-1-carboxylate

Using the compound obtained in Example 33 (b), a reaction was performed in the same manner as described in Example 31 (a) to obtain the title compound (yield: 38%).
¹H-NMR (CDCl₃): 7.43 (d, 1H), 7.30-7.26 (m, 1H), 6.95 (d, 1H), 4.12 (br d, 2H), 3.81 (br d, 2H), 2.65 (br t, 2H), 2.05-1.97 (m, 1H), 1.66-1.61 (br d, 2H), 1.45 (s, 9H), 1.35-1.13 (m, 2H)

### d) 4-(6-Chloro-2-oxobenzothiazolin-3-ylmethyl)piperidine

Using the compound obtained in Example 33 (c), a reaction was performed in the same manner as described in Example 31 (b) to obtain the title compound (yield: 95%).
¹H-NMR (CDCl₃): 7.42 (d, 1H), 7.30-7.27 (m, 1H), 6.96 (d, 1H), 3.80 (d, 2H), 3.09 (br d, 2H), 2.54 (td, 2H), 2.00-1.93 (m, 1H), 1.64 (brd, 2H), 1.28 (ddd, 2H)

### e) 3-[[1-[2-(4-Fluorophenyl)-2-oxoethyl]-4-piperidinyl]methyl]-6-chloro-2-oxobenzothiazoline

Using the compound obtained in Example 33 (d), a reaction was performed in the same manner as described in Example 31 (c) to obtain the title compound (yield: 93%).
¹H-NMR (CDCl₃): 8.08 (m, 2H), 7.42 (d, 1H), 7.28 (dd, 1H), 7.15-7.09 (m, 2H), 6.96 (d, 1H), 3.82 (d, 2H), 3.74 (s, 2H), 2.98 (br d, 2H), 2.16-2.08 (m, 2H), 1.99-1.81 (m, 1H), 1.66 (br d, 2H), 1.59-1.51 (m, 2H)

### f) 3-[[1-[2-(4-Fluorophenyl)-2-oxoethyl]-4-piperidinyl]methyl]-6-chloro-2-oxobenzothiazoline hydrochloride

Using the compound obtained in Example 33 (e), a reaction was performed in the same manner as described in Example 31 (d) to obtain the title compound (yield: 66%). Melting point: 216-229°C.
¹H-NMR (DMSO-d₆): 9.85 (br s, 1H), 8.18-8.02 (m, 2H), 7.85 (s, 1H), 7.47-7.41 (m, 4H), 5.17-4.96 (m, 2H), 3.97-3.88 (m, 2H), 3.56-3.48 (m, 2H), 2.97 (br s, 2H), 2.07 (br s, 1H), 1.86 (m, 2H)

### Example 34:

### 1-(4-Fluorophenyl)-2-[4-(2-oxobenzothiazolin-3-ylmethyl)piperidin-1-yl]ethanol hydrochloride

### a) tert-Butyl 4-(2-oxobenzothiazolin-3-ylmethyl)piperidine-1-carboxylate

Using 2-oxobenzothiazoline, a reaction was performed in the same manner as described in Example 31 (a) to obtain the title compound (yield: quantitative).
¹H-NMR (CDCl₃): 7.43 (d, 1H), 3.32 (t, 1H), 7.17 (t, 1H), 7.03 (d, 1H), 4.15-4.08 (m, 2H), 3.84-3.82 (m, 2H), 2.64 (br t, 2H), 2.04-1.99 (m, 1H), 1.67-1.62 (m, 2H), 1.45 (s, 9H), 1.36-1.23 (m, 2H)

### b) 4-(2-Oxobenzothiazolin-3-ylmethyl)piperidine

Using the compound obtained in Example 34 (a), a reaction was performed in the same manner as described in Example 31 (b) to obtain the title compound (yield: 76%).
¹H-NMR (CDCl₃): 7.42 (dd, 1H), 7.31 (dd, 1H), 7.12 (t, 1H), 7.03 (d, 1H), 3.82 (d, 2H), 3.10 (br d, 2H), 2.55 (td, 2H), 2.06-1.96 (m, 1H), 1.67 (br d, 2H), 1.32 (ddd, 2H)

### c) 3-[[1-[2-(4-Fluorophenyl)-2-oxoethyl]-4-piperidinyl]methyl]-2-oxobenzothiazoline

Using the compound obtained in Example 34 (b), a reaction was performed in the same manner as described in Example 31 (c) to obtain the title compound (yield: 98%).
¹H-NMR (CDCl₃): 8.06 (dd, 2H), 7.43 (d, 1H), 7.31 (t, 1H), 7.18-7.02 (m, 4H), 4.12 (d, 2H), 3.72 (s, 2H), 2.96 (d, 2H), 2.16-2.04 (m, 2H), 1.95-1.89 (m, 1H), 1.71-1.66 (m, 2H), 1.60-1.52 (m, 2H)

### d) 1-(4-Fluorophenyl)-2-[4-(2-oxobenzothiazolin-3-ylmethyl)piperidin-1-yl]ethanol

Using the compound obtained in Example 34 (c), a reaction was performed in the same manner as described in Example 32 (a) to obtain the title compound (yield: 71%).
¹H-NMR (CDCl₃): 7.44 (d, 1H), 7.43-7.30 (m, 3H), 7.20 (t, 1H), 7.06-6.99 (m, 3H), 4.75 (dd, 1H), 3.86 (d, 2H), 3.15 (br d, 1H), 2.82 (brd, 1H), 2.49-2.38 (m, 3H), 2.07-1.94 (m, 3H), 1.72-1.68 (m, 2H), 1.56-1:37 (m, 2H)

### e) 1-(4-Fluorophenyl)-2-[4-(2-oxobenzothiazolin-3-ylmethyl)piperidin-1-yl]ethanol hydrochloride

Using the compound obtained in Example 34 (d), a reaction was performed in the same manner as described in Example 32 (b) to obtain the title compound (yield: 91%). Melting point: 222-235°C.
¹H-NMR (DMSO-d₆): 9.71 (br s, 1H), 7.69 (d, 1H), 7.54-7.37 (m, 4H), 7.29-7.20 (m, 3H), 6.28 (d, 1H), 5.12 (br s, 1H), 4.01-3.89 (m, 2H), 3.68-3.55 (m, 2H), 3.27-3.12 (m, 2H), 2.96-2.88 (m, 2H), 2.07-1.61 (m, 3H)

### Example 35:

### 3-[[1-[2-(4-Fluorophenyl)-2-oxoethyl]-4-piperidinyl]methyl]-6-nitro-2-oxobenzothiazoline hydrochloride

### a) tert-Butyl 4-(6-nitro-2-oxobenzothiazolin-3-ylmethyl)piperidine-1-carboxylate

Using 6-nitro-2-oxobenzothiazoline, a reaction was performed in the same manner as described in Example 31 (a) to obtain the title compound (yield: 95%).
¹H-NMR (CDCl₃): 8.39 (d, 1H), 8.26 (dd, 1H), 7.14 (d, 1H), 4.13 (br s, 2H), 3.90 (br d, 2H), 2.66 (br t, 2H), 2.06-2.00 (m, 1H), 1.66-1.62 (m, 2H), 1.47 (s, 9H), 1.39 (ddd, 2H)

### b) 4-(6-Nitro-2-oxobenzothiazolin-3-ylmethyl)piperidine

Using the compound obtained in Example 35 (a), a reaction was performed in the same manner as described in Example 31 (b) to obtain hydrochloride of the title compound (yield: 92%).
¹H-NMR (DMSO-d₆): 8, 91 (br s, 1H), 8.75 (d, 1H), 8.64 (br s, 1H), 8.26 (dd, 1H), 7.64 (d, 1H), 3.95 (d, 2H), 3.22 (br d, 2H), 2.76 (br dd, 2H), 2.10-2.04 (m, 1H), 1.74 (br d, 2H), 1.44 (br dd, 2H)

Using this hydrochloride, a reaction was performed in the same manner as described in Example 31 (b) to obtain the title compound (yield: 98%).
¹H-NMR (CDCl₃): 8.37 (d, 1H), 8.25 (dd, 1H), 7.13 (d, 1H), 3.88 (d, 2H), 3.09 (dt, 2H), 2.53 (td, 2H), 2.01-1.96 (m, 1H), 1.65 (br s, 2H), 1.37-1.21 (m, 2H)

### c) 3-[[1-[2-(4-Fluorophenyl)-2-oxoethyl]-4-piperidinyl]methyl]-6-nitro-2-oxobenzothiazoline

Using the compound obtained in Example 35 (b), a reaction was performed in the same manner as described in Example 31 (c) to obtain the title compound (yield: 97%).
¹H-NMR (CDCl₃): 8.37 (d, 1H), 8.28 (dd, 1H), 8.07-8.02 (m, 2H), 7.16-7.09 (m, 3H), 3.91 (d, 2H), 3.73 (s, 2H), 2.98 (br d, 2H), 2.18-2.10 (m, 2H), 1.93-1.88 (m, 1H), 1.68-1.52 (m, 4H)

### d) 3-[[1-[2-(4-Fluorophenyl)-2-oxoethyl]-4-piperidinyl]methyl]-6-nitro-2-oxobenzothiazoline hydrochloride

Using the compound obtained in Example 35 (c), a reaction was performed in the same manner as described in Example 31 (d) to obtain the title compound (yield: 82%). Melting point: 213-226°C.
¹H-NMR (DMSO-d₆): 10.09 (br s, 1H), 8.77 (d, 1H), 8.28 (br d, 1H), 8.18-8.05 (m, 2H), 7.70 (d, 1H), 7.46 (br t, 2H), 5.15-5.01 (m, 2H), 4.07-3.99 (m, 2H), 3.52-3.02 (m, 6H), 2.13 (br s, 1H), 1.99-1.73 (m, 2H)

### Example 36: 1-(4-Fluorophenyl)-2-[4-(6-nitro-2-oxobenzothiazolin-3-ylmethyl)-piperidin-1-yl]ethanol hydrochloride

### a) 1-(4-Fluorophenyl)-2-[4-(6-nitro-2-oxobenzothiazolin-3-ylmethyl)piperidin-1-yl]-ethanol

Using the compound obtained in Example 35 (c), a reaction was performed in the same manner as described in Example 32 (a) to obtain the title compound (yield: 68%).
¹H-NMR (CDCl₃): 8.39 (d, 1H), 8.26 (dd, 1H), 7.35-7.30 (m, 2H), 7.15 (d, 1H), 7.05-6.99 (m, 2H), 4.68 (dd, 1H), 3.93 (d, 2H), 3.17 (br d, 1H), 2.84 (br d, 1H), 2.49-2-23 (m, 4H), 2.07-1.41 (m, 5H)

### b) 1-(4-Fluorophenyl)-2-[4-(6-nitro-2-oxobenzothiazolin-3-ylmethyl)piperidin-1-yl]-ethanol hydrochloride

Using the compound obtained in Example 36 (a), a reaction was performed in the same manner as described in Example 32 (b) to obtain the title compound (yield: 80%). Melting point: 243-252°C.
¹H-NMR (DMSO-d₆): 9.68 (br s, 1H), 8.75 (d, 1H), 8.27 (d, 1H), 7.47-7.43 (m, 2H), 7.24-7.12 (m, 3H), 5.13 (br s, 1H), 4.00-3.98 (m, 2H), 3.64-2.67 (m, 6H), 2.12-1.93 (m, 1H), 1.16-1.04 (m, 5H)

### Example 37: 3-[[1-[2-(4-Fluorophenyl)-2-oxoethyl]-4-piperidinyl]methyl]-6-fluoro-2-oxobenzothiazoline hydrochloride (Compound 195 in Table 1)

### a) tert-Butyl 4-(6-fluoro-2-oxobenzothiazolin-3-ylmethyl)piperidine-1-carboxylate

Using 6-fluoro-2-oxobenzothiazoline, a reaction was performed in the same manner as described in Example 31 (a) to obtain the title compound (yield: 75%).
¹H-NMR (CDCl₃): 7.19 (dd, 1H), 7.03 (m, 1H), 6.96 (dd, 1H), 4.12 (m, 2H), 3.82 (d, 2H), 2.65 (t, 2H), 2.02 (m, 1H), 1.64 (d, 2H), 1.45 (s, 9H), 1.29 (dq, 2H)

### b) 4-(6-Fluoro-2-oxobenzothiazolin-3-ylmethyl)piperidine

Using the compound obtained in Example 37 (a), a reaction was performed in the same manner as described in Example 31 (b) to obtain the title compound (yield: 72%).
¹H-NMR (CDCl₃): 7.19 (dd, 1H), 7.03 (m, 1H), 6.97 (dd, 1H), 3.81 (d, 2H), 3.09 (dt, 2H), 2.55 (dt, 2H), 1.98 (m, 1H), 1.65 (d, 2H), 1.28 (dq, 2H)

### c) 3-[[1-[2-(4-Fluorophenyl)-2-oxoethyl]-4-piperidinyl]methyl]-6-fluoro-2-oxobenzothiazoline

Using the compound obtained in Example 37 (b), a reaction was performed in the same manner as described in Example 31 (c) to obtain the title compound (yield: quantitative).
¹H-NMR (CDCl₃): 8.06 (m, 2H), 7.18 (dd, 1H), 7.12 (t, 2H), 7.04 (m, 1H), 6.97 (dd, 1H), 3.83 (d, 2H), 3.73 (s, 2H), 3.83 (d, 2H), 2.12 (dt, 2H), 1.88 (m, 1H), 1.67 (d, 2H), 1.52 (dq, 2H)

### d) 3-[[1-[2-(4-Fluorophenyl)-2-oxoethyl]-4-piperidinyl]methyl]-6-fluoro-2-oxobenzothiazoline hydrochloride

Using the compound obtained in Example 37 (c), a reaction was performed in the same manner as described in Example 31 (d) to obtain the title compound (yield: 63%). Melting point: 205-215°C
¹H-NMR (DMSO-d₆): 8.20 (m, 1H), 8.20-8.05 (m, 2H), 7.71 (dd, 1H), 7.52-7.44 (m, 3H), 7.28 (dt, 1H), 5.16-5.03 (m, 2H), 4.02-3.90 (m, 2H), 3.56-3.02 (m, 4H), 2.11 (m, 1H), 1.91-1.67 (m, 4H)

### Example 38: 3-[[1-[2-(4-Fluorophenyl)-2-oxoethyl]-4-piperidinyl]methyl]-6-methyl-2-oxobenzothiazoline hydrochloride

### a) 6-Methyl-2-oxobenzothiazoline

Using 2-amino-6-methylbenzothiazole, a reaction was performed in the same manner as described in Example 33 (a) and (b), and the product was crystallized from diethyl ether to obtain the title compound as white solid.
¹H-NMR (CDCl₃): 11.76 (bs, 1H), 7.37 (s, 1H), 7.08 (d, 1H), 6.99 (d, 1H), 2.30 (s, 3H)

### b) tert-Butyl 4-(6-methyl-2-oxobenzothiazolin-3-ylmethyl)piperidine-1-carboxylate

Using the compound obtained in Example 38 (a) (450 mg, 2.72 mmol), a reaction was performed in the same manner as described in Example 31 (a) to obtain the title compound as yellow oil. The resulting title compound was used for the following reaction without purification.

### c) 4-(6-Methyl-2-oxobenzothiazolin-3-ylmethyl)piperidine

Using the compound obtained in Example 38 (b), a reaction was performed in the same manner as described in Example 31 (b) to obtain the title compound as an oil (550 mg, yield: 77% for the two steps).
¹H-NMR (CDCl₃): 7.24 (s, 1H), 7.11 (d, 1H), 6.93 (d, 1H), 3.80 (d, 2H), 3.09 (m, 2H), 2.57 (m, 2H), 2.37 (s, 3H), 1.80 (m, 1H), 1.65 (m, 2H), 1.28 (m, 2H)

### d) 3-[[1-[2-(4-Fluorophenyl)-2-oxoethyl]-4-piperidinyl]methyl]-6-methyl-2-oxobenzothiazoline hydrochloride

Using the compound obtained in Example 38 (c), a reaction was performed in the same manner as described in Example 31 (c), and the product was purified by silica gel column chromatography (eluent: methylene chloride/methanol = 50:1) to obtain the free form of the title compound as an oil (yield: 61%).
¹H-NMR (CDCl₃): 8.09-8.04 (m, 2H), 7.23 (s, 1H), 7.14-7.09 (m, 3H), 6.92 (d, 1H), 3.81 (d, 2H), 3.72 (s, 2H), 2.98-2.94 (m, 2H), 2.37 (s, 3H), 2.14-2.06 (dt, 2H), 1.92 (m, 1H), 1.69 (2H, d), 1.55-1.45 (m, 2H)

The above free compound was dissolved in ethyl acetate, and the solution was added with 4 N hydrochloric acid solution in ethyl acetate to obtain the title compound as white solid (yield: 57%). Melting point: 212-217°C
¹H-NMR (DMSO-d₆): 9.87 (bs, 1H), 8.20-8.02 (m, 2H, 7.49-7.41 (m, 3H), 7.30 (d, 2H), 7.18 (d, 1H), 5.12-4.94 (m, 2H), 3.90-3.84 (m, 2H), 3.48-2.95 (m, 4H), 2.32 (s, 3H), 2.06 (m, 1H), 1.80-1.72 (m, 4H)

### Example 39: 3-[[1-[2-(4-Fluorophenyl)-2-oxoethyl]-4-piperidinyl]methyl]-5-chloro-2-oxobenzothiazoline hydrochloride (Compound 213 in Table 1)

### a) tert-Butyl 4-(5-chloro-2-oxobenzothiazolin-3-ylmethyl)piperidine-1-carboxylate

Using 5-chloro-2-oxobenzothiazoline, a reaction was performed in the same manner as described in Example 31 (a) to obtain the title compound (yield: 68%).
¹H-NMR (CDCl₃): 7.35 (d, 1H), 7.15 (dd, 1H), 7.02 (d, 1H), 4.13 (m, 2H), 3.80 (d, 2H), 2.67 (t, 2H), 2.02 (m, 1H), 1.64 (d, 2H), 1.45 (s, 9H), 1.29 (dq, 2H)

### b) 4-(5-Chloro-2-oxobenzothiazolin-3-ylmethyl)piperidine

Using the compound obtained in Example 39 (a), a reaction was performed in the same manner as described in Example 31 (b) to obtain the title compound (yield: 99%).
¹H-NMR (CDCl₃): 7.35 (d, 1H), 7.15 (dd, 1H), 7.03 (d, 1H), 3.80 (d, 2H), 3.16 (m, 2H), 2.60 (dt, 2H), 1.20 (m, 1H), 1.69 (d, 2H), 1.37 (dq, 2H)

### c) 3-[[1-[2-(4-Fluorophenyl)-2-oxoethyl]-4-piperidinyl]methyl]-5-chloro-2-oxobenzothiazoline

Using the compound obtained in Example 39 (b), a reaction was performed in the same manner as described in Example 31 (c) to obtain the title compound (yield: 93%).
¹H-NMR (CDCl₃):8.07 (m, 2H), 7.34 (d, 1H), 7.16-7.09 (m, 3H), 7.03 (d, 1H), 3.80 (d, 2H), 3.72 (s, 2H), 2.97 (d, 2H), 2.13 (dt, 2H), 1.89 (m, 1H), 1.67 (d, 2H), 1.52 (dq, 2H)

### d) 3-[[1-[2-(4-Fluorophenyl)-2-oxoethyl]-4-piperidinyl]methyl]-5-chloro-2-oxobenzothiazoline hydrochloride

Using the compound obtained in Example 39 (c), a reaction was performed in the same manner as described in Example 31 (d) to obtain the title compound (yield: quantitative).
¹H-NMR (DMSO-d₆): 9.95 (m, 1H), 8.07 (m, 2H), 7.33 (d, 1H), 7.65 (d, 1H), 7.47 (t, 2H), 7.29 (dd, 1H), 4.99 (m, 2H), 3.93 (d, 2H), 3.54-3.02 (m, 4H), 2.12 (m, 1H), 1.91-1.68 (m, 4H)

### Example 40: (R,S)-1-(4-Fluorophenyl)-2-[4-(5-chloro-2-oxobenzothiazolin-3-ylmethyl)-piperidin-1-yl]ethanol hydrochloride (Compound 214 in Table 2)

### a) (R,S)-1-(4-Fluorophenyl)-2-[4-(5-chloro-2-oxobenzothiazolin-3-ylmethyl)piperidin-1-yl]ethanol

Using the compound obtained in Example 39 (b), a reaction was performed in the same manner as described in Example 32 (a) to obtain the title compound (yield: 94%).
¹H-NMR (CDCl₃): 7.37-7.31 (m, 3H), 7.16 (dd, 1H), 7.05-6.99 (m, 3H), 7.02 (m, 2H), 4.68 (dd, 1H), 3.83 (d, 2H), 3.16 (d, 1H), 2.83 (d, 1H), 2.49-2.35 (m, 2H), 2.29 (dt, 1H), 2.02 (dt, 1H), 1.92 (m, 1H), 1.69 (m, 2H), 1.48 (dq, 2H)

### b) (R,S)-1-(4-Fluorophenyl)-2-[4-(5-chloro-2-oxobenzothiazolin-3-ylmethyl)piperidin-1-yl]ethanol hydrochloride

Using the compound obtained in Example 40 (a), a reaction was performed in the same manner as described in Example 32 (b) to obtain the title compound (yield: 68%). Melting point: 220°C
¹H-NMR (DMSO-d₆): 9.79 (m, 1H), 7.73 (d, 1H), 7.64 (d, 1H), 7.51-7.42 (m, 2H), 7.30-7.19 (m, 3H), 6.28 (d, 1H), 5.14 (m, 1H), 4.00-3.89 (m, 2H), 3.68-2.90 (m, 6H), 2.07 (m, 1H), 1.91-1.61 (m, 4H)

### Example 41: 3-[[1-(2-Phenyl-2-oxoethyl)-4-piperidinyl]methyl]-6-bromo-2-oxobenzothiazoline hydrochloride (Compound 181 in Table 1)

The compound obtained in Example 31 (b) and 2-bromoacetophenone were used to perform a reaction in the same manner as described in Example 31 (c), and the product was purified by silica gel column chromatography (eluent: methylene chloride/methanol = 30:1) to obtain the free form of the title compound as an oil.

The above free compound was dissolved in ethyl acetate/ethanol (3:1), and the solution was added with 4 N hydrochloric acid solution in ethyl acetate to obtain the title compound as white solid (yield: 48%, for two steps). Melting point: 217-223°C.
¹H-NMR (DMSO-d₆): 9.84 (bs, 1H), 8.09-7.95 (m, 3H), 7.76 (t, 1H), 7.65-7.56 (m, 3H), 7.43 (d, 1H), 5.13-4.98 (m, 2H), 3.99-3.91 (m, 2H), 3.52 (d, 2H), 3.06-2.91 (m, 2H), 2.08 (m, 1H), 1.91-1.58 (m, 4H)

### Example 42: 3-[[1-[2-(4-Chlorophenyl)-2-oxoethyl]-4-piperidinyl]methyl]-6-bromo-2-oxobenzothiazoline hydrochloride (Compound 177 in Table 2)

The compound obtained in Example 31 (b) and 2-bromo-4'-chloroacetophenone were used to perform a reaction in the same manner as described in Example 31 (c), and the product was purified by silica gel column chromatography (eluent: methylene chloride/methanol = 30:1) to obtain the free form of the title compound as an oil.

The above free compound was dissolved in ethyl acetate/ethanol (3:1), and added with 4 N hydrochloric acid solution in ethyl acetate to obtain the title compound as white solid (yield: 48%, for two steps). Melting point: 210-218°C.
¹H-NMR (DMSO-d₆): 9.90 (bs, 1H), 8.09-7.95 (m, 3H), 7.71 (d, 2H), 7.58 (dd, 1H), 7.42 (d, 1H), 5.12-4.94 (m, 2H), 4.01-3.88 (m, 2H), 3.54-3.48 (m, 2H), 3.15-2.94 (m, 2H), 2.08 (m, 1H), 1.91-1.68 (m, 4H)

### Example 43: 3-[[1-[2-(4-Methoxyphenyl)-2-oxoethyl]-4-piperidinyl]methyl]-6-bromo-2-oxobenzothiazoline hydrochloride (Compound 179 in Table 1)

The compound obtained in Example 31 (b) and 2-bromo-4'-methoxyacetophenone were used to perform a reaction in the same manner as described in Example 31 (c), and the product was purified by silica gel column chromatography (eluent: methylene chloride/methanol = 30:1) to obtain the free form of the title compound as an oil.

The above free compound was dissolved in ethyl acetate/ethanol (3:1), and the solution was added with 4 N hydrochloric acid solution in ethyl acetate to obtain the title compound as white solid (yield: 78%, for two steps). Melting point: 165-170°C.
¹H-NMR (DMSO-d₆): 9.84 (bs, 1H), 8.97-7.93 (m, 3H), 7.60 (dd, 1H), 7.43 (d, 1H), 7.13 (d, 2H), 5.07-4.93 (m, 2H), 3.99-3.89 (m, 2H), 3.88 (s, 3H). 3.54-3.48 (m, 2H), 3.08-2.94 (m, 2H), 2.08 (m, 1H), 1.91-1.68 (m, 4H)

### Test Example: Sigma binding site-binding inhibition test

A sigma 2-selective receptor binding inhibition test was performed by using [³H]-di-o-tolylguanidine (DTG, final concentration: 1 nM. 37 Ci/mmol, New England Nuclear, Dupont de Nemours) as a radioactive ligand. A crude P2 membrane fraction was prepared from livers of male rats (Sprague-Dawley rats) according to a method described in the literature (X. He, et al., J. Med. Chem., 36, pp.566-571, 1993). In the presence of 500 nM pentazocine and a control compound at various concentrations (haloperidol, 10⁻¹⁰ to 10⁻⁶ M) or a test ligand at various concentrations (10⁻¹⁰ to 10⁻⁵ M) together with a radioactive ligand, the P2 fraction (0.4 ml) was incubated in 50 mM Tris-HCl (pH 7.4, final volume: 0.5 ml) at 25°C for 2 hours. The reaction mixture was rapidly filtered on a Brandel cell harvester using Whatman GF/B filter paper, which was immersed beforehand in 0.5% polyethyleneimine for 1 hour, to stop the reaction. The filter paper was washed 4 times with the ice cooled incubation buffer. The non-specific results were evaluated by using 1 µM haloperidol, and the scintillation analysis and the curve analysis were performed as described above. The Kd value for [³H]-DTG was 6.9 nM.

**Table 3**

| Test compound | Sigma 2 Ki (nM) |
|---|---|
| Haloperidol | 29 |
| Comparative Example 1 | 8.7 |
| Example 2 | 7.0 |
| Example 3 | 35 |
| Example 4 | 4.0 |
| Example 5 | 7.2 |
| Example 6 | 25 |
| Example 9 | 9.3 |
| Example 11 | 36 |
| Example 15 | 28 |
| Example 18 | 23 |
| Example 19 | 21 |
| Example 21 | 17 |
| Example 22 | 9.4 |
| Example 23 | 7.3 |
| Example 27 | 17 |
| Example 28 | 19 |
| Example 31 | 6.3 |
| Example 32 | 4.0 |
| Example 33 | 4.5 |
| Example 35 | 13 |
| Example 36 | 31 |
| Example 39 | 3.1 |
| Example 40 | 4.7 |

### Formulation Examples

The pharmaceutical compositions of present invention can be manufactured by utilizing methods and pharmaceutical additives conventionally used in the field of the art. Typical formulation examples are shown below. However, the pharmaceutical compositions of present invention are not limited to these examples.

### 1) Tablet

| | |
|---|---|
| Compound of Example 2 | 5-50 mg |
| Calcium phosphate | 20 mg |
| Lactose | 30 mg |
| Talc | 10 mg |
| Magnesium stearate | 5 mg |
| Potato starch | ad 200 mg |

### 2) Suspension

Water is added to the compound of Example 2 (1-5 mg), carboxymethylcellulose sodium (50 mg), sodium benzoate (1 mg) and sorbitol (500 mg) to produce an aqueous suspension for oral administration in a total volume of 1 ml.

### 3) Injection

The compound of Example 2 as an active ingredient is mixed in an amount of 1.5% by weight with a mixture of propylene glycol (10% by volume) and distilled water for injection by stirring, and the resulting solution is sterilized by filtration through a membrane filter to produce a composition for injection.

### 4) Ointment

| | |
|---|---|
| Compound of Example 2 | 5-1,000 mg |
| Stearyl alcohol | 3 g |
| Lanolin | 5 g |
| White Vaseline | 15 g |
| Water | ad 100 g |

### Industrial Applicability

The compounds of the present invention have high affinity for the sigma binding site. Therefore, the compounds of the present invention are useful as sigma ligands for therapeutic and/or preventive treatment of various kinds of diseases and symptoms in which the sigma ligands involve.

## Claims

1. A compound represented by the following formula (I): X-Q-C(R¹)(R²)-Z, a salt thereof, and a hydrate thereof and a solvate thereof, wherein: R¹ and R² each independently represent hydrogen atom, an alkyl group, a cycloalkyl group, a hydroxyalkyl group, or an alkenyl group; Z represents 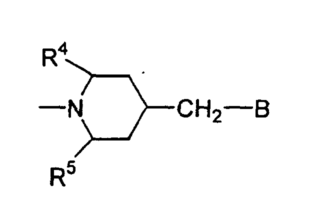 wherein R⁴ and R⁵ each independently represent hydrogen atom or an alkyl group, or R⁴ and R⁵ represent a 5- to 7-membered heterocyclic group together with other intervening atoms; and B represents a group represented by the following formula: 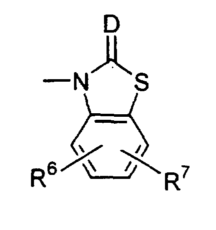 wherein R⁶ and R⁷ each independently represent a substituent selected from the group consisting of hydrogen atom, a halogen atom, nitro group, an alkyl group, a cycloalkyl group, an alkenyl group, a halogenated alkyl group, hydroxyl group, an alkoxyl group, a halogenated alkoxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group, and an alkynyl group; and D represents sulfur atom, oxygen atom, or a group represented by NR⁸ wherein R⁸ represents a substituent selected from the group consisting of hydrogen atom, an alkyl group, a cycloalkyl group, a cycloalkyl-substituted alkyl group, an alkenyl group, a halogenated alkyl group, a hydroxyalkyl group, an alkoxyalkyl group, an alkoxycarbonyl group, phenyl group, an alkylsulfinyl group, an alkylsulfonyl group, a substituted or unsubstituted sulfamoyl group, an alkylsulfonylamino group, an alkylcarbonylamino group, a substituted or unsubstituted carbamoyl group, an acyl group, cyano group and an alkynyl group; and
when X represents an aryl group, which may have one or more substituents on the ring, selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group or when X represents a heteroaryl group, which may have one or more substituents on the ring, selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group and an alkynyl group, Q represents a group represented by-CO-, -C(=NOH)- or -C(Y)(A)- wherein Y represents hydrogen atom, an alkyl group, an alkenyl group, an alkynyl group, a monocyclic or polycyclic cycloalkyl group, a cycloalkyl-substituted alkyl group, an aryl group, an aryl-substituted alkyl group, a heteroaryl group, or a heteroaryl-substituted alkyl group, and A represents a group represented by -OR⁹ wherein R⁹ represents hydrogen atom, an alkyl group, a cycloalkyl group, a cycloalkyl-substituted alkyl group, an alkenyl group, an aryl group, an aryl-substituted alkyl group, a hydroxyalkyl group, or an acyl group, and when X represents a 8- to 10-membered bicyclic heteroaryl group containing one or two hetero atoms, Q represents a single bond.

2. The compound or a salt thereof, or a hydrate thereof or a solvate thereof according to claim 1, wherein both of R¹ and R² are hydrogen atoms, R⁴ and R⁵ each independently represent hydrogen atom or an alkyl group, R⁶ and R⁷ each independently represent a substituent selected from the group consisting of hydrogen atom, a halogen atom, nitro group, an alkyl group, a halogenated alkyl group, and an alkoxyl group, R⁸ represents a substituent selected from the group consisting of hydrogen atom, an alkyl group, a halogenated alkyl group and an acyl group; when X represents a substituted or unsubstituted phenyl group (the substituent is at least one substituents selected from the group consisting of a halogen atom, an alkyl group, a halogenated alkyl group, an alkoxyl group, a halogenated alkoxyl group, cyano group and a substituted or unsubstituted aminosulfonyloxy group), Q is a group represented by -CO-, -C(=NOH)- or -C(Y)(A)- where Y is hydrogen atom, and A is a group represented by -OR⁹ wherein R⁹ is hydrogen atom, an alkyl group, or an acyl group, and when X is a 8- to 10-membered bicyclic heteroaryl group containing one or two hetero atoms, which may have one or more substituents on the ring, selected from the group consisting of a halogen atom, nitro group, an alkyl group, a cycloalkyl group, an alkenyl group, a halogenated alkyl group, hydroxyl group, an alkoxyl group, a halogenated alkoxyl group, carboxyl group, an alkoxycarbonyl group, phenyl group, a substituted or unsubstituted amino group, an alkylthio group, an alkylsulfinyl group, an alkylsulfonyl group, a substituted or unsubstituted sulfamoyl group, a substituted or unsubstituted carbamoyl group, an acyl group, cyano group, an alkynyl group, Q is a single bond.

3. The compound or a salt thereof, or a hydrate thereof or a solvate thereof according to claim 2, wherein both of R⁴ and R⁵ are hydrogen atoms, R⁶ is hydrogen atom, R⁷ is a substituent selected from the group consisting of hydrogen atom, a halogen atom, nitro group, an alkyl group, a halogenated alkyl group, and an alkoxyl group, X represents a substituted or unsubstituted phenyl group (the substituent is at least one substituent selected from the group consisting of a halogen atom, an alkyl group, a halogenated alkyl group, an alkoxyl group, a halogenated alkoxyl group, cyano group, and a substituted or unsubstituted aminosulfonyloxy group), and Q is a group represented by -CO-, -C(=NOH)- or -C(Y)(A)- wherein Y is hydrogen atom, and A is a group represented by -OR⁹ wherein R⁹ is hydrogen atom, an alkyl group, or an acyl group.

4. The compound or a salt thereof, or a hydrate thereof or a solvate thereof according to any one of claims 2 or 3, wherein R⁶ is hydrogen atom, R⁷ is a halogen atom, and D is a group represented by NR⁸ wherein R⁸ is hydrogen atom or an alkyl group.

5. The compound or a salt thereof, or a hydrate thereof or a solvate thereof according to any one of claims 2 to 4, wherein R⁶ is hydrogen atom, R⁷ is a halogen atom, and D is oxygen atom.

6. The compound or a salt thereof, or a hydrate thereof or a solvate thereof according to any one of claims 2 through 5, wherein Q is -CO- or -CH(OH)-.

7. The compound or a salt thereof, or a hydrate thereof or a solvate thereof according to claim 3, wherein X is p-fluorophenyl group, R⁷ is chlorine atom, D is NH and Q is -CO- or -CH(OH)-.

8. The compound or a salt thereof, or a hydrate thereof or a solvate thereof according to claim 3, wherein X is p-fluorophenyl group, R⁷ is chlorine atom, D is NCH₃, and Q is -CO- or -CH(OH)-.

9. The compound or a salt thereof, or a hydrate thereof or a solvate thereof according to claim 3, wherein X is p-fluorophenyl group, R⁷ is bromine atom, D is oxygen atom and Q is -CO- or -CH(OH)-.

10. A medicament comprising a substance selected from the group consisting of a compound and a salt thereof, and a hydrate thereof and a solvate thereof according to any one of claims 1 through 9 as an active ingredient.

11. Use of the compound of claims 1 to 9 for the production of a medicament, which is used for therapeutic and/or preventive treatment of a disease caused or promoted by the nerve controlling function of a sigma ligand.

12. A sigma ligand comprising a substance selected from the group consisting of a compound and a salt thereof, and a hydrate thereof and a solvate thereof according to any one of claims 1 through 9.

13. Use of the compound of claims 1 to 9 for the production of a medicament, which is used as antispychotic drug.

## Patentansprüche

1. Verbindung, dargestellt durch die folgende Formel (I): X-Q-C(R¹)(R²)-Z, deren Salz, und deren Hydrat und deren Solvat, worin: R¹ und R² jeder unabhängig voneinander ein Wasserstoffatom, eine Alkyl-Gruppe, eine Cycloalkyl-Gruppe, eine Hydroxyalkyl-Gruppe oder eine Alkenyl Gruppe darstellen; Z 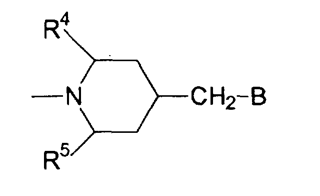 repräsentiert, worin R⁴ und R⁵ jeder unabhängig voneinander ein Wasserstoffatom oder eine Alkyl-Gruppe darstellen, oder R⁴ und R⁵ zusammen mit anderen dazwischenliegenden Atomen eine 5- bis 7-gliedrige heterozyklische Gruppe darstellen; und B eine durch die folgende Formel repräsentierte Gruppe darstellt: 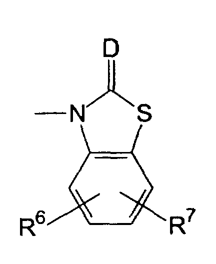 worin R⁶ und R⁷ jeder unabhängig voneinander einen Substituenten darstellen, ausgewählt aus der Gruppe bestehend aus einem Wasserstoffatom, einem Halogen-Atom, einer Nitro-Gruppe, einer Alkyl-Gruppe, einer Cycloalkyl-Gruppe, einer Alkenyl-Gruppe, einer halogenierten Alkyl-Gruppe, einer Hydroxyl-Gruppe, einer Alkoxyl-Gruppe, einer halogenierten Alkoxyl-Gruppe, einer Alkoxycarbonyl-Gruppe, einer Phenyl-Gruppe, einer substituierten oder unsubstituierten Amino-Gruppe, eine Alkylthio-Gruppe, einer Alkylsulfinyl-Gruppe, einer Alkylsulfonyl-Gruppe, einer substituierten oder unsubstituierten Sulfamoyl-Gruppe, einer substituierten oder unsubstituierten Carbamoyl-Gruppe, einer Acyl-Gruppe, einer Cyano-Gruppe und einer Alkinyl-Gruppe; und D ein Schwefelatom, ein Sauerstoffatom oder eine Gruppe, repräsentiert durch NR⁸, darstellt, in der R⁸ einen Substituenten darstellt, ausgewählt aus der Gruppe bestehend aus einem Wasserstoffatom, einer Alkyl-Gruppe, einer Cycloalkyl-Gruppe, einer Cycloalkyl-substituierten Alkyl-Gruppe, einer Alkenyl-Gruppe, einer halogenierten Alkyl-Gruppe, einer Hydroxyalkyl-Gruppe, einer Alkoxyalkyl-Gruppe, einer Alkoxycarbonyl-Gruppe, einer Phenyl-Gruppe, einer Alkylsulfinyl-Gruppe, einer Alkylsulfonyl-Gruppe, einer substituierten oder unsubstituierten Sulfamoyl-Gruppe, einer Alkylsulfonylamino-Gruppe, einer Alkylcarbonylamino-Gruppe, einer substituierten oder unsubstituierten Carbamoyl-Gruppe, einer Acyl-Gruppe, einer Cyano Gruppe und einer AIkinyl-Gruppe; und
wenn X eine Aryl-Gruppe darstellt, die einen oder mehrere Substituenten am Ring aufweisen kann, ausgewählt aus der Gruppe bestehend aus einem Halogenatom, einer Nitro-Gruppe, einer Alkyl-Gruppe, einer Cycloalkyl-Gruppe, einer Alkenyl-Gruppe, einer halogenierten Alkyl-Gruppe, einer Hydroxyl-Gruppe, einer Alkoxyl-Gruppe, einer halogenierten Alkoxyl-Gruppe, einer Carboxyl-Gruppe, einer Alkoxycarbonyl-Gruppe, einer Phenyl-Gruppe, einer substituierten oder unsubstituierten Amino-Gruppe, einer Alkylthio-Gruppe, einer Alkylsulfinyl-Gruppe, einer Alkylsulfonyl-Gruppe, einer substituierten oder unsubstituierten Sulfamoyl-Gruppe, einer substituierten oder unsubstituierten Carbamoyl-Gruppe, einer Acyl-Gruppe, einer Cyano-Gruppe und einer Alkinyl-Gruppe, oder wenn X eine Heteroaryl-Gruppe darstellt, welche einen oder mehrere Substituenten am Ring aufweisen kann, ausgewählt aus der Gruppe bestehend aus einem Halogenatom, einer Nitro-Gruppe, einer Alkyl-Gruppe, einer Cycloalkyl-Gruppe, einer Alkenyl-Gruppe, einer halogenierten Alkyl-Gruppe, einer Hydroxyl-Gruppe, einer Alkoxyl-Gruppe, einer halogenierten Alkoxyl-Gruppe, einer Carboxyl-Gruppe, einer Alkoxycarbonyl-Gruppe, einer Phenyl-Gruppe, einer substituierten oder unsubstituierten Amino-Gruppe, einer Alkylthio-Gruppe, einer Alkylsulfinyl-Gruppe, einer Alkylsulfonyl-Gruppe, einer substituierten oder unsubstituierten Sulfamoyl-Gruppe, einer substituierten oder unsubstituierten Carbamoyl-Gruppe, einer Acyl-Gruppe, einer Cyano-Gruppe und einer Alkinyl-Gruppe, stellt Q eine Gruppe dar, repräsentiert durch -CO-, -C(=NOH)-oder -C(Y)(A)-, in der Y ein Wasserstoffatom, eine Alkyl-Gruppe, eine Alkenyl-Gruppe, eine Alkinyl-Gruppe, eine monozyklische oder polyzyklische Cycloalkyl-Gruppe, eine Cycloalkyl-substituierte Alkyl-Gruppe, eine Aryl-Gruppe, eine Aryl-substituierte Alkyl-Gruppe, eine Heteroaryl-Gruppe oder eine Heteroaryl-substituierte Alkyl-Gruppe repräsentiert, und A eine Gruppe darstellt, repräsentiert durch -OR⁹, in der R⁹ ein Wasserstoffatom, eine Alkyl-Gruppe, eine Cycloalkyl-Gruppe, eine Cycloalkyl-substituierte Alkyl-Gruppe, eine Alkenyl-Gruppe, eine Aryl-Gruppe, eine Aryl-substituierte Alkyl-Gruppe, eine Hydroxyalkyl-Gruppe oder eine Acyl-Gruppe darstellt, und wenn X eine 8- bis 10-gliedrige bizyklische Heteroaryl-Gruppe darstellt, die ein oder zwei Heteroatome enthält, repräsentiert Q eine Einfachbindung.

2. Verbindung oder deren Salz, oder deren Hydrat oder deren Solvat gemäß Anspruch 1, in der sowohl R¹ als auch R² Wasserstoffatome darstellen, R⁴ und R⁵ jeder unabhängig voneinander ein Wasserstoffatom oder eine Alkyl-Gruppe repräsentieren, R⁶ und R⁷ jeder unabhängig voneinander einen Substituenten, ausgewählt aus der Gruppe bestehend aus einem Wasserstoffatom, einem Halogenatom, einer Nitro-Gruppe, einer Alkyl-Gruppe, einer halogenierten Alkyl-Gruppe und einer Alkoxyl-Gruppe darstellen, R⁸ einen Substituenten, ausgewählt aus der Gruppe bestehend aus einem Wasserstoffatom, einer Alkyl-Gruppe, einer halogenierten Alkyl-Gruppe und einer Acyl-Gruppe repräsentiert; wenn X eine substituierte oder unsubstituierte Phenyl-Gruppe darstellt (der Substituent ist zumindest ein Substituent ausgewählt aus der Gruppe bestehend aus einem Halogenatom, einer Alkyl-Gruppe, einer halogenierten Alkyl-Gruppe, einer Alkoxyl-Gruppe, einer halogenierten Alkoxyl-Gruppe, einer Cyano-Gruppe und einer substituierten oder unsubstituierten Aminosulfonyloxy-Gruppe), stellt Q eine Gruppe dar, repräsentiert durch -CO-, -C(=NOH)- oder -C(Y)(A)-, in der Y ein Wasserstoffatom darstellt und A für eine Gruppe steht, die durch -OR⁹ dargestellt ist, in der R⁹ ein Wasserstoffatom, eine Alkyl-Gruppe oder eine Acyl-Gruppe ist, und wenn X eine 8- bis 10-gliedrige bizyklische Heteroaryl-Gruppe ist, die einen oder zwei Heteroatome enthält, welche einen oder mehrere Substituenten am Ring aufweisen kann, ausgewählt aus der Gruppe bestehend aus einem Halogenatom, einer Nitro-Gruppe, einer Alkyl-Gruppe, einer Cycloalkyl-Gruppe, einer Alkenyl-Gruppe, einer halogenierten Alkyl-Gruppe, einer Hydroxyl-Gruppe, einer Alkoxyl-Gruppe, einer halogenierten Alkoxyl-Gruppe, einer Carboxyl-Gruppe, einer Alkoxycarbonyl-Gruppe, einer Phenyl-Gruppe, einer substituierten oder unsubstituierten Amino-Gruppe, einer Alkylthio-Gruppe, einer Alkylsulfinyl-Gruppe, einer Alkylsulfonyl-Gruppe, einer substituierten oder unsubstituierten Sulfamoyl-Gruppe, einer substituierten oder unsubstituierten Carbamoyl-Gruppe, einer Acyl-Gruppe, einer Cyano-Gruppe, einer Alkinyl-Gruppe, stellt Q eine Einfachbindung dar.

3. Verbindung oder deren Salz, oder deren Hydrat oder deren Solvat gemäß Anspruch 2, in der sowohl R⁴ als auch R⁵ Wasserstoffatome sind, R⁶ ein Wasserstoffatom darstellt, R⁷ ein Substituent ist, ausgewählt aus der Gruppe bestehend aus einem Wasserstoffatom, einem Halogenatom, einer Nitro-Gruppe, einer Alkyl-Gruppe, einer halogenierten Alkyl-Gruppe, und einer Alkoxyl-Gruppe, X eine substituierte oder unsubstituierte Phenyl-Gruppe repräsentiert (der Substituent ist zumindest ein Substituent ausgewählt aus der Gruppe bestehend aus einem Halogenatom, einer Alkyl-Gruppe, einer halogenierten Alkyl-Gruppe, einer Alkoxyl-Gruppe, einer halogenierten Alkoxyl-Gruppe, einer Cyano-Gruppe und einer substituierten oder unsubstituierten Aminosulfonyloxy-Gruppe), und Q eine Gruppe darstellt, repräsentiert durch -CO-, -C(=NOH)- oder -C(Y)(A)-, worin Y ein Wasserstoffatom ist, und A eine Gruppe darstellt, repräsentiert durch -OR⁹, wobei R⁹ ein Wasserstoffatom, eine Alkyl-Gruppe oder eine Acyl-Gruppe darstellt.

4. Verbindung oder deren Salz, oder deren Hydrat oder deren Solvat gemäß irgend einem der Ansprüche 2 oder 3, wobei R⁶ ein Wasserstoffatom darstellt, R⁷ für ein Halogenatom steht und D eine Gruppe ist, repräsentiert durch NR⁸, in der R⁸ ein Wasserstoffatom oder eine Alkyl-Gruppe darstellt.

5. Verbindung oder deren Salz, oder deren Hydrat oder deren Solvat gemäß irgend einem der Ansprüche 2 bis 4, wobei R⁶ ein Wasserstoffatom ist, R⁷ für ein Halogenatom steht und D ein Sauerstoffatom darstellt.

6. Verbindung oder deren Salz, oder deren Hydrat oder deren Solvat gemäß irgend einem der Ansprüche 2 bis 5, wobei Q -CO- oder -CH(OH)- darstellt.

7. Verbindung oder deren Salz, oder deren Hydrat oder deren Solvat gemäß Anspruch 3, wobei X eine p-Fluorophenyl-Gruppe darstellt, R⁷ für ein Chloratom steht, D NH ist und Q -CO- oder -CH(OH)- darstellt.

8. Verbindung oder deren Salz, oder deren Hydrat oder deren Solvat gemäß Anspruch 3, wobei X eine p-Fluorophenyl-Gruppe darstellt, R⁷ für ein Chloratom steht, D NCH₃ ist und Q -CO- oder -CH(OH)- darstellt.

9. Verbindung oder deren Salz, oder deren Hydrat oder deren Solvat gemäß Anspruch 3, wobei X eine p-Fluorophenyl-Gruppe darstellt, R⁷ für ein Bromatom steht, D ein Sauerstoffatom ist und Q -CO- oder -CH(OH)- darstellt.

10. Medikament umfassend eine Substanz ausgewählt aus der Gruppe bestehend aus einer Verbindung und deren Salz, und deren Hydrat und deren Solvat gemäß irgend einem der Ansprüche 1 bis 9 als ein aktiver Bestandteil.

11. Verwendung der Verbindung der Ansprüche 1 bis 9 zur Herstellung eines Medikamentes, welches zur therapeutischen und/oder präventiven Behandlung einer Krankheit verwendet wird, die durch die Nerv-kontrollierende Funktion eines Sigma-Liganden hervorgerufen oder gefördert wird.

12. Sigma-Ligand, umfassend eine Substanz ausgewählt aus der Gruppe bestehend aus einer Verbindung und deren Salz, und deren Hydrat und deren Solvat gemäß irgend einem der Ansprüche 1 bis 9.

13. Verwendung der Verbindung der Ansprüche 1 bis 9 zur Herstellung eines Medikamentes, welches als antipsychotisches Arzneimittel verwendet wird.

## Revendications

1. Composé représenté par la formule (I) suivante : X-Q-C(R¹)(R²)-Z, un sel de celui-ci et un hydrate de celui-ci et un solvate de celui-ci, dans laquelle
R¹ et R² représentent chacun indépendamment, l'atome d'hydrogène, un groupe alkyle, un groupe cycloalkyle, un groupe hydroxyalkyle ou un groupe alcényle ; Z représente : 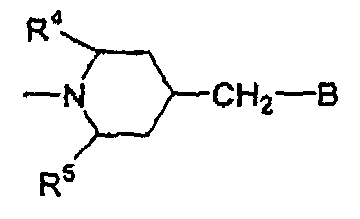 où R⁴ et R⁵ représentent chacun indépendamment, l'atome d'hydrogène ou un groupe alkyle, ou R⁴ et R⁵ représentent un groupe hétérocyclique de 5 à 7 membres, avec les autres atomes intervenant, et B représente un groupe représenté par la formule suivante : 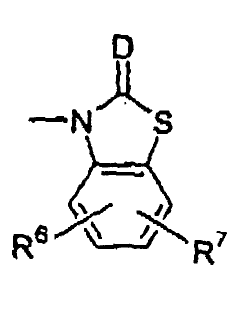 dans laquelle R⁶ et R⁷ représentent chacun indépendamment, un substituant choisi parmi le groupe consistant en l'atome d'hydrogène, un atome d'halogène, le groupe nitro, un groupe alkyle, un groupe cycloalkyle, un groupe alcényle, un groupe alkyle halogéné, le groupe hydroxyle, un groupe alcoxyle, un groupe alcoxyle halogéné, un groupe alcoxycarbonyle, le groupe phényle, un groupe amino substitué ou non substitué, un groupe alkylthio, un groupe alkylsulfinyle, un groupe alkylsulfonyle, un groupe sulfamoyle substitué ou non substitué, un groupe carbamoyle substitué ou non substitué, un groupe acyle, le groupe cyano, et un groupe alcynyle ; et D représente l'atome de soufre, l'atome d'oxygène ou un groupe représenté par NR⁸, où R⁸ représente un substituant choisi parmi le groupe consistant en l'atome d'hydrogène, un groupe alkyle, un groupe cycloalkyle, un groupe alkyle substitué par cycloalkyle, un groupe alcényle, un groupe alkyle halogéné, un groupe hydroxyalkyle, un groupe alcoxyalkyle, un groupe alcoxycarbonyle, le groupe phényle, un groupe alkylsulfinyle, un groupe alkylsulfonyle, un groupe sulfamoyle substitué ou non substitué, un groupe alkylsulfonylamino, un groupe alkylcarbonylamino, un groupe carbamoyle substitué ou non substitué, un groupe acyle, le groupe cyano, et un groupe alcynyle ; et
lorsque X représente un groupe aryle, qui peut avoir un ou plusieurs subtituants sur le cycle, choisis parmi le groupe consistant en un atome d'halogène, le groupe nitro, un groupe alkyle, un groupe cycloalkyle, un groupe alcényle, un groupe alkyle halogéné, le groupe hydroxyle, un groupe alcoxyle, un groupe alcoxyle halogéné, un groupe carboxyle, un groupe alcoxycarbonyle, le groupe phényle, un groupe amino substitué ou non substitué, un groupe alkylthio, un groupe alkylsulfinyle, un groupe alkylsulfonyle, un groupe sulfamoyle substitué ou non substitué, un groupe carbamoyle substitué ou non substitué, un groupe acyle, le groupe cyano, et un groupe alcynyle, ou lorsque X représente un groupe hétéroaryle, qui peut avoir un ou plusieurs subtituants sur le cycle, choisis parmi le groupe consistant en un atome d'halogène, le groupe nitro, un groupe alkyle, un groupe cycloalkyle, un groupe alcényle, un groupe alkyle halogéné, le groupe hydroxyle, un groupe alcoxyle, un groupe alcoxyle halogéné, un groupe carboxyle, un groupe alcoxycarbonyle, le groupe phényle, un groupe amino substitué ou non substitué, un groupe alkylthio, un groupe alkylsulfinyle, un groupe alkylsulfonyle, un groupe sulfamoyle substitué ou non substitué, un groupe carbamoyle substitué ou non substitué, un groupe acyle, le groupe cyano, et un groupe alcynyle, Q représente un groupe représenté par - CO, -C(=NOH)- ou -C(Y) (A)-, où Y représente l'atome d'hydrogène, un groupe alkyle, un groupe alcényle, un groupe alcynyle, un groupe cycloalkyle mono- ou polycyclique, un groupe alkyle substitué par cycloalkyle, un groupe aryle, un groupe alkyle substitué par aryle, un groupe hétéroaryle ou un groupe alkyle substitué par hétéroaryle, et A représente un groupe représenté par -OR⁹, où R⁹ représente l'atome d'hydrogène, un groupe alkyle, un groupe cycloalkyle, un groupe alkyle substitué par cycloalkyle, un groupe alcényle, un groupe aryle, un groupe alkyle substitué par aryle, un groupe hydroxyalkyle ou un groupe acyle, et lorsque X représente un groupe hétéroaryle bicyclique ayant 8 à 10 membres, contenant un ou deux hétéroatomes, Q représente une simple liaison.

2. Composé ou un sel de celui-ci, ou un hydrate de celui-ci ou un solvate de celui-ci, selon la revendication 1, dans lequel R¹ et R² tous les deux sont des atomes d'hydrogène, R⁴ et R⁵ représentent chacun indépendamment, l'atome d'hydrogène ou un groupe alkyle, R⁶ et R⁷ représentent chacun indépendamment, un substituant choisi parmi le groupe consistant en l'atome d'hydrogène, un atome d'halogène, le groupe nitro, un groupe alkyle, un groupe alkyle halogéné, et un groupe alcoxyle, R⁸ représente un substituant choisi parmi le groupe consistant en l'atome d'hydrogène, un groupe alkyle, un groupe alkyle halogéné et un groupe acyle ; lorsque X représente un groupe phényle substitué ou non substitué (le substituant est au moins un des subtituants choisis parmi le groupe consistant en un atome d'halogène, un groupe alkyle, un groupe alkyle halogéné, un groupe alcoxyle, un groupe alcoxyle halogéné, le groupe cyano et un groupe aminosulfonyloxy substitué ou non substitué), Q représente un groupe représenté par -CO, -C(=NOH)- ou -C(Y)(A)-, où Y représente l'atome d'hydrogène, et A représente un groupe représenté par -OR⁹, où R⁹ représente l'atome d'hydrogène, un groupe alkyle, ou un groupe acyle, et lorsque X représente un groupe hétéroaryle bicyclique ayant 8 à 10 membres, contenant un ou deux hétéroatomes, qui peut avoir un ou plusieurs substituants sur le cycle, choisis parmi le groupe consistant en un atome d'halogène, le groupe nitro, un groupe alkyle, un groupe cycloalkyle, un groupe alcényle, un groupe alkyle halogéné, le groupe hydroxyle, un groupe alcoxyle, un groupe alcoxyle halogéné, le groupe carboxyle, un groupe alcoxycarbonyle, le groupe phényle, un groupe amino substitué ou non substitué, un groupe alkylthio, un groupe alkylsulfinyle, un groupe alkylsulfonyle, un groupe sulfamoyle substitué ou non substitué, un groupe carbamoyle substitué ou non substitué, un groupe acyle, le groupe cyano, un groupe alcynyle, Q représente une simple liaison.

3. Composé ou un sel de celui-ci, ou un hydrate de celui-ci ou un solvate de celui-ci, selon la revendication 2, dans lequel R⁴ et R⁵ sont tous les deux des atomes d'hydrogène, R⁶ est l'atome d'hydrogène, R⁷ est un substituant choisi parmi le groupe consistant en l'atome d'hydrogène, un atome d'halogène, le groupe nitro, un groupe alkyle, un groupe alkyle halogéné, et un groupe alcoxyle, X représente un groupe phényle, substitué ou non substitué (le substituant est au moins un subtituant choisi parmi le groupe consistant en un atome d'halogène, un groupe alkyle, un groupe alkyle halogéné, un groupe alcoxyle, un groupe alcoxyle halogéné, le groupe cyano, et un groupe aminosulfonyloxy substitué ou non substitué), et Q représente un groupe représenté par -CO, -C(=NOH)- ou - C(Y) (A)-, où Y représente l'atome d'hydrogène, et A représente un groupe représenté par -OR⁹, où R⁹ représente l'atome d'hydrogène, un groupe alkyle, ou un groupe acyle.

4. Composé ou un sel de celui-ci, ou un hydrate de celui-ci ou un solvate de celui-ci, selon l'une quelconque des revendications 2 ou 3, dans lequel R⁶ est l'atome d'hydrogène, R⁷ est un atome d'halogène, et D est un groupe représenté par NR⁸, où R⁸ est l'atome d'hydrogène ou un groupe alkyle.

5. Composé ou un sel de celui-ci, ou un hydrate de celui-ci ou un solvate de celui-ci, selon l'une quelconque des revendications 2 à 4, dans lequel R⁶ est l'atome d'hydrogène, R⁷ est un atome d'halogène, et D est l'atome d'oxygène.

6. Composé ou un sel de celui-ci, ou un hydrate de celui-ci ou un solvate de celui-ci, selon l'une quelconque des revendications 2 à 5, où Q est -CO- ou -CH(OH)-.

7. Composé ou un sel de celui-ci, ou un hydrate de celui-ci ou un solvate de celui-ci, selon la revendication 3, dans lequel X est le groupe p-fluorophényle, R⁷ est l'atome de chlore, D est NH et Q est -CO- ou -CH(OH)-.

8. Composé ou un sel de celui-ci, ou un hydrate de celui-ci ou un solvate de celui-ci, selon la revendication 3, dans lequel X est le groupe p-fluorophényle, R⁷ est l'atome de chlore, D est NCH₃ et Q est -CO- ou -CH(OH)-.

9. Composé ou un sel de celui-ci, ou un hydrate de celui-ci ou un solvate de celui-ci, selon la revendication 3, dans lequel X est le groupe p-fluorophényle, R⁷ est l'atome de brome, D est l'atome d'oxygène et Q est -CO- ou -CH(OH)-.

10. Médicament comprenant une substance choisie parmi le groupe consistant en un composé et un sel de celui-ci, et un hydrate de celui-ci et un solvate de celui-ci, selon l'une quelconque des revendications 1 à 9, comme ingrédient actif.

11. Utilisation du composé des revendications 1 à 9, pour la production d'un médicament, qui est utilisé pour le traitement thérapeutique et/ou préventif d'une maladie causée ou favorisée par la fonction contrôlant les nerfs d'un ligand sigma.

12. Ligand sigma comprenant une substance choisie parmi le groupe consistant en un composé et un sel de celui-ci, et un hydrate de celui-ci et un solvate de celui-ci, selon l'une quelconque des revendications 1 à 9.

13. Utilisation du composé des revendications 1 à 9, pour la production d'un médicament, qui est utilisé comme médicament antipsychotique.
